(19) Europäisches Patentamt
European Patent Office
Office européen des brevets

(11) **EP 4 732 876 A2**

(12) # EUROPEAN PATENT APPLICATION

(43) Date of publication:
**29.04.2026 Bulletin 2026/18**

(21) Application number: **25221447.3**

(22) Date of filing: **16.04.2021**

(51) International Patent Classification (IPC):
***A61M 16/20*** *(2006.01)*

(52) Cooperative Patent Classification (CPC):
**A61M 16/024; A61M 16/0816; A61M 16/0875;
A61M 16/109; A61M 16/1095; A61M 16/16;**
A61M 16/0051; A61M 16/0069; A61M 16/06;
A61M 16/0683; A61M 16/1005; A61M 16/125;
A61M 16/161; A61M 16/208; A61M 2016/0027;

(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.04.2020 US 202063011052 P
06.07.2020 US 202063048535 P**

(62) Document number(s) of the earlier application(s) in
accordance with Art. 76 EPC:
**21788971.6 / 4 135 806**

(71) Applicant: **ResMed Pty Ltd
Bella Vista, NSW 2153 (AU)**

(72) Inventors:
• **DANTANARAYANA, Muditha Pradeep
Bella Vista, New South Wales 2153 (AU)**
• **SHORT, Skye Kimberly
Bella Vista, New South Wales 2153 (AU)**
• **HARRIS, Craig Edward
Bella Vista, New South Wales 2153 (AU)**

• **ZHU, Chengwei
Bella Vista, New South Wales 2153 (AU)**
• **MAURER, Dimitri Marco
Bella Vista, New South Wales 2153 (AU)**
• **MAIKIM, Jessie
Bella Vista, New South Wales 2153 (AU)**
• **OLLEY, Liam
Bella Vista, New South Wales 2153 (AU)**

(74) Representative: **Vossius & Partner
Patentanwälte Rechtsanwälte mbB
Siebertstraße 3
81675 München (DE)**

Remarks:
•This application was filed on 08.12.2025 as a
divisional application to the application mentioned
under INID code 62.
•Claims filed after the date of receipt of the
application (Rule 68(4) EPC).

(54) **ACOUSTIC DETECTION AND/OR ANALYSIS IN RESPIRATORY TREATMENT APPARATUS**

(57)    The present invention refers to an intermediate component for pneumatically connecting an air delivery tube to a respiratory treatment apparatus, the air delivery tube configured to deliver breathable gas provided by the respiratory treatment apparatus to a patient interface, said intermediate component comprising: an outlet end configured to connect to an air delivery tube; an inlet end configured to connect to the respiratory treatment apparatus; a sound port configured to facilitate propagation of sound outside of the intermediate component; and a sound permeable membrane configured to cover the port.

EP 4 732 876 A2

**(Cont. next page)**

(52) Cooperative Patent Classification (CPC): (Cont.)
A61M 2016/0039; A61M 2205/121;
A61M 2205/123; A61M 2205/127; A61M 2205/13;
A61M 2205/14; A61M 2205/18; A61M 2205/276;
A61M 2205/3306; A61M 2205/3317;
A61M 2205/332; A61M 2205/3331;
A61M 2205/3368; A61M 2205/3375;
A61M 2205/3389; A61M 2205/3592;
A61M 2205/3653; A61M 2205/42; A61M 2205/502;
A61M 2205/505; A61M 2205/581; A61M 2205/582;
A61M 2205/583; A61M 2205/8212; A61M 2230/63

## Description

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

## 1 CROSS REFERENCE TO RELATED APPLICATIONS

This application claims priority to U.S. Provisional Application No. 63/011,052, filed April 16, 2020, and U.S. Provisional Application No. 63/048,535, filed July 06, 2020, the entire contents of each of which are incorporated herein by reference in their entirety.

## 2 BACKGROUND OF THE TECHNOLOGY

## 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

## 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient $CO_2$ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapy

Various therapies, such as Continuous Positive Airway Pressure (CPAP) therapy, Non-invasive ventilation (NIV) and Invasive ventilation (IV) have been used to treat one or more of the above respiratory disorders.

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.3 Treatment Systems

These therapies may be provided by a treatment system or device. Such systems and devices may also be used to screen, diagnose, or monitor a condition without treating it.

A treatment system may comprise a Respiratory Pressure Therapy Device (RPT device), an air circuit, a humidifier, a patient interface, and data management.

Another form of treatment system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH$_2$O relative to ambient pressure. For other forms of therapy, such as the delivery of oxygen, the patient interface may not include a seal sufficient to facilitate delivery to the airways of a supply of gas at a positive pressure of about 10 cmH$_2$O.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

## 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressurised. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH$_2$O).

| RPT Device name | A-weighted sound pressure level dB(A) | Year (approx.) |
|---|---|---|
| C-Series Tango™ | 31.9 | 2007 |
| C-Series Tango™ with Humidifier | 33.1 | 2007 |
| S8 Escape™ II | 30.5 | 2005 |
| S8 Escape™ II with H4i™ Humidifier | 31.1 | 2005 |
| S9 AutoSet™ | 26.5 | 2010 |

(continued)

| RPT Device name | A-weighted sound pressure level dB(A) | Year (approx.) |
|---|---|---|
| S9 AutoSet™ with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore medical humidifiers may have more stringent safety constraints than industrial humidifiers.

### 2.2.3.4 Acoustic analysis

A patient, a caregiver, a clinician, an insurance company or a technician may wish to collect data in relation to a respiratory therapy, whether they relate to the patient, individual components used for therapy, or the therapy system as a whole. There exist numerous situations in providing respiratory therapy to a patient where one or more parties involved therein may benefit from collection of therapy related data, and leveraging the collected data.

In particular, some components of a respiratory therapy system need to be replaced at a higher frequency than others for effective therapy. For example, a patient interface comprising a silicone seal-forming portion may be replaced by some patients in periods of months (e.g. 3 months), whereas therapy devices may be replaced or upgraded every few years (e.g. 3 years). For those components that are to be replaced at relatively frequent intervals (e.g. a patient interface), a patient or caregiver regularly faces challenges in being reliably and accurately notified, at a low cost, when their component is due to be replaced. When it is replaced, the new component may necessitate the patient or caregiver to change one or more settings in the therapy system (e.g. a software setting in the RT device) to ensure that the system is taking full advantage of the new component. The ability to identify components of a respiratory therapy system is therefore important both for optimising therapy and for keeping patients and caregivers informed about replacement timing.

One solution is to configure devices to identify components of the respiratory therapy systems of which they are a part, by acoustic means. In particular, such devices comprise structures and processes configured to analyse acoustic reflections from system components to identify those components from their "acoustic signatures" in a more accurate manner than previously known.

In such devices, a microphone is located and configured to sense sound in the air circuit. The sound from the microphone is analysed to generate the acoustic signatures of system components and thereby identify them.

Three problems can affect sound quality and consistency between devices and over time: variation in relative position of microphone and air circuit as a result of manufacturing tolerances; escape of sound to ambient; and vibration of the device during use that transmits to the microphone. There is therefore a need for an improved method of coupling the microphone to the air circuit.

3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

An aspect of the present technology relates to an intermediate component for coupling an air delivery tube to a respiratory treatment apparatus. The respiratory treatment apparatus may include a pressure generator and/or a water reservoir to which the air delivery tube is coupled via the intermediate component. In a broader case, the intermediate component may include any independent component or a portion of a component, that is in the airflow path (air-path) or is in a fluid communication with the airflow path, so as to receive sounds from the air-path. The intermediate component may be removable, replaceable and/or cleanable. In the specific aspect, where the intermediate component couples a water reservoir to the air delivery tube, the term "intermediate" in the expression "intermediate component" may refer to this coupling. However, it may also refer to the fact that at least in some specific aspects of the described technology, the component is located between the sound source and the sound sensor and provides the link between them.

In examples of the preceding aspects the water reservoir may include a cavity structured to hold a volume of water and receive a flow of breathable gas, the air delivery tube may be configured to pass a flow of breathable gas that has been humidified in the water reservoir to a patient interface, and the intermediate component may include: (a) an outlet end configured to connect the air delivery tube to the intermediate component; (b) an inlet end configured to connect the water reservoir to the intermediate component, a central axis of the inlet end is provided at an angle greater than zero to a central axis of the outlet end; (c) a sound port configured to facilitate propagation of sound outside of the intermediate component; (d) a port seal disposed around the sound port, configured to provide a peripheral sealing formation on an outside surface of the intermediate component, and including a sound permeable membrane configured to cover the port; (e) the membrane is coplanar with an inside surface of the intermediate component; (f) the membrane is impermeable to liquids and/or gases; (g) the peripheral sealing formation includes a ridge configured to chassis into which the intermediate component is inserted; (h) the ridge extend between 0.4 to 0.8 mm above an outside surface of the intermediate component; (i) the peripheral sealing formation includes a lip extending from a perimeter of the port towards a central axis of the port; (j) the lip extend between 0.4 to 0.8 mm above an outside surface of the intermediate component; and/or (k) the lip extends from perimeter of the port at an angle above the port.

An aspect of one form of the present technology relates to an intermediate component for coupling an air delivery tube to a respiratory treatment apparatus, the air delivery tube configured to deliver breathable gas provided by the respiratory treatment apparatus to a patient interface, the intermediate component comprising: an outlet end configured to connect the air delivery tube to the intermediate component; an inlet end configured to connect the respiratory treatment apparatus to the intermediate component, wherein a central axis of the inlet end is provided at an angle greater than zero to a central axis of the outlet end; a sound port configured to facilitate propagation of sound outside of the intermediate component; and a port seal disposed around the sound port, configured to provide a peripheral sealing formation on an outside surface of the intermediate component, and including a sound permeable membrane configured to cover the port.

In examples of the preceding aspects: (a) the membrane is coplanar with an inside surface of the intermediate component; (b) the membrane is impermeable to liquids and/or gases; (c) the peripheral sealing formation includes a ridge configured to chassis into which the intermediate component is inserted; (d) the ridge extend between 0.4 to 0.8 mm above an outside surface of the intermediate component; (e) the peripheral sealing formation includes a lip extending from a perimeter of the port towards a central axis of the port; (f) the lip extend between 0.4 to 0.8 mm above an outside surface of the intermediate component; (g) the lip extends from perimeter of the port at an angle above the port; and/or (h) the inlet end is configured to connect to a water reservoir disposed in the respiratory treatment apparatus and including a cavity structured to hold a volume of water and provide a flow of breathable gas that has been humidified to the air deliver tube via the intermediate component.

An aspect of one form of the present technology relates to an apparatus for treatment of a respiratory condition, the apparatus comprising: a pressure generator configured to generate a flow of breathable gas; an air delivery tube configured to pass the flow of breathable gas from the pressure generator to a patient interface; an intermediate component configured to connect pneumatically the air delivery tube to the pressure generator, the intermediate component comprising a port configured to facilitate propagation of sound outside of the intermediate component; a sound sensor disposed outside of the intermediate component and adjacent to the port of the intermediate component, the sound sensor configured to sense sound propagated outside the intermediate component; and a controller. The controller is configured to: receive a sound signal generated by the sound sensor as a result of sensing sound during operation of the apparatus, analyse the received sounds signal, and effect a response based at least in part on the analysing.

In examples of the preceding aspects: (a) the response comprises at least one of: recording a result of the analysing, displaying a result of the analysing, forwarding a result of the analysing, and controlling operation of the pressure generator based at least in part on the analysing; (b) further comprising: a water reservoir including a cavity structured to hold a volume of water and receive the flow of breathable gas so that the flow of breathable gas is humidified before being passed to the patient interface; and a water reservoir dock structured and arranged to receive the water reservoir in an operative position, wherein; the intermediate component is removably coupled to the water reservoir dock so as to receive a humidified flow of breathable gas and pass it to the air delivery tube; (c) the apparatus comprising a chassis including a chassis opening, wherein the port in the intermediate component is located on a first side of the chassis opening, and the sound sensor is positioned on a second side of the chassis opening; (d) at least one of the sensor and the port are aligned with respect to the chassis opening; (e) the apparatus comprises at least one of a membrane and a port seal, the membrane configured to cover the port and to transmit sound from inside of the intermediate component to the outside of the intermediate component, and the port seal being disposed so as to provide a sealing engagement between the port and the chassis opening; (f) the membrane and the port seal comprise an integral body permanently attached to the port; (g) the intermediate component is of a generally tubular shape and the water reservoir dock includes a generally tubular opening for receiving the intermediate component, the intermediate component and the generally tubular opening being configured for a generally frictionless insertion of the intermediate component into the opening; (h) at least one of the intermediate component and the tubular opening comprises one or more engagement formations arranged so that, during an insertion of the intermediate component into the opening, proximate the end of an insertion path, an engagement of at least one of the engagement formations brings the intermediate component into its operational configuration in which the port seal seals both the port and a chassis opening, and wherein at least one of, the sealing engagement of the port seal with the port and chassis, and supporting engagement effected by the at least one engagement formation, is configured to impede dislodgement of the intermediate component from its operational configuration in the absence of an appreciable external force; (i) one or more of the engagement formations includes an elevation feature; (j) further comprises a port seal configured to surround the port, wherein the port seal includes a peripheral sealing formation including a ridge configured to abut the surface of a chassis around the chassis opening, when the intermediate component is coupled to the humidifier; (k) the ridge extends between 0.4 to 0.8 mm above an outside surface of the intermediate component; (l) further comprising a port seal configured to surround the port, wherein the port seal includes a peripheral sealing formation including a lip configured to abut the surface of a chassis around the chassis opening, when the intermediate component is coupled to the humidifier; (m) the lip extends between 0.4 to 0.8 mm above an outside surface of the intermediate component; (n) wherein the lip extends from perimeter of the port at an angle above the port and toward a central axis of the port; (o) the port seal covers an inside surface of the port and includes the membrane; (p) the membrane is coplanar with at least an inside or an outside surface of the intermediate component; (q) the membrane is impermeable to liquids and/or gases; (r) the controller is configured to determine a characteristic of air delivery tube or a patient interface, based on the analysing; (s) the controller is further configured to determine a type or a size of an air delivery tube or a type or a size of a patient interface coupled to the air delivery tube based on the analysing; (t) the intermediate component comprises an outlet end configured to connect the air delivery tube to the intermediate component and an inlet end configured to connect the water reservoir to the intermediate component, wherein a central axis of the inlet end is substantially transverse to a central axis of the outlet end; (u) an inside corner at the inlet end of the intermediate component is rounded; (v) an inside corner comprises a bellow including a span between opposite sides of the bellow that is equal to or less than twice a radius of the inside corner; (w) an inside corner comprises a curvature of a radius of 0.2-5 mm; (x) the water reservoir includes an outlet tube comprising an outlet for delivering a humidified flow of breathable gas to the air delivery tube, and the intermediate component comprises an inlet seal adapted to from a seal with the outlet tube of the water reservoir; (y) the intermediate component comprises an outlet end, and the air delivery tube comprises a dock connector including a radial lip seal adapted to form a seal with the outlet end of the intermediate component, to pneumatically connect the air delivery tube to the intermediate component; (z) the apparatus, wherein a central axis of the port in the intermediate component is generally aligned with a central axis of the chassis opening on a first side of the chassis opening, and the sound sensor is positioned on a second side of the chassis opening less than 2 mm from the chassis opening; (aa) the sensor generally aligned with the central axes of the port and the chassis, (ab) the intermediate component is configured to pneumatically connect the air delivery tube to the water reservoir, and to mechanically connect the air delivery tube to the reservoir dock; (ac) the air delivery tube is configured to electrically connect with the reservoir dock; (ad) the air delivery tube is configured to form a mechanical and electrical connections simultaneously when the air delivery tube is connected to the intermediate component; (ae) the air delivery tube comprises a dock connector including retaining bumps adapted to engage within respective holes provided to the intermediate component to mechanically connect the air delivery tube to the intermediate component; and/or (af) the apparatus further comprising a transducer configured to generate a flow signal representing a property of flow of air, wherein the controller is configured to; control operation of the pressure generator; and during operation of the pressure generator; receive the flow signal from the transducer and sound signal sensed by the sound sensor; analyse the received sounds signal; and modify operation of the pressure generator based at least in part on the analysing and the flow signal.

An aspect of one form of the present technology relates to an intermediate component for coupling an air delivery tube to a respiratory treatment apparatus, the air delivery tube configured to deliver breathable gas provided by the respiratory treatment apparatus to a patient interface, the intermediate component comprising: an outlet end configured to connect the air delivery tube to the intermediate component; an inlet end configured to connect the respiratory treatment apparatus to the intermediate component; and a sound port configured to facilitate propagation of sound outside of the intermediate component.

In examples of the preceding aspects: (a) further including at least one of: a sound permeable membrane configured to cover the port, and a port seal disposed around the sound port, the port seal being configured to provide a peripheral sealing formation on an outside surface of the intermediate component; (b) a central axis of the inlet end is provided at an angle greater than zero to a central axis of the outlet; (c) the membrane is coplanar with an inside surface of the intermediate component; (d) the membrane is impermeable to liquids and/or gases; (e) the peripheral sealing formation includes a ridge configured to seal the sound port against an opening in a wall of a water reservoir dock, into which the intermediate component is inserted; (f) the ridge extends between 0.4 to 0.8 mm above an outside surface of the intermediate component; (g) the peripheral sealing formation includes a lip extending from a perimeter of the port towards a central axis of the port; (h) the lip extends between 0.4 to 0.8 mm above an outside surface of the intermediate component; (i) the lip extends from perimeter of the port at an angle above the port; (j) the inlet end is configured to connect to a water reservoir disposed in the respiratory treatment apparatus and including a cavity structured to hold a volume of water and provide a flow of breathable gas that has been humidified to the air delivery tube via the intermediate component; and/or (k) wherein at least two of; the central axis of the inlet end of the intermediate component, the central axis of the outlet end of the intermediate component and the central axis of the port, are transverse to each other.

An aspect of one form of the present technology relates to an apparatus comprising: a pressure generator configured to generate a flow of breathable gas; a water reservoir including a cavity structured to hold a volume of water and receive the flow of breathable gas; a water reservoir dock structured and arranged to receive the water reservoir in an operative position; an air delivery tube configured to pass the flow of breathable gas that has been humidified in the water reservoir to a patient interface; an intermediate component removably coupled to the water reservoir dock, configured to connect the air delivery tube to the water reservoir, and comprises a port configured to facilitate propagation of sound outside of the intermediate component; a sound sensor disposed adjacent to the port of the intermediate component and configured to receive sound propagated into the intermediate component from the air delivery tube and the water reservoir; and circuitry configured to receive a sound signal sensed by the sound sensor during operation of the pressure generator, analyse the received sounds signal, and control operation of the pressure generator based at least in part on the analysing.

In examples of the preceding aspects: (a) the intermediate component is configured to pneumatically connect the air delivery tube to the water reservoir, and to mechanically connect the air delivery tube to the reservoir dock; (b) further comprising a chassis coupled to the water reservoir dock and including a chassis opening, wherein the port in the intermediate component is aligned with the chassis opening on a first side of the chassis opening, and the sound sensor is positioned on a second side of the chassis opening; (c) further comprising a port seal disposed around the port and configured to provide a peripheral sealing formation on an outside surface of the intermediate component; (d) the peripheral sealing formation includes a ridge configured to abut the surface of a chassis, coupled to the water reservoir dock, around the chassis opening; (e) the ridge extend between 0.4 to 0.8 mm above an outside surface of the intermediate component; (f) the ridge is compressed against the surface of the chassis when the intermediate component is in an operative position and is configured to prevent the intermediate component to disassemble without sufficient force; (g) the peripheral sealing formation includes a lip configured to abut the surface of a chassis, coupled to the water reservoir dock, around the chassis opening; (h) the lip extend between 0.4 to 0.8 mm above an outside surface of the intermediate component; (i) the lip extends from sides of the port and above at least a portion of the port, the portion of the lip extending above the port is deflected downward when the intermediate component is in an operative position and is configured to prevent the intermediate component to disassemble without sufficient force; (j) the lip extends from perimeter of the port at an angle above the port and toward a central axis of the port; (k) the port seal covers the inside surface of the port and includes a membrane configured to cover the port and to transmit sound from inside of the intermediate component to the outside of the intermediate component; (l) the membrane is coplanar with an inside surface of the intermediate component; (m) the membrane is impermeable to liquids and/or gases; (n) the circuitry is further configured to determine a type of air delivery tube coupled to the water reservoir based on the analysing; (o) the circuitry is further configured to determine a type of patient interface coupled to the air delivery tube based on the analysing; (p) the circuitry is further configured to determine a type of patient interface coupled to the air delivery tube based on the analysing; (q) the intermediate component comprises an outlet end configured to connect the air delivery tube to the intermediate component and an inlet end configured to connect the water reservoir to the intermediate component, wherein a central axis of the inlet end is provided at an angle that is 90° or greater to a central axis of the outlet end; (r) an inside corner formed by the angle is rounded; (s) an inside corner formed by the angle comprises a bellow including a span between opposite sides of the bellow that is equal to or less than twice a radius of the inside corner; (t) an inside corner formed by a curvature of a radius of 0.2-5 mm; (u) the air delivery tube is configured to electrically connect with the reservoir dock; (v) the air delivery tube is configured to form the mechanical and electrical connections simultaneously when the air delivery tube is connected to the intermediate component; (w) the water reservoir includes an outlet tube providing an outlet for delivering a humidified flow of breathable gas to the air delivery tube, and the intermediate component comprises an inlet seal adapted to from a seal with the outlet tube of the water reservoir; (x) the intermediate component comprises an tubular body including an outlet end, and the air delivery tube comprises a dock connector including a radial lip seal adapted to form a seal with the outlet end to pneumatically connect the air delivery tube to the intermediate component; (y) the air delivery tube comprises a dock connector including retaining bumps adapted to engage within respective holes provided to the intermediate component to mechanically connect the air delivery tube to the intermediate component; and/or (z) a chassis coupled to the water reservoir dock and including a chassis opening, wherein a central axis of the port in the intermediate component is aligned with a central axis of the chassis opening on a first side of the chassis opening, and the sound sensor is positioned on a second side of the chassis opening less than 2 mm from the chassis opening.

An aspect of one form of the present technology relates to a respiratory treatment apparatus comprising: a pressure generator configured to generate a flow of air for treating a respiratory disorder; a transducer configured to generate a flow signal representing a property of the flow of air; an air delivery tube configured to pass the flow of air to a patient interface; an intermediate component removably coupled to the respiratory treatment apparatus, configured to connect the air delivery tube to the pressure generator, and comprises a sound port configured to facilitate propagation of sound outside of the intermediate component; a sound sensor disposed adjacent to the port of the intermediate component and configured to receive sound propagated into the intermediate component from the air delivery tube and the pressure generator; a controller configured to: control operation of the pressure generator; during operation of the pressure generator, receive the flow signal from the transducer and sound signal sensed by the sound sensor; analyse the received sounds signal; modify operation of the pressure generator based at least in part on the analysing and the flow signal.

In examples of the preceding aspects: (a) the intermediate component is configured to pneumatically connect the air delivery tube to the respiratory treatment apparatus, and to mechanically connect the air delivery tube to the respiratory treatment apparatus; (b) further comprises a chassis and including a chassis opening, wherein the port in the intermediate component is aligned with the chassis opening on a first side of the chassis opening, and the sound sensor is positioned on a second side of the chassis opening; (c) further comprising a port seal disposed around the port and configured to provide a peripheral sealing formation on an outside surface of the intermediate component; (d) the peripheral sealing formation includes a ridge configured to abut the surface of a chassis; (e) the ridge extend between 0.4 to 0.8 mm above an outside surface of the intermediate component; (f) the ridge is compressed against the surface of a chassis when the intermediate component is in an operative position and is configured to prevent the intermediate component to disassemble without sufficient force; (g) the peripheral sealing formation includes a lip configured to abut the surface of a chassis; (h) the lip extend between 0.4 to 0.8 mm above an outside surface of the intermediate component; (i) the lip extends from sides of the port and above at least a portion of the port, the portion of the lip extending above the port is deflected towards the port when the intermediate component is in an operative position and is configured to prevent the intermediate component to disassemble without sufficient force; (j) the lip extends from perimeter of the port at an angle above the port and toward a central axis of the port; (k) the port seal covers the inside surface of the port and includes a membrane configured to cover the port and to transmit sound from inside of the intermediate component to the outside of the intermediate component; (l) the membrane is impermeable to liquids and/or gases; (m) the controller is further configured to determine a type of air delivery tube coupled to the water reservoir based on the analysing, a type of patient interface coupled to the air delivery tube based on the analysing, and/or a type of patient interface coupled to the air delivery tube based on the analysing; (n) the intermediate component comprises an outlet end configured to connect the air delivery tube to the intermediate component and an inlet end configured to connect to the pressure generator, wherein a central axis of the inlet end is provided at an angle that is 90° or greater to a central axis of the outlet end; (o) an inside corner formed by the angle comprises a bellow including a span between opposite sides of the bellow that is equal to or less than twice a radius of the inside corner; an inside corner formed by a curvature with a radius of 0.2-5 mm; (p) the air delivery tube is configured to form the mechanical and electrical connections simultaneously when the air delivery tube is connected to the intermediate component; (q) the intermediate component comprises an tubular body including an outlet end, and the air delivery tube comprises a dock connector including a radial lip seal adapted to form a seal with the outlet end to pneumatically connect the air delivery tube to the intermediate component; (r) the air delivery tube comprises a dock connector including retaining bumps adapted to engage within respective holes provided to the intermediate component to mechanically connect the air delivery tube to the intermediate component; and/or (s) further comprising a chassis including a chassis opening, wherein a central axis of the port in the intermediate component is aligned with a central axis of the chassis opening on a first side of the chassis opening, and the sound sensor is positioned on a second side of the chassis opening less than 2 mm from the chassis opening.

An aspect of one form of the present technology relates to respiratory treatment apparatus including a source of a flow of air at positive pressure to ambient, a chassis or housing constructed and arranged to be fixed in location in use relative to the source, an inlet pneumatic connection structure for connecting to the source to receive sealably the flow of air at positive pressure from the source in use, a container to hold a body of water in use, the container being configured to direct the flow of air so that the flow of air contacts a surface of the body of water in use so that water vapour may transfer from the body of water to the flow of air in use to increase the absolute humidity of the flow of air, the container including a wall constructed at least in part from a material having a relatively high thermal conductivity, a heating element, a temperature sensor, a controller to control the heating element, and an outlet pneumatic connection structure to receive the flow of air with increased absolute humidity. The chassis or housing is configured to hold the container in location close relative to the heating element so that heat energy may transfer from the heating element to the body of water to increase the absolute humidity of the flow of air. The controller is constructed and arranged to energise the heating element to heat the water without boiling the water. The respiratory treatment apparatus includes a sealing arrangement so that in use the flow of air with increased absolute humidity received at the outlet pneumatic connection structure has a positive pressure with respect to ambient.

Another aspect of the present technology relates to a CPAP system including a humidifier, a patient interface, and an air delivery tube to deliver humidified air to the patient interface. In an example, the humidifier is integrated with an RPT device structured to produce a flow of air at positive pressure.

Another aspect of the present technology relates to a humidifier including a water reservoir including a cavity structured to hold a volume of water, and a water reservoir dock structured and arranged to receive the water reservoir in an operative position.

Another aspect of the present technology relates to an apparatus for humidifying a flow of breathable gas. The apparatus includes a water reservoir including a cavity structured to hold a volume of water, a water reservoir dock structured and arranged to receive the water reservoir in an operative position, and an air delivery tube configured to pass the flow of breathable gas that has been humidified in the water reservoir to a patient interface. The air delivery tube is structured and arranged to form a direct pneumatic seal with the water reservoir. The air delivery tube may include a sound port configured to facilitate propagation of sound outside of the air delivery tube and a port seal disposed around the sound port, configured to provide a peripheral sealing formation on an outside surface of the air delivery tube, and includes a sound permeable membrane configured to cover the port.

Another aspect of the present technology relates to an apparatus for humidifying a flow of breathable gas including a water reservoir including a cavity structured to hold a volume of water, a water reservoir dock structured and arranged to receive the water reservoir in an operative position, an air delivery tube configured to pass the flow of breathable gas that has been humidified in the water reservoir to a patient interface, and an intermediate component removably and non-rotatably coupled to the water reservoir dock. The intermediate component is configured to pneumatically connect the water reservoir to the air delivery tube. The intermediate component comprises a one-piece construction of a relatively rigid material including an inlet end adapted to interface with the water reservoir and an outlet end adapted to interface with the air delivery tube. The air delivery tube includes a dock connector structured and arranged to form a bayonet-style connection with the water reservoir dock which mechanically and electrically connects the air delivery tube with the water reservoir dock. The intermediate component including a sound port configured to facilitate propagation of sound outside of the intermediate component and a port seal disposed around the sound port, configured to provide a peripheral sealing formation on an outside surface of the intermediate component, and includes a sound permeable membrane configured to cover the port.

Another aspect of the present technology relates to an apparatus for humidifying a flow of breathable gas. The apparatus includes a water reservoir including a cavity structured to hold a volume of water, a water reservoir dock structured and arranged to receive the water reservoir in an operative position, an air delivery tube configured to pass the flow of breathable gas that has been humidified in the water reservoir to a patient interface, and an intermediate component removably and non-rotatably coupled to the water reservoir dock. The intermediate component is configured to pneumatically connect the air delivery tube to the water reservoir, and the intermediate component is configured to mechanically connect the air delivery tube to the reservoir dock. The intermediate component including a sound port configured to facilitate propagation of sound outside of the intermediate component and a port seal disposed around the sound port, configured to provide a peripheral sealing formation on an outside surface of the intermediate component, and includes a sound permeable membrane configured to cover the port.

One aspect of the present technology is a magnetic coupler that flexibly couples the microphone to the air circuit to improve sound quality and consistency in an acoustic component-identifying respiratory therapy system The magnetic coupler self-aligns to ensure consistency of the relative horizontal and vertical position of the microphone and the air circuit. The coupler also acts as a complete sealed path to minimise escape of sound to ambient. The flexible nature of the coupler damps some of the vibration of the device. The coupler may be a "bellows" type coupler to give added vertical flexibility. Optionally, to reduce transmission of vibration from PCBA to microphone, the microphone may be mounted on rubber feet on a tab on the PCBA partially surrounded by a cut-out channel.

In accordance with one aspect of the present technology, there is disclosed a coupler configured connect to a sensor and pass sound from a port in an air circuit of an RPT device to the sensor. In some aspects of the present technology, the sensor may be coupled to a circuit board disposed above the port; the circuit board includes a channel cut-out at least partially around a portion of circuit board supporting the sensor, the coupler includes a bellow, and/or the coupler provides a magnetic connection to the air circuit.

An aspect of one form of the present technology relates to an apparatus for treatment of a respiratory condition, the apparatus comprising: a pressure generator configured to generate a flow of breathable gas; an air delivery tube configured to pass the flow of breathable gas from the pressure generator to a patient interface; an intermediate component configured to pneumatically connect the air delivery tube to the pressure generator, the intermediate component comprising a port configured to facilitate propagation of sound outside of the intermediate component; a sensor disposed adjacent to the port of the intermediate component, the sensor configured to sense sound propagated through the port; a flexible coupler configured to connect to the sensor and pass sound from the port to the sensor; and a controller. The controller configured to: receive a sound signal generated by the sensor as a result of sensing sound during operation of the apparatus, analyse the received sound signal, and effect a response based at least in part on the analysing.

In examples of the preceding aspects: (a) the response comprises at least one of: recording a result of the analysing, displaying a result of the analysing, forwarding a result of the analysing, and controlling operation of the pressure generator based at least in part on the analysing; (b) the apparatus comprising a chassis including a chassis opening, wherein the port in the intermediate component is located on a first side of the chassis opening, and the sensor is positioned on a second side of the chassis opening; (c) the apparatus comprising a circuit board located on the second side of the chassis opening, wherein the sensor is coupled to the circuit board; (d) the apparatus comprising a circuit board to which the sensor is coupled, wherein the coupler includes an outlet end configured to directly couple to the sensor and an inlet end configured to removably couple to the intermediate component; (e) at least a portion of the outlet end is directly coupled to the circuit board; (f) the outlet end includes a plurality of rubber feet configured to connect the coupler to the circuit board; (g) the circuit board includes a channel cut through the circuit board and provided at least partially around a portion of the circuit board coupled to the sensor for isolating vibrations from the circuit board to the sensor; (h) the channel is provided at least partially around through holes on the circuit board electrically connected to the sensor; (i) the channel forms a tab, the tab configured to deflect in a vertical directions from the surface of the circuit board; (j) the inlet end includes a first connecting element configured to removably couple to a second connecting element connected to the intermediate component; (k) the first connecting element includes a magnet and/or the second connecting element includes a magnet; (l) one of the first connecting element or the second connecting element includes a metal ring; (m) the first connecting element has a ring shape and/or the second connecting element has a ring shape; (n) an inside diameter of the first connecting element is the same as an inside diameter of the second connecting element; (o) the second connecting element is disposed below a surface of the intermediate component around the port; (p) further comprising a membrane configured to cover the port and to transmit sound from inside of the intermediate component to the outside of the intermediate component; (q) the membrane is configured to cover an end of the coupler adjacent to the intermediate component; (r) the membrane is provided below an outside surface of the intermediate component (s) the coupler includes an outlet end configured to couple to the sensor and an inlet end configured to couple to the intermediate component, and the coupler includes one or more bellows disposed between the outlet end and the inlet end; (t) the one or more bellows are adapted to allow displacement of the inlet end relative to the outlet end in a horizontal direction and/or a vertical direction; (u) further comprising a circuit board to which the sensor is coupled, wherein the coupler is configured to directly engage the sensor without a direct connection to the circuit board; and/or (v) the air delivery tube configured to pass the flow of breathable gas from the pressure generator to a patient interface.

An aspect of one form of the present technology relates to an apparatus for treatment of a respiratory condition, the apparatus comprising: a pressure generator configured to generate a flow of breathable gas; an air delivery tube configured to pass the flow of breathable gas from the pressure generator to a patient interface; an intermediate component configured to pneumatically connect the air delivery tube to the pressure generator, the intermediate component comprising a port configured to facilitate propagation of sound outside of the intermediate component; a circuit board located adjacent to the port; a sensor assembly disposed between the circuit board and the port of the intermediate component, the sensor assembly including a sensor coupled to the circuit board and configured to sense sound propagated outside the intermediate component, and a first connecting element configured to removably couple to a second connecting element disposed at least partially around the port in the intermediate component; and a controller. The controller is configured to: receive a sound signal generated by the sensor as a result of sensing sound during operation of the apparatus, analyse the received sound signal, and effect a response based at least in part on the analysing.

In examples of the preceding aspects: (a) the sensor is directly coupled to the circuit board; (b) the sensor is disposed in a flexible housing including a plurality of rubber feet configured to couple the flexible housing to the circuit board; (c) an end of the sensor assembly adjacent to the port is configured to displace in a vertical direction and/or a horizontal direction when the end of the sensor assembly adjacent to the port is misaligned from the port when the intermediate component is in an assembled position; (d) the first connecting element is a magnetic ring and the second connecting element is a magnetic or metallic ring configured to removably couple to the magnetic ring in the first connecting element; (e) the circuit board includes a channel cut through the circuit board and provided at least partially around a portion of the circuit board connected to the sensor assembly for isolating vibrations from the circuit board to the sensor; (f) the channel is provided at least partially around through holes on the circuit board electrically connected to the sensor; (g) the channel forms a tab including the through holes, the tab configured to deflect in a vertical direction from the surface of the circuit board; (h) the second connecting element is disposed below a surface of the intermediate component around the port; (i) further comprising a membrane configured to cover the port at an inside surface of the intermediate component or at an outside surface of the intermediate component and to transmit sound from inside of the intermediate component to the outside of the intermediate component; and/or (j) the membrane is impermeable to liquids and/or gases.

An aspect of one form of the present technology relates to an apparatus for treatment of a respiratory condition, the apparatus comprising: a pressure generator configured to generate a flow of breathable gas, the pressure generator configured to pneumatically connect to an air delivery tube configured to pass the flow of breathable gas from the pressure generator to a patient interface, the air delivery tube comprising a port configured to facilitate propagation of sound outside of the air delivery tube; a sensor configured to sense sound propagated through the port when the pressure generator is connected to the air delivery tube; a flexible coupler configured to connect to the sensor and pass sound from the port to the sensor; and a controller. The controller configured to: receive a sound signal generated by the sensor as a result of sensing sound during operation of the apparatus, analyse the received sound signal, and effect a response based at least in part on the analysing.

In examples of the preceding aspects: (a) the response comprises at least one of: recording a result of the analysing, displaying a result of the analysing, forwarding a result of the analysing, and controlling operation of the pressure generator based at least in part on the analysing; (b) the apparatus comprising a chassis including a chassis opening, wherein the port is located on a first side of the chassis opening, and the sensor is positioned on a second side of the chassis opening; (c) the apparatus further comprising a circuit board located on the second side of the chassis opening, wherein the sensor is coupled to the circuit board; (d) the apparatus further comprising a circuit board to which the sensor is coupled, wherein the coupler includes an outlet end configured to directly couple to the sensor and an inlet end configured to removably couple to the air delivery tube; (e) at least a portion of the outlet end is directly coupled to the circuit board; (f) the outlet end includes a plurality of rubber feet configured to connect the coupler to the circuit board; (g) the circuit board includes a channel cut through the circuit board and provided at least partially around a portion of the circuit board coupled to the sensor for isolating vibrations from the circuit board to the sensor; (h) the channel is provided at least partially around through holes on the circuit board electrically connected to the sensor; (i) the channel forms a tab, the tab configured to deflect in a vertical direction from the surface of the circuit board; (j) the inlet end includes a first connecting element configured to removably couple to a second connecting element connected to the air delivery tube; (k) the first connecting element includes a magnet and/or the second connecting element includes a magnet; (l) one of the first connecting element or the second connecting element includes a metal ring; (m) the first connecting element has a ring shape and/or the second connecting element has a ring shape; (n) an inside diameter of the first connecting element is the same as an inside diameter of the second connecting element; (o) the second connecting element is disposed below a surface of the air delivery tube around the port; (p) the apparatus further comprising a membrane configured to cover the port and to transmit sound from inside of the delivery tube to the outside of the delivery tube; (q) the membrane is configured to cover an end of the coupler adjacent to the delivery tube; (r) the membrane is provided below an outside surface of the delivery tube; (s) the coupler includes an outlet end configured to couple to the sensor and an inlet end configured to couple to the delivery tube, and the coupler includes one or more bellows disposed between the outlet end and the inlet end; (t) the one or more bellows are adapted to allow displacement of the inlet end relative to the outlet end in a horizontal direction and/or a vertical direction; (u) the apparatus further comprising a circuit board to which the sensor is coupled, wherein the coupler is configured to directly engage the sensor without a direct connection to the circuit board; and/or (v) the apparatus further comprising the air delivery tube.

An aspect of one form of the present technology relates to an apparatus for treatment of a respiratory condition, the apparatus comprising: a pressure generator configured to generate a flow of breathable gas; an intermediate component pneumatically connected to an air delivery tube, the intermediate component comprising a port configured to facilitate propagation of sound outside of the intermediate component; a sensor attached externally to the intermediate component and located adjacent to the port of the intermediate component, the sensor configured to sense sound propagated through the air delivery tube; and a controller configured to: receive a sound signal generated by the sensor as a result of sensing sound during operation of the apparatus, analyse the received sound signal, and effect a response based at least in part on the analysing.

In examples of the preceding aspects: (a) the intermediate component is configured to pneumatically connect an air delivery tube to the pressure generator; (b) the response comprises at least one of: recording a result of the analysing, displaying a result of the analysing, forwarding a result of the analysing, and controlling operation of the pressure generator based at least in part on the analysing; (c) the apparatus further comprises a chassis including a chassis opening, wherein the port in the intermediate component is located on a first side of the chassis opening, and the sensor is positioned on a second side of the chassis opening; (d) the apparatus further comprises a circuit board located on the second side of the chassis opening, wherein the sensor is coupled to the circuit board; (e) the apparatus further comprises a flexible coupler configured to pass sound from the port to the sensor; (f) the apparatus further comprises a circuit board to which the sensor is coupled, wherein the coupler includes an outlet end configured to directly engage the sensor and an inlet end configured to removably engage the intermediate component; (g) one end of the coupler is removably engaged with (or is at least in contact with) the intermediate component and/or the other one is engaged with (or is at least in contact with) the sensor; (h) at least a portion of the outlet end is in contact with the circuit board; (i) the outlet end includes an end cap; (j) the end cap includes one or more feet configured to contact the circuit board and/or one or more connecting ports for connecting the sensor to the circuit board; (k) the circuit board includes a channel cut through the circuit board and provided at least partially around a portion of the circuit board coupled to the sensor for isolating vibrations from the circuit board to the sensor; (l) the channel is provided at least partially around through holes on the circuit board electrically connected to the sensor; (m) the channel forms a tab, the tab configured to deflect in a transverse direction to the surface of the circuit board; (n) the inlet end includes a first connecting element configured to removably couple to a second connecting element connected to the intermediate component; (o) the first connecting element includes a magnet and/or the second connecting element includes a magnet; (p) one of the first connecting element or the second connecting element includes a metal ring; (q) the first connecting element has a ring shape and/or the second connecting element has a ring shape; (r) the second connecting element is disposed below a surface of the intermediate component around the port; (s) the coupler includes one or more bellows disposed between the outlet end and the inlet end; (t) the one or more bellows are adapted to allow displacement of the inlet end relative to the outlet end in a horizontal direction and/or a vertical direction; (u) the coupler is configured to directly contact the sensor without a direct connection to the circuit board; (v) the apparatus further comprises the air delivery tube configured to pass the flow of breathable gas from the pressure generator to a patient interface; (w) the apparatus further comprises a membrane configured to cover the port and to transmit sound from inside of the intermediate component to the outside of the intermediate component; (x) the membrane is provided below an outside surface of the intermediate component; (y) the apparatus comprises at least one of a membrane and a port seal, the membrane configured to cover the port and to transmit sound from inside of the intermediate component to the outside of the intermediate component, and the port seal being disposed so as to provide a sealing engagement between the port and the chassis opening when the intermediate component is in its operational configuration; (z) the apparatus further comprises a water reservoir including a cavity structured to hold a volume of water and receive the flow of breathable gas so that the flow of breathable gas is humidified before being passed to the patient interface; and a water reservoir dock structured and arranged to receive the water reservoir in an operative position, wherein the intermediate component is removably coupled to the water reservoir dock so as to receive a humidified flow of breathable gas and pass it to the air delivery tube; (aa) the intermediate component is of a generally tubular shape and the water reservoir dock includes a generally tubular opening for receiving the intermediate component, the intermediate component and the generally tubular opening being configured for a generally frictionless insertion of the intermediate component into the opening; (ab) at least one of the intermediate component and the generally tubular opening comprises at least one engagement formation arranged so that, during an insertion of the intermediate component into the opening, an engagement of at least one of the engagement formations brings the intermediate component into its operational configuration in which at least one of the following is effected: a sealing engagement of the port seal with the chassis opening; and supporting engagement effected by the at least one engagement formation is configured to impede dislodgement of the intermediate component from its operational configuration in the absence of an appreciable external force; (ac) the engagement of at least one of the engagement formations occurs at a later point of an insertion path; (ad) one or more of the engagement formations includes an elevation feature causing an upward movement of at least a portion of the intermediate component; (ae) the elevation feature is provided on a bottom of the tubular opening and pushes the intermediate component upwards to reduce a clearance between a top of the tubular opening and the intermediate component after the intermediate component is inserted a predetermined distance into the tubular opening; (af) each of at least one of the intermediate component and the generally tubular opening comprising a plurality of engagement formations, each of the engagement formations arranged to engage approximately simultaneously during the insertion of the intermediate component into the opening; (ag) the apparatus further comprises a port seal configured to surround the port, wherein the port seal includes a peripheral sealing formation including a ridge configured to abut a surface of the chassis around the chassis opening, when the intermediate component is coupled to a water reservoir dock; (ah) the apparatus further comprises a port seal configured to surround the port, wherein the port seal includes a peripheral sealing formation including a lip configured to abut a surface of a chassis around the chassis opening, when the intermediate component is coupled to a humidifier; (ai) wherein the lip extends from perimeter of the port at an angle above the port and toward a central axis of the port; (aj) wherein the port seal covers an inside surface of the port and includes the membrane; (ak) wherein the membrane is coplanar with at least an inside or an outside surface of the intermediate component; (al) wherein the membrane is impermeable to liquids and/or gases; (am) wherein the controller is configured to determine a characteristic of the air delivery tube or a patient interface, based on the analysing; (an) wherein the controller is further configured to determine a type or a size of the air delivery tube or a type or a size of a patient interface coupled to the air delivery tube, based on the analysing; (ao) wherein the intermediate component comprises an outlet end configured to connect the air delivery tube to the intermediate component and an inlet end configured to connect the water reservoir to the intermediate component, wherein an air path between the inlet end and the outlet end is nonlinear and includes at least one turn, wherein at least the turn closest to the port is curved; (ap) a central axis of the inlet end is substantially transverse to a central axis of the outlet end, defining corresponding transverse air paths, an outer and an inner corner, each corner comprising a rounded inner surface; (aq) the transverse air path between the inlet end cross adjacent the inlet end, the inlet end includes an inlet seal adapted to interface with the water reservoir; (ar) an inside corner comprises a bellow including a span between opposite sides of the bellow that is equal to or less than twice a radius of the inside corner and/or an inner surface of the inside corner comprises a curvature of a radius of 0.2-5 mm; (as) the intermediate component is configured to pneumatically connect the air delivery tube to the water reservoir, and to mechanically connect the air delivery tube to the water reservoir dock; (at) the air delivery tube is configured to form a mechanical and electrical connections simultaneously when the air delivery tube is connected to the intermediate component; (au) the apparatus further comprises a transducer configured to generate a flow signal representing a property of flow of air, wherein the controller is configured to: control operation of the pressure generator; and during operation of the pressure generator: receive the flow signal from the transducer and sound signal sensed by the sensor; analyse the received sounds signal; and modify operation of the pressure generator based at least in part on the analysing and the flow signal; (av) further comprising: a chassis including a chassis opening extending through the chassis, wherein the port in the intermediate component is located adjacent to a first end of the chassis opening; and a circuit board located adjacent to a second end of the chassis opening, wherein the sensor is positioned on the circuit board and aligned with the chassis opening; (aw) the second end of the chassis opening includes a larger opening than an opening of the first end; (ax) the second end of the chassis opening is provided by a side wall extending from a surface of the chassis facing the circuit board; (ay) wherein the sensor is positioned at least partially inside of the chassis opening and/or the side wall; (az) further comprising a seal disposed between the chassis and the circuit board and adjacent the second end of the chassis opening; (aaa) the seal includes a peripheral sealing formation including a lip or a ridge configured to abut a surface of the circuit board adjacent the sensor; the peripheral sealing formation may extend from a surface of the seal facing the circuit board and surrounding the second end of the chassis opening; (aab) further comprising a generally tubular opening in a chassis of the apparatus for receiving the intermediate component of a generally tubular shape, and the intermediate component comprises an inlet end adapted to be inserted into the opening, an outlet end adapted to interface with the air delivery tube, a flange arranged between the inlet end and the outlet end, and one or more flexible bumpers provided adjacent to a side of the flange facing the inlet end (e.g for softening abutment with a wall of the tubular opening during insertion and for absorbing vibration in use); (aac) the intermediate component further comprises a barbed tab at one end of the intermediate component opposite to the outlet end, wherein the barbed tab is structured to provide a snap-fit connection with a locking member of the chassis and the one or more bumpers are depressed against a portion of the chassis by the flange during the snap-fit connection and force the barbed tab against the locking member to impede disengagement of the barbed tab from the locking member in the absence of an appreciable external force; (aad) during initial insertion of the intermediate component into the generally tubular opening, minimal resistance is provided between the intermediate component and the generally tubular opening, and resistance is increased at a later stage of the insertion at a location where one or more engagement features of the intermediate component engage respective engagement features in the generally tubular opening of the chassis, the engagement between respective engagement features of the intermediate component and the chassis opening directing the intermediate component into its operational engagement configuration with the opening; (aae) further comprising a port seal disposed so as to provide a sealing engagement between the port and the chassis opening in an operative position, wherein during a later stage of insertion of the intermediate component into the generally tubular opening, an interaction between respective engagement features of the intermediate component and the chassis opening positions the intermediate component to ensure a sealing engagement between the port seal and a surface of the chassis around the chassis opening (e.g., when the intermediate component is in its operative position); (aaf) further comprising a water reservoir including a cavity structured to hold a volume of water and receive the flow of breathable gas so that the flow of breathable gas is humidified before being passed to the patient interface; and a water reservoir dock structured and arranged to receive the water reservoir in the operative position, wherein the inlet end of the intermediate component is removably coupled to the water reservoir dock so as to receive a humidified flow of breathable gas and pass it to the air delivery tube, the inlet end includes an inlet seal, and in the operative position, the port seal provides sealing engagement between the port and the chassis opening and the inlet seal provides a sealing engagement between the inlet end and the water reservoir dock; (aag) the intermediate component further comprises a guide rib on an outside surface of the intermediate component and/or a guide rail on the outside surface of the intermediate component, the guide rib and the guide rail are structured and arranged to assist in alignment and insertion of the intermediate component into the generally tubular opening in a chassis by engagement with corresponding guide slots extending into the generally tubular opening in the chassis; (aah) the guide rib is provided on forward and upper side of the intermediate component and the guide rail is provided on a lower side of the intermediate component; (aai) the chassis includes a generally tubular opening for receiving the intermediate component of a generally tubular shape, and the intermediate component comprises an inlet end adapted to be inserted into the opening and an outlet end adapted to interface with the air delivery tube, wherein a clearance between the port seal and the chassis near the chassis opening is provided during insertion of the intermediate component into the opening and the port seal begins to engage the chassis after an edge of the port passes a central axis or an edge of the chassis opening during insertion of the intermediate component into the opening; (aaj) after the port seal begins to engage the chassis, the intermediate component is further inserted a predetermined distance to bring the intermediate component into its operational configuration; (aak) further comprising a flexible housing overmoulded on the sensor that is displaced from the circuit board at least partially past the chassis opening; and/or (aal) the pressure generator, the intermediate component, the controller and the sensor are commonly housed by a housing of the apparatus.

An aspect of one form of the present technology relates to an apparatus for treatment of a respiratory condition, the apparatus comprising: a pressure generator configured to generate a flow of breathable gas; an air delivery tube configured to connect to the pressure generator and comprising a port configured to facilitate propagation of sound outside of the air delivery tube; a sensor disposed outside of the air delivery tube and adjacent to the port of the air delivery tube, the sensor configured to sense sound propagated through the port; and a controller configured to: receive a sound signal generated by the sensor as a result of sensing sound during operation of the apparatus, analyse the received sound signal, and effect a response based at least in part on the analysing.

In examples of the preceding aspects: (a) the response comprises at least one of: recording a result of the analysing, displaying a result of the analysing, forwarding a result of the analysing, and controlling operation of the pressure generator based at least in part on the analysing; (b) the apparatus further comprises a chassis including a chassis opening, wherein the port is located on a first side of the chassis opening, and the sensor is positioned on a second side of the chassis opening; (c) the apparatus further comprises a circuit board located on the second side of the chassis opening, wherein the sensor is coupled to the circuit board; (d) the apparatus further comprises a flexible coupler configured to pass sound from the port to the sensor; (e) the apparatus further comprises a circuit board to which the sensor is coupled, wherein the coupler includes an outlet end configured to directly engage the sensor and an inlet end configured to removably engage the air delivery tube; (f) one end of the coupler is removably coupled to the air delivery tube and/or the other one is coupled to the sensor; (f) at least a portion of the outlet end is in contact with the circuit board; (g) the outlet end includes an end cap; (g) the end cap includes one or more feet configured to contact the circuit board and/or one or more connecting ports for connecting the sensor to the circuit board; (h) the circuit board includes a channel cut through the circuit board and provided at least partially around a portion of the circuit board coupled to the sensor for isolating vibrations from the circuit board to the sensor; (i) the channel is provided at least partially around through holes on the circuit board electrically connected to the sensor; (j) the channel forms a tab, the tab configured to deflect in a transverse direction to the surface of the circuit board; (k) the inlet end includes a first connecting element configured to removably couple to a second connecting element connected to the air delivery tube; (l) the first connecting element includes a magnet and/or the second connecting element includes a magnet; (m) one of the first connecting element or the second connecting element includes a metal ring; (n) the first connecting element has a ring shape and/or the second connecting element has a ring shape; (o) the second connecting element is disposed below a surface of the air delivery tube around the port; (p) the coupler includes one or more bellows disposed between the outlet end and the inlet end; (q) the one or more bellows are adapted to allow displacement of the inlet end relative to the outlet end in a horizontal direction and/or a vertical direction; (r) the coupler is configured to directly contact the sensor without a direct connection to the circuit board; (s) the apparatus further comprises the air delivery tube configured to pass the flow of breathable gas from the pressure generator to a patient interface; (t) the apparatus further comprises a membrane configured to cover the port and to transmit sound from inside of the air delivery tube to the outside of the air delivery tube; (u) the membrane is provided below an outside surface of the air delivery tube; (v) the apparatus comprises at least one of a membrane and a port seal, the membrane configured to cover the port and to transmit sound from inside of the air delivery tube to the outside of the air delivery tube, and the port seal being disposed so as to provide a sealing engagement between the port and the chassis opening; (w) the apparatus further comprises a water reservoir including a cavity structured to hold a volume of water and receive the flow of breathable gas so that the flow of breathable gas is humidified before being passed to a patient interface; and a water reservoir dock structured and arranged to receive the water reservoir in an operative position, wherein the air delivery tube is removably coupled to the water reservoir dock so as to receive a humidified flow of breathable gas and pass it to the air delivery tube; (y) the air delivery tube comprises at least one engagement formation arranged so that, during an insertion of the air delivery tube into an opening, proximate an end of an insertion path, an engagement of at least one of the engagement formations brings the air delivery tube into its operational configuration in which the port seal seals both the port and a chassis opening, and wherein at least one of, a sealing engagement of the port seal with the port and chassis opening, and supporting engagement effected by the at least one engagement formation, is configured to impede dislodgement of the air delivery tube from its operational configuration in the absence of an appreciable external force; (z) one or more of the engagement formations includes an elevation feature; (aa) the apparatus further comprises a port seal configured to surround the port, wherein the port seal includes a peripheral sealing formation including a ridge configured to abut a surface of the chassis around the chassis opening, when the air delivery tube is coupled to a water reservoir dock; (ab) the apparatus further comprises a port seal configured to surround the port, wherein the port seal includes a peripheral sealing formation including a lip configured to abut a surface of a chassis around the chassis opening, when the air delivery tube is coupled to a humidifier; (ac) wherein the lip extends from perimeter of the port at an angle above the port and toward a central axis of the port; (ad) wherein the port seal covers an inside surface of the port and includes the membrane; (ae) wherein the membrane is coplanar with at least an inside or an outside surface of the air delivery tube; (af) wherein the membrane is impermeable to liquids and/or gases; (ag) wherein the controller is configured to determine a characteristic of the air delivery tube or a patient interface, based on the analysing; (ah) wherein the controller is further configured to determine a type or a size of the air delivery tube or a type or a size of a patient interface coupled to the air delivery tube, based on the analysing; and/or (ai) the apparatus further comprises a transducer configured to generate a flow signal representing a property of flow of air, wherein the controller is configured to: control operation of the pressure generator; and during operation of the pressure generator: receive the flow signal from the transducer and sound signal sensed by the sensor; analyse the received sounds signal; and modify operation of the pressure generator based at least in part on the analysing and the flow signal.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

4 BRIEF DESCRIPTION OF THE DRAWINGS

The present technology is illustrated by way of example, and not by way of limitation, in the figures of the accompanying drawings, in which like reference numerals refer to similar elements including:

4.1 TREATMENT SYSTEMS

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

4.2 RESPIRATORY SYSTEM AND FACIAL ANATOMY

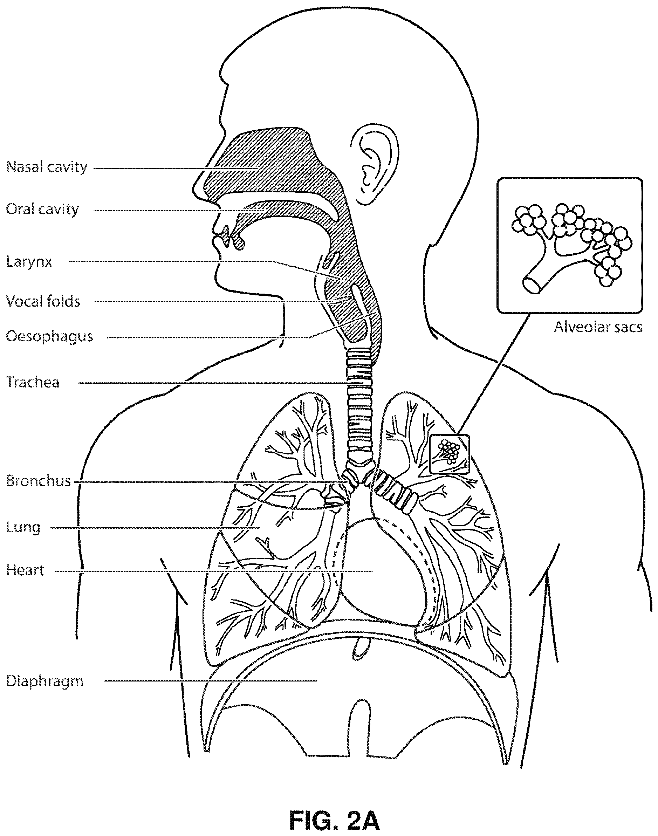

Fig. 2A shows an overview of a human respiratory system including the nasal and oral cavities, the larynx, vocal folds, oesophagus, trachea, bronchus, lung, alveolar sacs, heart and diaphragm.

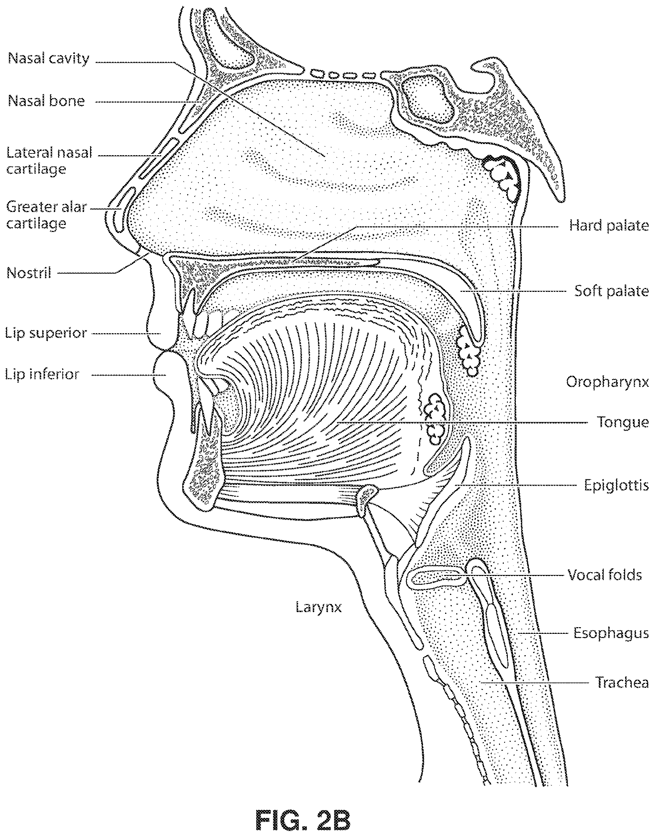

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

4.3 PATIENT INTERFACE

Fig. 3A shows a patient interface in the form of a nasal mask in accordance with one form of the present technology.

Fig. 3B shows a schematic of a cross-section through a structure at a point. An outward normal at the point is indicated. The curvature at the point has a positive sign, and a relatively large magnitude when compared to the magnitude of the curvature shown in Fig. 3C.

Fig. 3C shows a schematic of a cross-section through a structure at a point. An outward normal at the point is indicated. The curvature at the point has a positive sign, and a relatively small magnitude when compared to the magnitude of the curvature shown in Fig. 3B.

Fig. 3D shows a schematic of a cross-section through a structure at a point. An outward normal at the point is indicated. The curvature at the point has a value of zero.

Fig. 3E shows a schematic of a cross-section through a structure at a point. An outward normal at the point is indicated. The curvature at the point has a negative sign, and a relatively small magnitude when compared to the magnitude of the curvature shown in Fig. 3F.

Fig. 3F shows a schematic of a cross-section through a structure at a point. An outward normal at the point is indicated. The curvature at the point has a negative sign, and a relatively large magnitude when compared to the magnitude of the curvature shown in Fig. 3E.

Fig. 3G shows the surface of a structure, with a one dimensional hole in the surface. The illustrated plane curve forms the boundary of a one dimensional hole.

Fig. 3H shows a cross-section through the structure of Fig. 3G. The illustrated surface bounds a two dimensional hole in the structure of Fig. 3G.

Fig. 3I shows a perspective view of the structure of Fig. 3G, including the two dimensional hole and the one dimensional hole. Also shown is the surface that bounds a two dimensional hole in the structure of Fig. 3G.

4.4 BREATHING WAVEFORMS

Fig. 4 shows a model typical breath waveform of a person while sleeping.

4.5 RPT DEVICE AND HUMIDIFIER

Fig. 5A shows an exploded perspective view of an RPT device 4000 in accordance with one form of the present technology.

Fig. 5B shows a perspective view of an RPT device 4000 comprising an outlet cap with a muffler 4124 in accordance with one form of the present technology.

Fig. 5C shows a perspective view of an RPT device 4000 with an integrated humidifier 5000 comprising a water reservoir 5110 in accordance with one form of the present technology.

Fig. 5D is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

Fig. 5E is a schematic diagram of the electrical components of an RPT device in accordance with one form of the present technology.

Fig. 5F is a schematic diagram of the algorithms implemented in an RPT device in accordance with one form of the present technology.

Fig. 5G shows a schematic of a humidifier in accordance with one form of the present technology.

Fig. 6A is a perspective view of an integrated RPT device and humidifier comprising a water reservoir according to an example of the present technology.

Fig. 6B is a perspective view of the integrated RPT device and humidifier of Fig. 6A with the water reservoir removed from the reservoir dock.

Fig. 7 is a perspective view of a pneumatic block according to an example of the present technology.

Fig. 8 is an exploded view of a water reservoir including a circular metal plate according to an example of the present technology.

Fig. 9 is a perspective view showing a reservoir dock, an intermediate component, and an air delivery tube according to an example of the present technology, the air delivery tube oriented for engagement with the intermediate component and a locking and contact assembly provided to the reservoir dock.

Fig. 10 is a perspective view showing the reservoir dock and the air delivery tube of Fig. 9, the air delivery tube engaged with the locking and contact assembly provided to the reservoir dock in an unlocked, engaged position.

Fig. 11 is a perspective view showing the reservoir dock and the air delivery tube of Fig. 9, the air delivery tube engaged with the locking and contact assembly provided to the reservoir dock in a locked position.

Fig. 12 is a perspective view showing the reservoir dock, the intermediate component, and the air delivery tube of Fig. 9.

Fig. 13 is an exploded view showing the reservoir dock, the intermediate component, the air delivery tube, and the locking and contact assembly of the reservoir dock of Fig. 9.

Fig. 14 is another exploded view showing the reservoir dock, the intermediate component, the air delivery tube, and the locking and contact assembly of the reservoir dock of Fig. 9.

Fig. 15 is an exploded view showing the reservoir dock and the locking and contact assembly thereof, the intermediate component, and the air delivery tube of Fig. 9.

Fig. 16 is an enlarged top perspective view of the reservoir dock of Fig. 9, including the dock outlet and the locking and contact assembly.

Fig. 17 is an enlarged perspective view showing the locking and contact assembly provided to the reservoir dock of Fig. 9, without the intermediate component.

Fig. 18 is another enlarged perspective view showing the locking and contact assembly provided to the reservoir dock of Fig. 9, without the intermediate component.

Fig. 19 is a rear perspective view showing an intermediate component according to an example of the present technology.

Fig. 20 is a front view of the intermediate component of Fig. 19.

Fig. 21 is a top view of the intermediate component of Fig. 19.

Fig. 22 is an exploded view of the intermediate component of Fig. 19.

Fig. 23 is a perspective view showing a reservoir dock, an intermediate component, and an air delivery tube according to an example of the present technology, the air delivery tube oriented for engagement with the intermediate component and a contact assembly provided to the reservoir dock.

Fig. 24 is a perspective view showing the reservoir dock, the intermediate component and the air delivery tube of Fig. 23, the air delivery tube engaged with the intermediate component and the contact assembly provided to the reservoir dock in a locked position.

Fig. 25 is a perspective view showing the reservoir dock and the engaged intermediate component of Fig. 23.

Fig. 26 is an exploded view showing the reservoir dock, the intermediate component, and the air delivery tube of Fig. 23.

Fig. 27 is a perspective view showing the dock outlet of the reservoir dock of Fig. 23 with the intermediate component removed.

Fig. 28A is a perspective view showing the intermediate component and the contact assembly provided to the reservoir dock of Fig. 23.

Fig. 28B is a cross-sectional view taken along a line indicated in Fig. 25, showing connection of the intermediate component to the reservoir dock according to an example of the present technology.

Fig. 28C is a cross sectional view taken along a line indicated in Fig. 26, of the intermediate component (without the lower tab 9795), according to an example of the present technology.

Fig. 28D is another cross-sectional view taken along a line indicated in Fig. 25, showing connection of the intermediate component to the reservoir dock according to an example of the present technology.

Fig. 28E is another cross-sectional view taking along a line indicated in Fig. 28D, showing connection of the intermediate component to the reservoir dock according to an example of the present technology.

Fig. 28F is another cross-sectional view taken along a line indicated in Fig. 28D, showing connection of the intermediate component to the reservoir dock and connection of a pinch arm to a chassis according to an example of the present technology.

Fig. 28G is another cross-sectional view taken along a line indicated in Fig. 28F, showing connection of a pinch arm to a chassis according to an example of the present technology.

Fig. 29 is a top perspective view of an intermediate component according to an example of the present technology.

Fig. 30 is a bottom perspective view of the intermediate component of Fig. 29.

Fig. 31 is a front view of the intermediate component of Fig. 29.

Fig. 32 is a top plan view of the intermediate component of Fig. 29.

Fig. 33 is an exploded view of the intermediate component of Fig. 29.

Fig. 34A is a perspective view of an intermediate component including a ridge sealing formation on a sound port according to an example of the present technology.

Fig. 34B is a partial cross sectional view taken along line AA indicated in Fig. 23, of an intermediate component, when including the ridge sealing formation of Fig. 34A aligned against a corresponding chassis sound opening, according to an example of the present technology.

Fig. 34C is a side cross sectional view taken along a line indicated in Fig. 34A, of a ridge sealing formation according to an example of the present technology.

Fig. 34D is a side cross sectional view of the ridge sealing formation of Fig. 34C, being compressed against a chassis according to an example of the present technology.

Fig. 35A is a perspective view of an intermediate component including a lip sealing formation on a sound port according to an example of the present technology.

Fig. 35B is a partial cross sectional view taken along line AA indicated in Fig. 23, of an intermediate component, when including the lip sealing formation of Fig. 35A aligned against a corresponding chassis sound opening, according to an example of the present technology.

Fig. 35C is a side cross sectional view taken along a line indicated in Fig. 35A, of a lip sealing formation according to an example of the present technology.

Fig. 35D is a side cross sectional view of the lip sealing formation of Fig. 35C, being compressed against a chassis according to an example of the present technology.

Fig. 36A shows a side cross-sectional view taken along line AA indicated in Fig. 23, of an intermediate component being inserted into the receiving chassis opening before the port seal engages the chassis according to an example of the present technology.

Fig. 36B shows the side cross-sectional view of the intermediate component of Fig. 36A, when being inserted further into the receiving chassis opening so that the port seal starts to engage the chassis, according to an example of the present technology.

Fig. 36C shows a side cross-sectional view of the intermediate component of Fig. 36A, when being fully inserted into the receiving chassis opening, with the port seal having engaged the chassis according to an example of the present technology.

Fig. 36D shows a side cross-sectional view of the intermediate component of Fig. 29, when being fully inserted into the receiving chassis opening, with the port seal having engaged the chassis according to an example of the present technology.

Fig. 37 illustrates example components of a system for detecting sound signals according to the present technology.

Fig. 38A illustrates a perspective view of a coupler coupled between a circuit board and an intermediate component according to an example of the present technology.

Fig. 38B illustrates an exploded view of a coupler coupled between a circuit board and an intermediate component, shown in Fig. 38A, according to an example of the present technology.

Fig. 38C illustrates a coupler coupled to an intermediate component shown in Fig. 38A, according to an example of the present technology.

Fig. 38D illustrates a second connected element coupled to an intermediate component according to an example of the present technology.

Fig. 39A illustrates a cross-sectional view of a coupler between a circuit board and an intermediate component according to an example of the present technology.

Fig. 39B illustrates an exploded view of a coupler between a circuit board and an intermediate component, shown in Fig. 38A, according to an example of the present technology.

Fig. 39C illustrates another exploded view of a circuit board, a sensor, a coupler, and a first connecting element according to an example of the present technology.

Fig. 39D illustrates a side view of a circuit board, a sensor, a coupler, and a first connecting element shown in Fig. 39C.

Fig. 40A illustrates escape of sound that may occur without including a coupler between a sensor and an intermediate component.

Fig. 40B illustrates escape of sound that may occur in a system including a coupler between a sensor and an intermediate component according to an example of the present technology.

Fig. 41A illustrates a rubber interface for coupling a sensor to a circuit board according to an example of the present technology.

Fig. 41B illustrates another view of the rubber interface for coupling a sensor to a circuit board shown in Fig. 41A according to an example of the present technology.

Fig. 41C illustrates an assembly including a circuit board, intermediate component and a rubber interface for removably coupling a sensor to the intermediate component according to an example of the present technology.

Fig. 42A illustrates a circuit board including a cut-out channel according to an example of the present technology.

Fig. 42B illustrates an exploded view of an assembly including a circuit board shown in Fig. 42A, a sensor, a connecting element, and an intermediate component according to an example of the present technology.

Fig. 42C illustrates a perspective view of the assembled circuit board, sensor, connecting element, and intermediate component shown in Fig. 42B according to an example of the present technology.

Fig. 43 shows a sensor disposed in a flexible housing according to an example of the present technology.

Fig. 44 illustrates an example methodology for acoustic detection and analysis according to the present technology.

## 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

## 5.1 THERAPY

In one form, the present technology comprises a method for treating a respiratory disorder comprising the step of applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

## 5.2 TREATMENT SYSTEMS

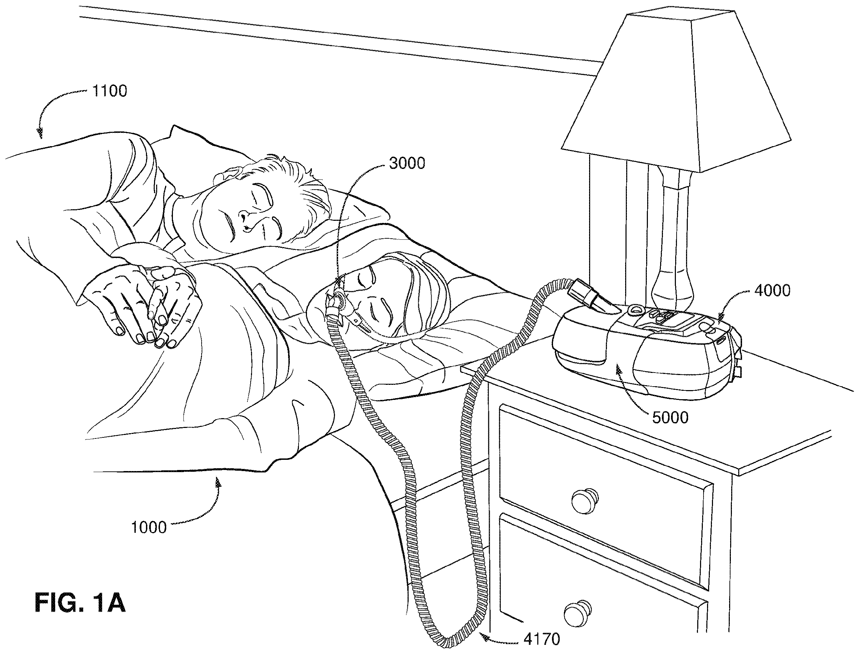

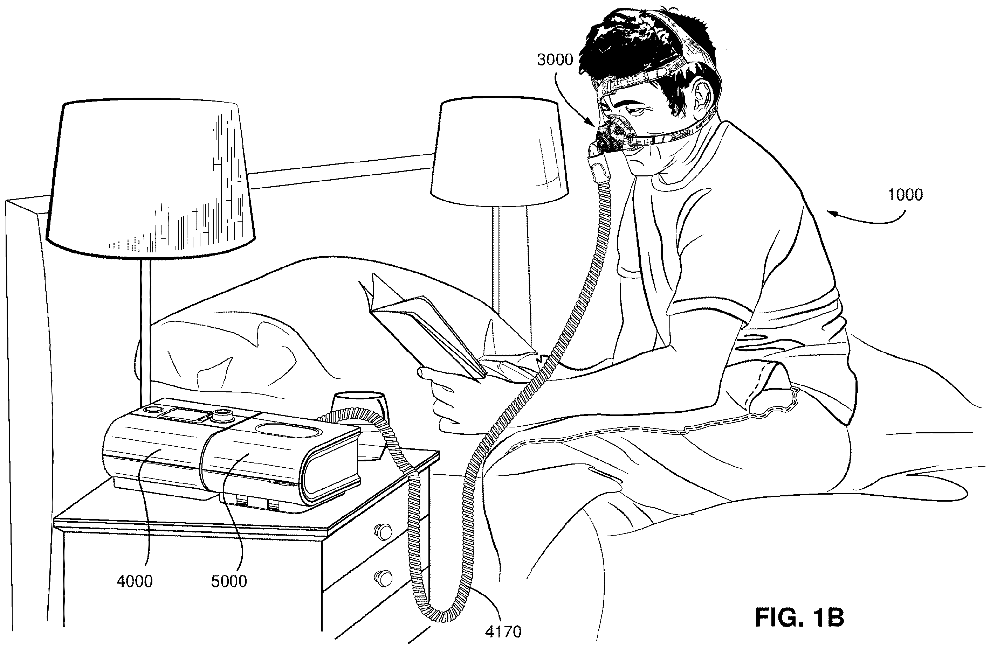

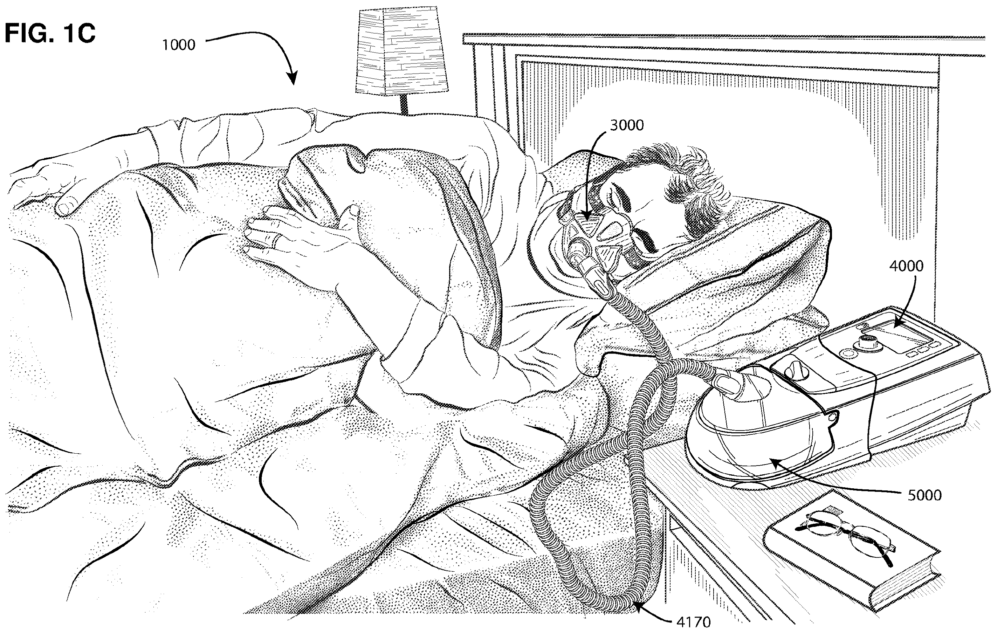

In one form, the present technology comprises an apparatus or device for treating a respiratory disorder. The apparatus or device may comprise an RPT device 4000 for supplying pressurised air to the patient 1000 via an air circuit 4170 to a patient interface 3000, e.g., see Figs. 1A to 1C.

5.3 PATIENT INTERFACE

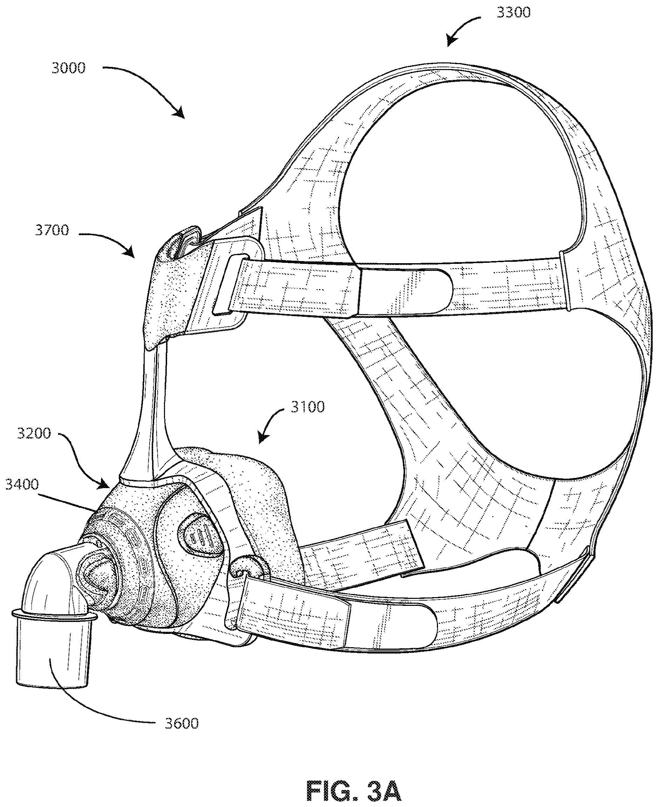

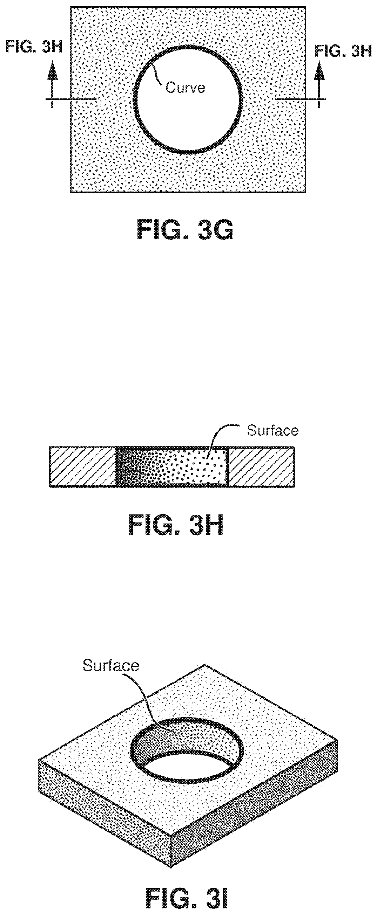

Fig. 3A shows a non-invasive patient interface 3000 in accordance with one aspect of the present technology comprising the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to facilitate the supply of air at positive pressure to the airways.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH$_2$O with respect to ambient.

The patient interface 3000 in accordance with one form of the present technology is constructed and arranged to be able to provide a supply of air at a positive pressure of at least 10 cmH$_2$O with respect to ambient.

The patient interface 3000 in accordance with one form of the present technology is constructed and arranged to be able to provide a supply of air at a positive pressure of at least 20 cmH$_2$O with respect to ambient.

5.4 RPT DEVICE

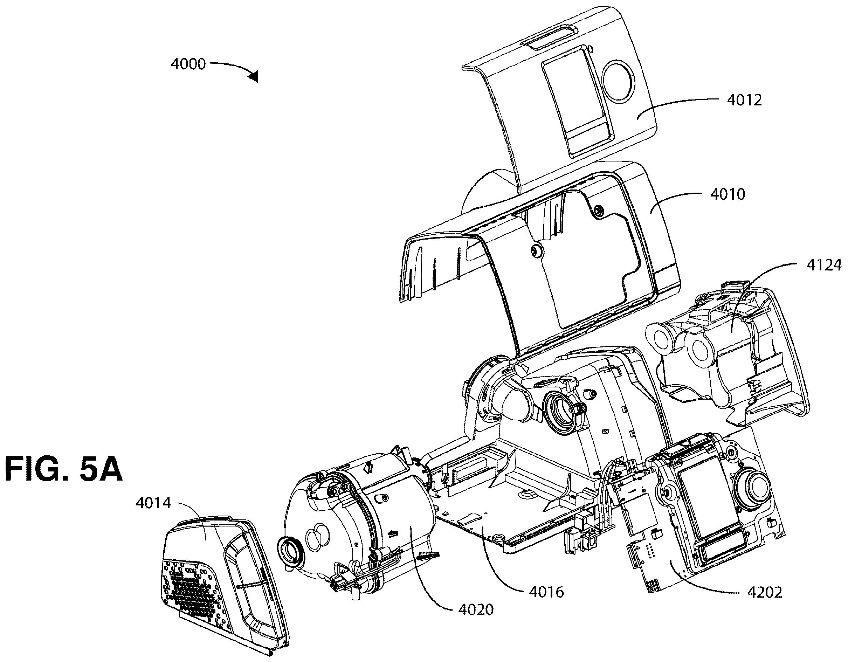

An exploded view of an RPT device 4000 in accordance with one aspect of the present technology is shown in Fig. 5A. An RPT device 4000 may comprise mechanical, pneumatic, and/or electrical components and be configured to execute one or more algorithms. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH$_2$O, or at least 10cmH$_2$O, or at least 20 cmH$_2$O.

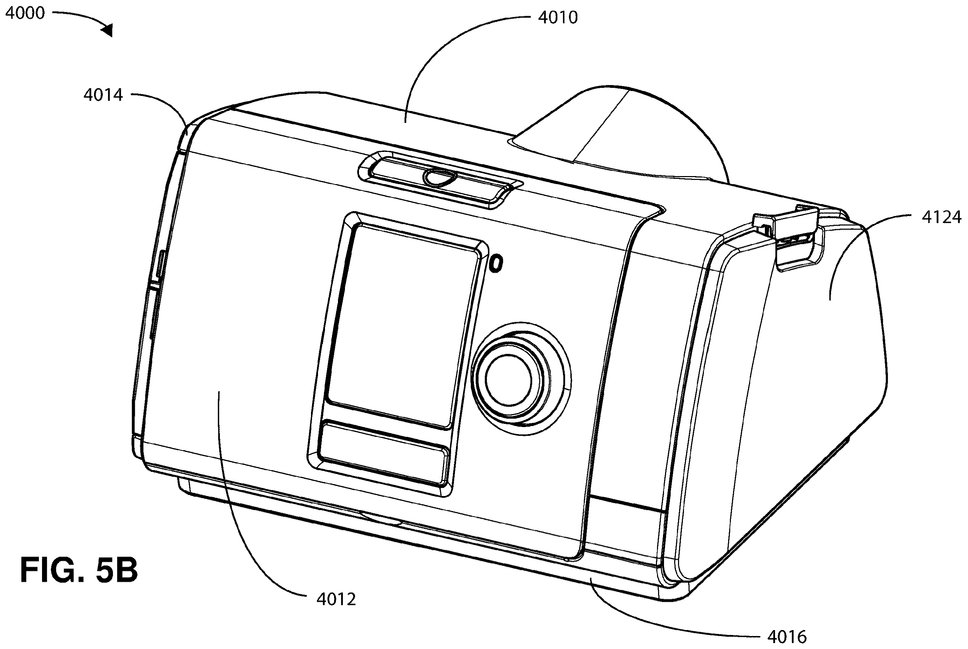

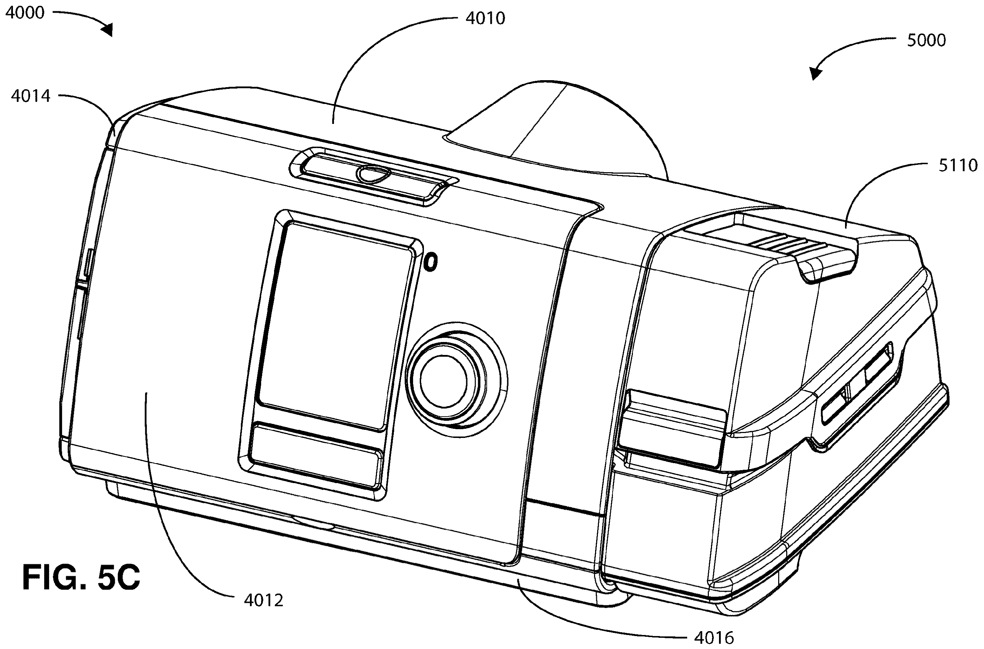

The RPT device 4000 may include an external housing having one or more panel(s) such as a main panel (e.g., external housing 4010), a front panel 4012 and a side panel 4014. The RPT device 4000 may also comprise an outlet cap with a muffler 4124 as shown in Figs. 5A and 5B. The outlet cap with a muffler 4124 may be removable and replaced with a water reservoir 5110 (see Fig. 5C). In such forms, the RPT device 4000 may be considered to include an integrated humidifier 5000. Thus, the RPT device 4000 may be used with or without humidification depending upon whether the water reservoir 5110 or the outlet cap with a muffler 4124 respectively is attached. The structure and operation of muffler 4124 is similar to that of the end cap muffler described in WO 2015/089582, which is incorporated herein by reference in its entirety. Preferably the RPT device 4000 comprises a chassis 4016 that supports one or more internal components of the RPT device 4000. In one form the RPT device 4000 comprises a pressure generator 4140, which may be housed in a pneumatic block 4020 coupled to the chassis 4016.

Further examples and details of an exemplary RPT device are described in PCT Publication No. WO 2015/089582.

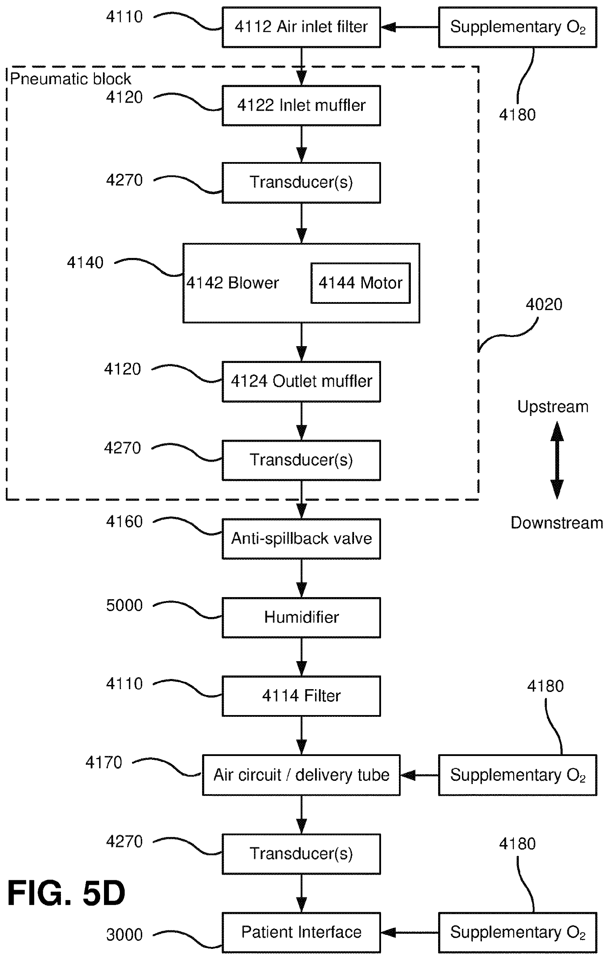

The pneumatic path of the RPT device 4000 (e.g. shown in Fig. 5D) may comprise an inlet air filter 4112, an inlet muffler 4122, a pressure generator 4140 capable of supplying air at positive pressure (preferably a blower 4142) and an outlet muffler 4124 (or a water reservoir 5110 if humidification is required). One or more sensors or transducers 4270, such as pressure sensors and flow sensors may be included in the pneumatic path. The pneumatic path may also include anti-spill back valve 4160 to prevent water from the humidifier 5000 spilling back to the electrical components of the RPT device 4000.

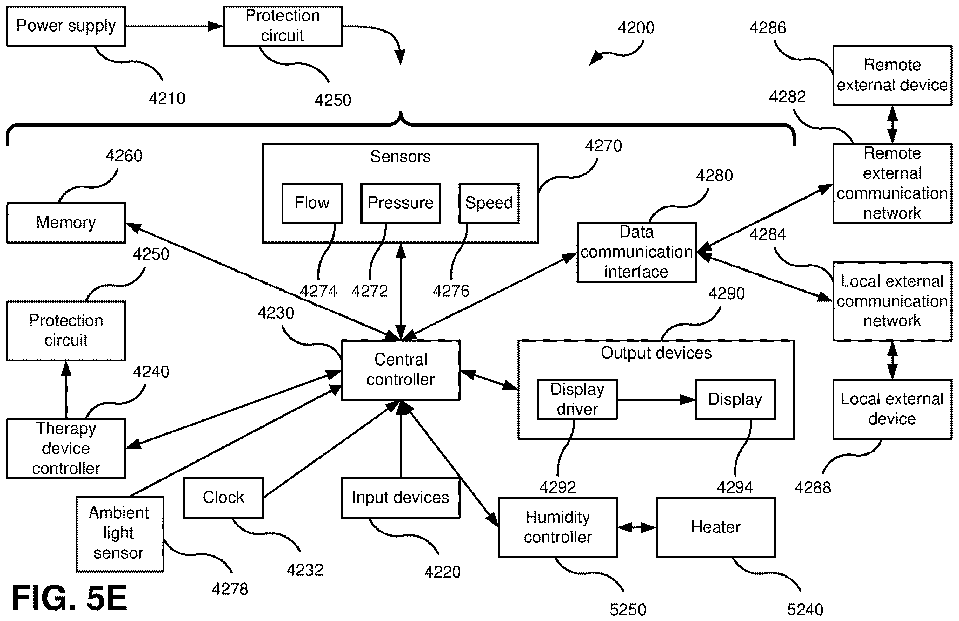

As shown in Fig. 5E, the RPT device 4000 may have an electrical power supply 4210, one or more input devices 4220, a central controller 4230, a therapy device controller 4240, one or more protection circuits 4250, memory 4260, sensors/transducers 4270, data communication interface 4280 and one or more output devices 4290. Electrical components 4200 may be mounted on a single Printed Circuit Board Assembly (PCBA) 4202 (e.g., see Fig. 5A). In an alternative form, the RPT device 4000 may include more than one PCBA 4202.

5.4.1 RPT device mechanical & pneumatic components

An RPT device may comprise one or more of the following components in an integral unit. In an alternative form, one or more of the following components may be located as respective separate units.

**5.4.1.1 Air filter(s)**

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 5.4.1.3 Pressure generator

In one form of the present technology, a pressure generator 4140 for producing a flow, or a supply, of air at positive pressure is a controllable blower 4142. For example the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH$_2$O to about 20 cmH$_2$O, or in other forms up to about 30 cmH$_2$O. The blower may be as described in any one of the following patents or patent applications the contents of which are incorporated herein by reference in their entirety: U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing sound in the RPT device and/or properties of the flow of air such as a flow rate, a pressure or a temperature at that point in the pneumatic path.

In one form of the present technology, one or more transducers 4270 may be located proximate to the patient interface 3000.

In one form of the present technology, one or more transducers 4270 may be located in the RPT device.

In one form, a signal from a transducer 4270 may be filtered, such as by low-pass, high-pass or band-pass filtering.

#### 5.4.1.4.1 Flow rate sensor

A flow rate sensor 4274 in accordance with the present technology may be based on a differential pressure transducer, for example, an SDP600 Series differential pressure transducer from SENSIRION.

In one form, a signal representing a flow rate from the flow rate sensor 4274 is received by the central controller 4230.

#### 5.4.1.4.2 Pressure sensor

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal from the pressure sensor 4272 is received by the central controller 4230.

### 5.4.1.4.3 Motor speed transducer

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 5.4.1.4.4 Ambient light sensor 4278

As the RPT device 4000 is often used in a bedroom environment, for example to be used while the patient 1000 is about to go to sleep, or is asleep, it may be important to ensure that any light-emitting features of the RPT device 4000 is not excessively bright.

In one form of the present technology an ambient light sensor 4278 is used to determine the light level in the ambient area around the RPT device 4000. An ambient light signal from the ambient light sensor 4278 may be provided as an input to the central controller 4230, for example to adjust a brightness of a display or any other light-emitting features, such as a backlight for input devices 4220 or any notification lights.

The display 4294 may be configured to operate at one of a plurality of predetermined brightness settings. The brightness setting may be chosen according to a signal output of the ambient light sensor 4278.

### 5.4.1.4.5 Sound sensor

In one form of the present technology the transducer 4270 may include a sound sensor, which may be a microphone, configured to generate a signal representing sound in the RPT device. The sound sensor may be configured to transform sound in the audible range to a patient and/or sound in an inaudible range into electrical signals. The sound sensor may generate an analog or a digital signal.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

### 5.4.2.3 Central controller

In one form of the present technology, the central controller 4230 is one or a plurality of processors suitable to control an RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

### 5.4.2.4 Clock

The RPT device 4000 may include a clock 4232 that is connected to the central controller 4230.

### 5.4.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

### 5.4.2.6 Protection circuits

The one or more protection circuits 4250 in accordance with the present technology may comprise an electrical protection circuit, a temperature and/or pressure safety circuit.

### 5.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 5.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 5.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

#### *5.4.2.9.1 Display driver*

A display driver 4292 receives as an input the characters, symbols, or images intended for display on the display 4294, and converts them to commands that cause the display 4294 to display those characters, symbols, or images.

#### *5.4.2.9.2 Display*

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 5.4.3 RPT device algorithms

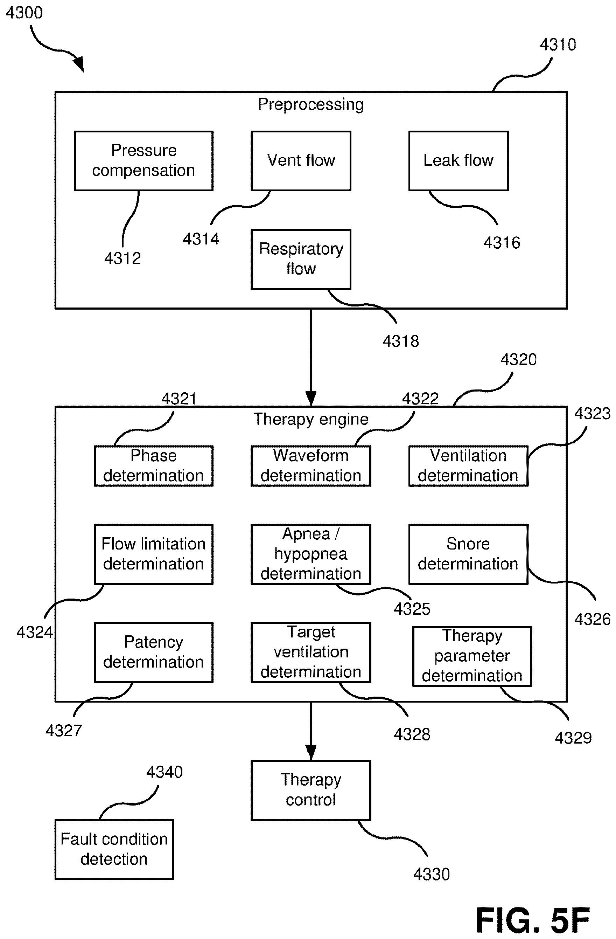

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules, e.g., see Fig. 5F.

### 5.4.3.1 Pre-processing module

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface or mask pressure *Pm,* the respiratory flow rate *Qr,* and the leak flow rate *Ql.*

In various forms of the present technology, the pre-processing module 4310 comprises one or more of the following algorithms: pressure compensation 4312, vent flow rate estimation 4314, leak flow rate estimation 4316, and respiratory flow rate estimation 4318.

#### *5.4.3.1.1 Pressure compensation*

In one form of the present technology, a pressure compensation algorithm 4312 receives as an input a signal indicative of the pressure in the pneumatic path proximal to an outlet of the pneumatic block. The pressure compensation algorithm 4312 estimates the pressure drop through the air circuit 4170 and provides as an output an estimated pressure, *Pm,* in the patient interface 3000.

#### *5.4.3.1.2 Vent flow rate estimation*

In one form of the present technology, a vent flow rate estimation algorithm 4314 receives as an input an estimated pressure, *Pm,* in the patient interface 3000 and estimates a vent flow rate of air, *Qv,* from a vent 3400 in a patient interface 3000.

*5.4.3.1.3 Leak flow rate estimation*

In one form of the present technology, a leak flow rate estimation algorithm 4316 receives as an input a total flow rate, $Qt$, and a vent flow rate $Qv$, and provides as an output an estimate of the leak flow rate $Ql$. In one form, the leak flow rate estimation algorithm estimates the leak flow rate $Ql$ by calculating an average of the difference between total flow rate $Qt$ and vent flow rate $Qv$ over a period sufficiently long to include several breathing cycles, e.g. about 10 seconds.

In one form, the leak flow rate estimation algorithm 4316 receives as an input a total flow rate $Qt$, a vent flow rate $Qv$, and an estimated pressure, $Pm$, in the patient interface 3000, and provides as an output a leak flow rate $Ql$, by calculating a leak conductance, and determining a leak flow rate $Ql$ to be a function of leak conductance and pressure, $Pm$. Leak conductance is calculated as the quotient of low pass filtered non-vent flow rate equal to the difference between total flow rate $Qt$ and vent flow rate $Qv$, and low pass filtered square root of pressure $Pm$, where the low pass filter time constant has a value sufficiently long to include several breathing cycles, e.g. about 10 seconds. The leak flow rate $Ql$ may be estimated as the product of leak conductance and a function of pressure, $Pm$.

*5.4.3.1.4 Respiratory flow rate estimation*

In one form of the present technology, a respiratory flow rate estimation algorithm 4318 receives as an input a total flow rate, $Qt$, a vent flow rate, $Qv$, and a leak flow rate, $Ql$, and estimates a respiratory flow rate of *air, Qr*, to the patient, by subtracting the vent flow rate $Qv$ and the leak flow rate $Ql$ from the total flow rate $Qt$.

**5.4.3.2 Therapy Engine Module**

In one form of the present technology, a therapy engine module 4320 receives as inputs one or more of a pressure, $Pm$, in a patient interface 3000, and a respiratory flow rate of air to a patient, $Qr$, and provides as an output one or more therapy parameters.

In one form of the present technology, a therapy parameter is a treatment pressure $Pt$.

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

*5.4.3.2.1 Phase determination*

In one form of the present technology, the RPT device 4000 does not determine phase.

In one form of the present technology, a phase determination algorithm 4321 receives as an input a signal indicative of respiratory flow rate, $Qr$, and provides as an output a phase $\Phi$ of a current breathing cycle of a patient 1000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate $Qr$ has a value that exceeds a positive threshold, and a discrete value of 0.5 revolutions (thereby "cycling" the RPT device 4000) when a respiratory flow rate $Qr$ has a value that is more negative than a negative threshold. The inhalation time $Ti$ and the exhalation time $Te$ may be estimated as typical values over many respiratory cycles of the time spent with phase $\Phi$ equal to 0 (indicating inspiration) and 0.5 (indicating expiration) respectively.

Another implementation of discrete phase determination provides a tri-valued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate $Qr$. A continuous value of phase determined in this implementation is often referred to as "fuzzy phase". In one implementation of a fuzzy phase determination algorithm 4321, the following rules are applied to the respiratory flow rate *Qr*:

1. If the respiratory flow rate is zero and increasing fast then the phase is 0 revolutions.

2. If the respiratory flow rate is large positive and steady then the phase is 0.25 revolutions.

3. If the respiratory flow rate is zero and falling fast, then the phase is 0.5 revolutions.

4. If the respiratory flow rate is large negative and steady then the phase is 0.75 revolutions.

5. If the respiratory flow rate is zero and steady and the 5-second low-pass filtered absolute value of the respiratory flow rate is large then the phase is 0.9 revolutions.

6. If the respiratory flow rate is positive and the phase is expiratory, then the phase is 0 revolutions.

7. If the respiratory flow rate is negative and the phase is inspiratory, then the phase is 0.5 revolutions.

8. If the 5-second low-pass filtered absolute value of the respiratory flow rate is large, the phase is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase $\Phi$ is first discretely estimated from the respiratory flow rate *Qr* as described above, as are the inhalation time *Ti* and the exhalation time *Te*. The continuous phase $\Phi$ at any instant may be determined as the half the proportion of the inhalation time *Ti* that has elapsed since the previous trigger instant, or 0.5 revolutions plus half the proportion of the exhalation time *Te* that has elapsed since the previous cycle instant (whichever instant was more recent).

### 5.4.3.2.2 Waveform determination

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure *Pt* that varies as a function of phase $\Phi$ of a respiratory cycle of a patient according to a waveform template $\Pi(\Phi)$.

In one form of the present technology, a waveform determination algorithm 4322 provides a waveform template $\Pi(\Phi)$ with values in the range [0, 1] on the domain of phase values $\Phi$ provided by the phase determination algorithm 4321 to be used by the therapy parameter determination algorithm 4329.

In one form, suitable for either discrete or continuously-valued phase, the waveform template $\Pi(\Phi)$ is a square-wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template $\Pi(\Phi)$ is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template $\Pi(\Phi)$ from a library of waveform templates, dependent on a setting of the RPT device. Each waveform template $\Pi(\Phi)$ in the library may be provided as a lookup table of values $\Pi$ against phase values $\Phi$. In other forms, the waveform determination algorithm 4322 computes a waveform template $\Pi(\Phi)$ "on the fly" using a predetermined functional form, possibly parametrised by one or more parameters (e.g. time constant of an exponentially curved portion). The parameters of the functional form may be predetermined or dependent on a current state of the patient 1000.

In some forms of the present technology, suitable for discrete bi-valued phase of either inhalation ($\Phi = 0$ revolutions) or exhalation ($\Phi = 0.5$ revolutions), the waveform determination algorithm 4322 computes a waveform template $\Pi$ "on the fly" as a function of both discrete phase $\Phi$ and time *t* measured since the most recent trigger instant. In one such form, the waveform determination algorithm 4322 computes the waveform template $\Pi(\Phi, t)$ in two portions (inspiratory and expiratory) as follows:

$$\Pi(\Phi, t) = \begin{cases} \Pi_i(t), & \Phi = 0 \\ \Pi_e(t - T_i), & \Phi = 0.5 \end{cases}$$

where $\Pi_i(t)$ and $\Pi_e(t)$ are inspiratory and expiratory portions of the waveform template $\Pi(\Phi, t)$. In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

### 5.4.3.2.3 Ventilation determination

In one form of the present technology, a ventilation determination algorithm 4323 receives an input a respiratory flow rate $Qr$, and determines a measure indicative of current patient ventilation, *Vent.*

In some implementations, the ventilation determination algorithm 4323 determines a measure of ventilation *Vent* that is an estimate of actual patient ventilation. One such implementation is to take half the absolute value of respiratory flow rate, $Qr$, optionally filtered by low-pass filter such as a second order Bessel low-pass filter with a corner frequency of 0.11 Hz.

In other implementations, the ventilation determination algorithm 4323 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate $Qr$ produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle $K$ proportion (by time) of the inspiratory portion, where $0 < K < 1$. There is an arbitrarily large number of measures that are exactly proportional to ventilation if the flow rate shape is constant.

### 5.4.3.2.4 Determination of Inspiratory Flow limitation

In one form of the present technology, the central controller 4230 executes an inspiratory flow limitation determination algorithm 4324 for the determination of the extent of inspiratory flow limitation.

In one form, the inspiratory flow limitation determination algorithm 4324 receives as an input a respiratory flow rate signal $Qr$ and provides as an output a metric of the extent to which the inspiratory portion of the breath exhibits inspiratory flow limitation.

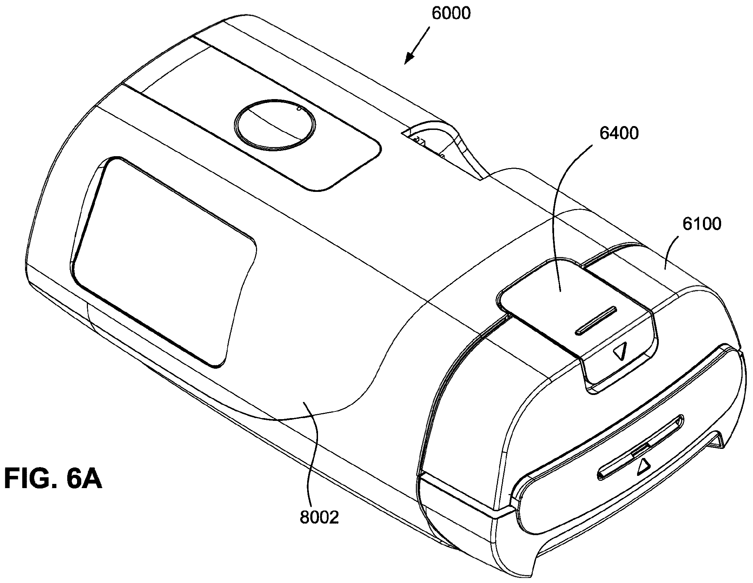

In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath, similar to the inspiratory portion of the breath shown in Fig. 6A. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the central controller 4230 for the preceding several inspiratory events. This is repeated over the same inspiratory events for the second such point, and so on. Thus, for example, sixty five scaled data points are generated by the central controller 4230, and represent a moving average of the preceding several inspiratory events, e.g., three events. The moving average of continuously updated values of the (e.g., sixty five) points are hereinafter called the "scaled flow rate ", designated as $Qs(t)$. Alternatively, a single inspiratory event can be utilised rather than a moving average.

From the scaled flow rate, two shape factors relating to the determination of partial obstruction may be calculated.

Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.

Shape factor 2 is calculated as the RMS deviation from unit scaled flow rate, taken over the middle (e.g. thirty two) points. An RMS deviation of about 0.2 units is taken to be normal. An RMS deviation of zero is taken to be a totally flow-limited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can be other than those described.

### 5.4.3.2.5 Determination of apneas and hypopneas

In one form of the present technology, the central controller 4230 executes an apnea / hypopnea determination algorithm 4325 for the determination of the presence of apneas and/or hypopneas.

In one form, the apnea / hypopnea determination algorithm 4325 receives as an input a respiratory flow rate signal $Qr$ and provides as an output a flag that indicates that an apnea or a hypopnea has been detected.

In one form, an apnea will be said to have been detected when a function of respiratory flow rate $Qr$ falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate $Qr$ falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

### 5.4.3.2.6 Determination of snore

In one form of the present technology, the central controller 4230 executes one or more snore determination algorithms 4326 for the determination of the extent of snore.

In one form, the snore determination algorithm 4326 receives as an input a respiratory flow rate signal $Qr$ and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal $Qr$ to reduce background noise, e.g., the sound of airflow in the system from the blower.

### 5.4.3.2.7 Determination of airway patency

In one form of the present technology, the central controller 4230 executes one or more airway patency determination algorithms 4327 for the determination of the extent of airway patency.

In one form, the airway patency determination algorithm 4327 receives as an input a respiratory flow rate signal $Qr,$ and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure $Pt.$ In one implementation, the forced oscillation is of frequency 2 Hz with amplitude about 1 cmH$_2$O.

In one form, airway patency determination algorithm 4327 receives as an input a respiratory flow rate signal $Qr$, and determines the presence or absence of a cardiogenic signal. The absence of a cardiogenic signal is taken to be an indication of a closed airway.

### 5.4.3.2.8 Determination of target ventilation

In one form of the present technology, the central controller 4230 takes as input the measure of current ventilation, *Vent,* and executes one or more target ventilation determination algorithms 4328 for the determination of a target value *Vtgt* for the measure of ventilation.

In some forms of the present technology, there is no target ventilation determination algorithm 4328, and the target value *Vtgt* is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value *Vtgt* from a value *Vtyp* indicative of the typical recent ventilation of the patient.

In some forms of adaptive servo-ventilation, the target ventilation *Vtgt* is computed as a high proportion of, but less than, the typical recent ventilation *Vtyp.* The high proportion in such forms may be in the range (80%, 100%), or (85%, 95%), or (87%, 92%).

In other forms of adaptive servo-ventilation, the target ventilation *Vtgt* is computed as a slightly greater than unity multiple of the typical recent ventilation *Vtyp.*

The typical recent ventilation *Vtyp* is the value around which the distribution of the measure of current ventilation *Vent* over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation *Vtyp* from the measure of current ventilation, *Vent.* One such measure is the output of a low-pass filter on the measure of current ventilation *Vent,* with time constant equal to one hundred seconds.

### 5.4.3.2.9 Determination of therapy parameters

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure *Pt.* In one implementation of this form, the therapy parameter determination algorithm 4329 determines the treatment pressure *Pt* using the equation

$$Pt = A\Pi(\Phi, t) + P_0 \qquad (1)$$

where:

- *A* is the amplitude,
- $\Pi(\Phi, t)$ is the waveform template value (in the range 0 to 1) at the current value $\Phi$ of phase and *t* of time, and
- $P_0$ is a base pressure.

If the waveform determination algorithm 4322 provides the waveform template $\Pi(\Phi, t)$ as a lookup table of values $\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude A and the base pressure $P_0$ may be set by the therapy parameter determination algorithm 4329 depending on the chosen respiratory pressure therapy mode in the manner described below.

### 5.4.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of air whose mask pressure *Pm* at the patient interface 3000 is equal to the treatment pressure *Pt.*

### 5.4.3.4 Detection of fault conditions

In one form of the present technology, the central controller 4230 executes one or more methods (fault condition detection algorithm 4340) for the detection of fault conditions. The fault conditions detected by the one or more methods may include at least one of the following:

- Power failure (no power, or insufficient power)
- Transducer fault detection
- Failure to detect the presence of a component
- Operating parameters outside recommended ranges (e.g. pressure, flow rate, temperature, $PaO_2$)

- Failure of a test alarm to generate a detectable alarm signal.

Upon detection of the fault condition, the corresponding fault condition detection algorithm 4340 signals the presence of the fault by one or more of the following:

- Initiation of an audible, visual &/or kinetic (e.g. vibrating) alarm
- Sending a message to an external device
- Logging of the incident

## 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated herewithin in its entirety by reference.

## 5.6 OXYGEN DELIVERY

In one form of the present technology, supplemental oxygen 4180 is delivered to one or more points in the pneumatic path, such as upstream of the pneumatic block 4020, to the air circuit 4170 and/or to the patient interface 3000.

## 5.7 HUMIDIFIER

### 5.7.1 Humidifier overview

In one form of the present technology there is provided a humidifier 5000 (e.g., as shown in Fig. 5C) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

### 5.7.2 RPT Device and Humidifier

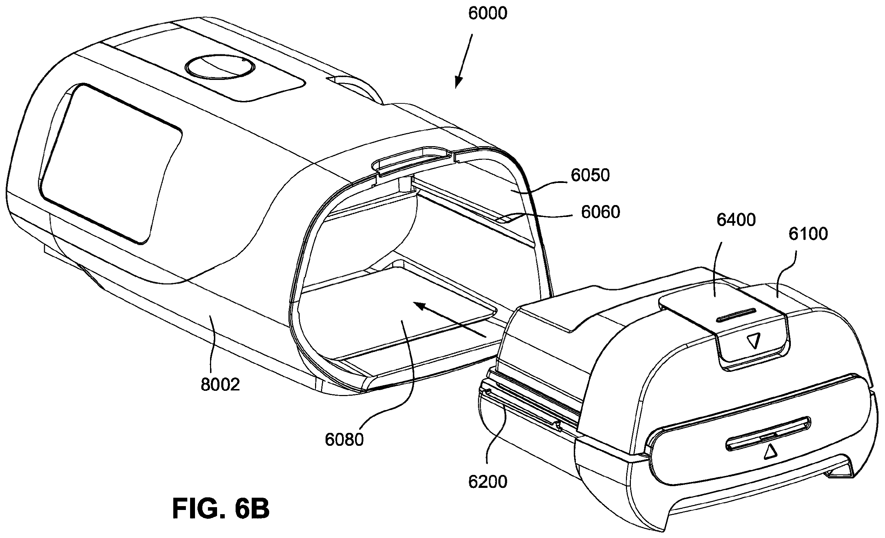

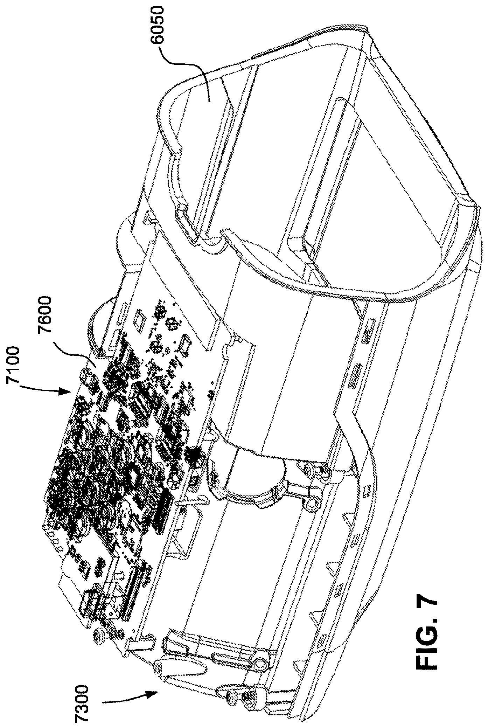

Figs. 6A, 6B, and 7 illustrate an integrated RPT device and humidifier 6000 according to an example of the present technology. As illustrated, the integrated RPT device and humidifier 6000 includes a reservoir dock 6050 structured and arranged to receive a water reservoir 6100 (also referred to as a humidifier tub or a humidifier reservoir). In the illustrated example, the integrated RPT device and humidifier 6000 comprises a humidifier that is integrated with an RPT device such that a pneumatic block 7100 of the RPT device comprises components that perform the function of the RPT device as well as components that perform the function of the humidifier. For example, as shown in Fig. 7, the reservoir dock 6050 is integrated with the pneumatic block 7100 of the RPT device to provide an integral unit, with the reservoir dock 6050 structured and arranged to receive the water reservoir 6100.

It should be appreciated that the humidifier (e.g., reservoir dock 6050) may be provided separately to the RPT device (e.g., pneumatic block 7100) in an alternative arrangement. In such arrangement, additional interfaces may be used to connect the humidifier (e.g., reservoir dock 6050) to the RPT device (e.g., pneumatic block 7100).

The RPT device comprises a blower supported within the pneumatic block 7100. The blower is structured and arranged for producing a flow, or a supply, of air at positive pressure, e.g., in the range of 2-50 cmH$_2$O. In an example, the blower may include a single stage design or a multi-stage design, e.g., two or more stage designs. The blower is operable to draw a supply of air into the pneumatic block 7100, e.g., through one or more intake openings in the pneumatic block, and into an inlet thereof (blower inlet), and provide a pressurized supply of air at an outlet (blower outlet). Examples and details of an exemplary blower are described in PCT Patent Application Publication No. WO 2013/020167, which is incorporated herein by reference in its entirety. The blower outlet is communicated with the humidifier, e.g., an inlet of the water reservoir 6100.

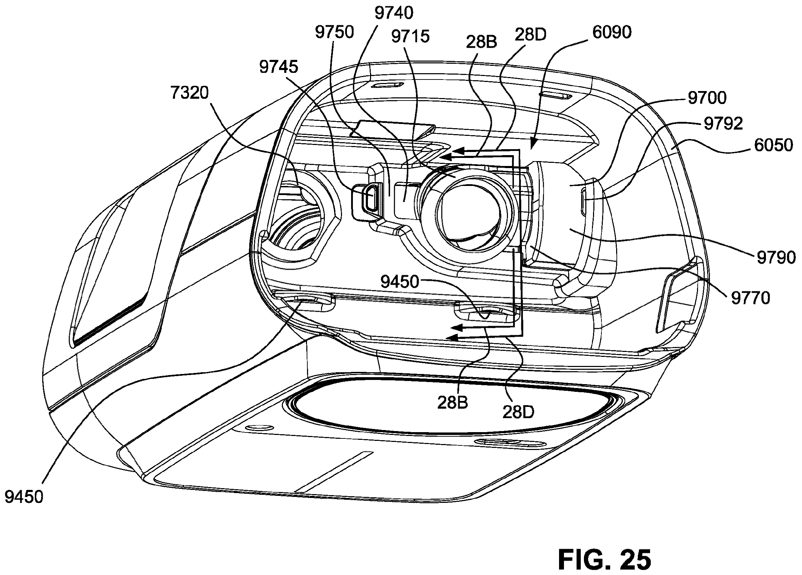

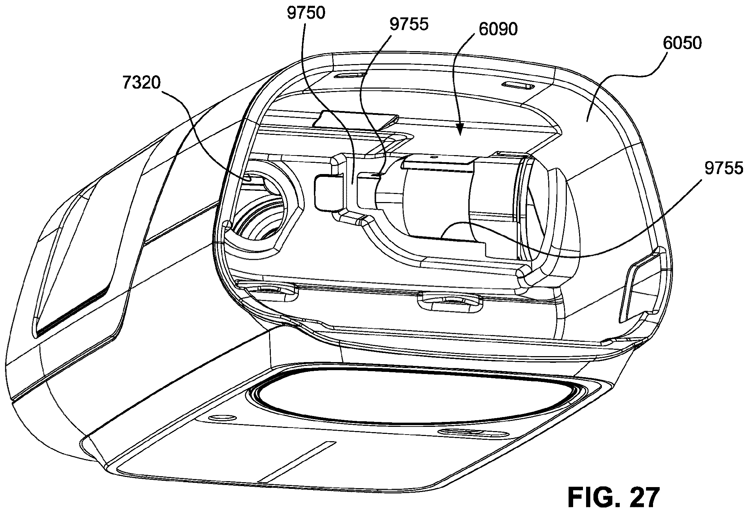

The pneumatic block 7100 includes a chassis assembly 7300, e.g., including a top chassis and a bottom chassis. The chassis assembly 7300 includes a chassis inlet (not shown) and a chassis outlet 7320 (e.g., see Figs. 25 and 27). In an example, an external housing, including one or more panels and/or one or more user inputs/displays, may enclose the pneumatic block 7100, e.g., see Figs. 6A and 6B. The chassis assembly 7300 supports and/or houses internal components of the pneumatic block 7100, e.g., the blower. The chassis assembly 7300 also supports a printed circuit board assembly (PCBA) 7600, which may include one or more components and of features described with reference to PCBA 4202. The chassis assembly 7300 and internal components of the pneumatic block cooperate to form a pneumatic air flow path that extends from the chassis inlet to the blower inlet of the blower and from the blower outlet of the blower to the chassis outlet 7320. The chassis outlet 7320 is adapted to communicate with the reservoir dock 6050 and an inlet of the water reservoir 6100 when the water reservoir is received in the reservoir dock 6050. The reservoir dock 6050 is also configured and arranged to allow communication between an outlet of the water reservoir 6100 and the air circuit 4170 as described in greater detail below.

## 5.7.3 Humidifier components

### 5.7.3.1 Water reservoir

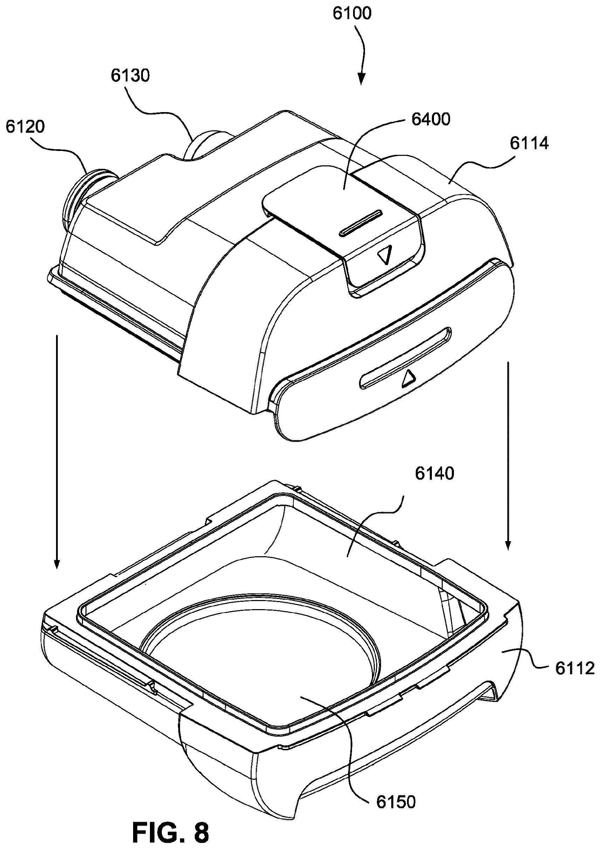

Figs. 6A, 6B and 8 show a water reservoir 6100 according to an example of the present technology. The water reservoir 6100 is configured to hold, or retain, a volume of liquid (e.g. water) to be evaporated for humidification of the flow of air. The water reservoir 6100 may be configured to hold a predetermined maximum volume of water in order to provide adequate humidification for at least the duration of a respiratory therapy session, such as one evening of sleep. Typically, the water reservoir is configured to hold several hundred millilitres of water, e.g. 300 millilitres (ml), 325 ml, 350 ml or 400 ml, although it is to be understood that other volumes of liquid may be utilised, e.g., at least 100 ml. In other forms, the humidifier may be configured to receive a supply of water from an external water source such as a building's water supply system.

In the illustrated examples, the water reservoir 6100 includes a reservoir base 6112 (also referred to as a reservoir body, a humidifier tub base, or a humidifier tub body) and a reservoir lid 6114 (also referred to as a humidifier tub lid) removably coupled to the reservoir base 6112. A deformable seal may be provided to the reservoir lid and/or to the reservoir base. When the reservoir lid 6114 is coupled to the reservoir base 6112, the seal is structured and arranged to engage between the reservoir lid 6114 and the reservoir base 6112 to seal the lid and the base and prevent egress of water from the water reservoir. The reservoir lid 6114 may be structured to be fully removable from the reservoir base 6112, e.g., for patient usability to clean the interior of the reservoir base and/or the reservoir lid. In an alternative example, the reservoir lid 6114 may be permanently attached to the reservoir base 6112.

According to one aspect, the water reservoir 6100 is configured to add humidity to a flow of air from the RPT device as the flow of air travels therethrough. In one form, the water reservoir 6100 may be configured to encourage the flow of air to travel in a tortuous path through the reservoir while in contact with the volume of water therein. For example, the water reservoir 6100 may comprise one or more flow elements, e.g., baffles, to encourage a tortuous flow path.

As described in more detail below, the water reservoir 6100 may be removably coupled with the reservoir dock 6050. In an example, insertion/removal of the water reservoir may be provided along a path extending in an anterior-posterior direction In an alternative example, at least a portion of the path for insertion/removal of the water reservoir may extend in an inferior-superior direction, e.g., at least a portion of the path for insertion includes a slope or drop down into an operative position.

The water reservoir 6100 may also be configured to discourage egress of liquid therefrom, such as when the reservoir is displaced and/or rotated from its normal, working orientation, such as through any apertures and/or in between its subcomponents. As the flow of air to be humidified by the humidifier is typically pressurised, the reservoir may also be configured to prevent losses in pneumatic pressure through leak and/or flow impedance.

Reservoir Base

As shown in Fig. 8, the reservoir base 6112 comprises a main body 6140 including a plurality of walls and a conductive portion 6150, typically provided to a bottom one of the walls to form a chamber or cavity to hold the volume of water.

The reservoir base 6112 is structured and arranged to engage or interface with the reservoir lid 6114.

The reservoir base 6112 may be structured and arranged to retain the reservoir lid 6114 to the reservoir base 6112, e.g., hinge arrangement and/or snap-fit locking tabs to releasably retain the reservoir lid to the reservoir base.

Conductive Portion

The conductive portion 6150 is configured to allow efficient transfer of heat from a heating element (e.g., heater plate 6080 of the reservoir dock 6050 shown in Fig. 6B) to the volume of liquid in the reservoir. In one form, the conductive portion may be arranged as a plate, although other shapes may also be suitable. All or a part of the conductive portion may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

Conductive portion including metal plate and/or thin film

In an example, the conductive portion 6150 may comprise a metal plate, a thin, non-metallic film (also referred to as a film plate or film base), or a combined layered arrangement of a metal plate and a thin, non-metallic film. As described below, the conductive portion 6150 is configured to thermally couple with the heater plate 6080 of the reservoir dock 6050 so as to allow thermal transfer of heat from the heater plate 6080 to the volume of liquid in the water reservoir 6100.

**5.7.3.2 Reservoir Dock**

The reservoir dock 6050 may be provided to the chassis assembly 7300 of the RPT device and configured and arranged to receive the water reservoir 6100. In some arrangements, the reservoir dock 6050 may comprise a locking feature such as a locking lever or tab, configured to retain the water reservoir 6100 in the reservoir dock 6050.
The reservoir dock 6050 includes a main body forming a cavity to receive the water reservoir 6100. As best shown in Fig. 27, a rear wall of the reservoir dock 6050 comprises the chassis outlet 7320 (also referred to as a dock inlet) structured and arranged to receive a pressurized flow of air from the outlet of the RPT device for delivery to the water reservoir 6100. The reservoir dock 6050 may also include a dock outlet structured and arranged to connect to or otherwise interface with either the air delivery tube 4170 or an intermediate component (e.g., intermediate component 6700, intermediate component 8700, or intermediate component 9700) that then connects to the air delivery tube 4170. In an example of the present technology, the reservoir dock 6050 may allow the air delivery tube 4170 to form a direct, pneumatic connection with the water reservoir 6100 so that the pressurized flow of air that has been humidified in the water reservoir 6100 is delivered directly from the water reservoir 6100 to the air delivery tube 4170.
The main body of the reservoir dock 6050 comprises a plurality of walls and a heating element (e.g., heater plate 6080) provided to a bottom one of the walls to form the cavity to receive the water reservoir 6100.

**5.7.3.3 Water Reservoir to Reservoir Dock Connection**

In use, the water reservoir 6100 is removably coupled with the reservoir dock 6050 by inserting the water reservoir 6100 into the reservoir dock 6050. In the case where the water reservoir is arranged for direct engagement with the air delivery conduit 4170, when the water reservoir 6100 is coupled to the reservoir dock 6050, an inlet seal of the inlet tube 6120 (or inlet) of the water reservoir 6100 is structured and arranged to provide a face seal with the chassis outlet 7320 (dock inlet) of the reservoir dock 6050. Similarly, the outlet seal 6132 of the outlet tube 6130 (or outlet) of the water reservoir 6100 is structured to provide a face seal with the air circuit or air delivery tube 4170, e.g., to prevent losses in pneumatic pressure through leak. In the illustrated example, the water reservoir 6100 is structured and arranged to form a direct, pneumatic seal with the air delivery conduit 4170, completely bypassing the RPT device and the reservoir dock 6050. The reservoir dock 6050 facilitates this direct connection, but is not part of it.
Removing the RPT device and the reservoir dock 6050 from the air delivery path eliminates the presence of an internally located coupling component between the water reservoir 6100 and the air delivery conduit 4170. This eliminates the need to disassemble and sterilize such coupling component, thus making sterilization much easier. In this way, when preparing the device for a different user, the water reservoir 6100 is the only component of the RPT device which needs to be replaced or sterilized.
When the water reservoir 6100 is inserted into the reservoir dock 6050 and it reaches the operative position, the conductive portion 6150 of the water reservoir 6100 aligns with and thermally contacts the heater plate 6080 of the reservoir dock 6050 to allow heat transfer from the heater plate 6080 to the water in the water reservoir 6100, e.g., surface of the conductive portion 6150 engages or contacts surface of the heater plate 6080. A biasing mechanism may be introduced that presses the water reservoir and the heater plate towards each other, thus varying the level of thermal contact between the conductive portion and the heater plate. In one example, a spring element provided to the water reservoir, the reservoir dock and/or the heater plate may be arranged to bias the water reservoir and the heater plate towards each other to increase contact pressure and improve thermal contact.
The chassis outlet 7320 (dock inlet) is configured to receive the pressurized flow of air from the blower of the RPT device, and to pass on the flow of air into the water reservoir 6100 via the inlet tube 6120 of the water reservoir 6100. Humidity (i.e., water vapour) is added to the flow of air as the air travels through the water reservoir 6100, and the humidified flow of air exits the water reservoir through the outlet tube 6130. Air flows directly from the outlet tube 6130 and into the air delivery tube 4170 to deliver the flow of humidified air to the patient.

### 5.7.3.4 Guiding structures for Insertion/Removal

In an example, an outer side portion of the water reservoir 6100 provides a dock engagement portion structured and arranged to interface and engage a reservoir engagement portion of the reservoir dock 6050. In an example, the water reservoir 6100 and reservoir dock 6050 may include guiding structures to facilitate insertion, removal, and alignment of the water reservoir 6100 with the reservoir dock 6050.

For example, as shown in Fig. 6B, opposing sides of the water reservoir 6100 along the dock engagement portion may include guiding surfaces (e.g. provided by guide rails 6200) arranged to engage corresponding guiding surfaces (e.g., provided by a guide slot 6060) along the reservoir engagement portion of the reservoir dock 6050 to guide the water reservoir 6100 into the reservoir dock 6050.

In an example, as shown in Fig. 6B, the water reservoir 6100 may be inserted/removed (e.g., by sliding or push/pull only) along a path extending in a lateral direction (i.e., anterior-posterior direction) into and out of the cavity of the reservoir dock 6050.

In an alternative example, at least a portion of the path for insertion/removal of the water reservoir may extend in an inferior-superior direction, e.g., at least a portion of the path for insertion of the water reservoir into the dock includes a slope, such as an elevation or drop down, into the operative position.

For example, the guiding structures of the water reservoir 6100 and reservoir dock 6050 may be structured and arranged to provide an initial horizontal or sloped insertion of the water reservoir with a subsequent drop down in the last section into the operative position. In an example, the reservoir dock may provide a sloping surface with an internal edge located on the bottom surface of the dock that has to be cleared by the water reservoir before it can be dropped down to its operative position. The cleared edge and/or the drop down itself may effectively lock the water reservoir into the operative position. Further locking features may also be used. Such "push and drop" configuration includes movement of the tub that has components in both a horizontal and a vertical direction. The optional inclusion of the edge ensures that during insertion of the water reservoir into the reservoir dock, the base of the water reservoir engages a single edge or a small surface, as opposed to being dragged over a much larger surface. This reduces any wear and potential damage to the heater plate. A spring element may be arranged (e.g., between the reservoir dock and the water reservoir) to increase contact pressure between the water reservoir and the heater plate, e.g., to improve thermal contact between the base plate of the reservoir and the heater plate of the dock.

### 5.7.3.5 Retaining Feature

In an example, as shown in Fig. 6B, the water reservoir 6100 may comprise a latch 6400 configured to releasably engage with a recessed slot 6055 in the reservoir dock 6050 to releasably retain the water reservoir 6100 in an operative position within the reservoir dock 6050. Such a locking arrangement prevents the water reservoir from disengaging from the dock, which in some arrangements, the water reservoir may be encouraged to do by the relatively high operational pressure within the dock during the operation of the device.

In the illustrated example, the latch 6400 is provided as a separate and distinct structure from the water reservoir 6100 and then secured or otherwise provided to the water reservoir 6100 in an operative position, e.g., the latch 6400 comprises a pre-formed structure that is secured to the reservoir lid 6114, or to other portions of the water reservoir 6100. In an example, the latch 6400 comprises a plastic or thermoplastic polymer material.

### 5.7.3.6 Air Delivery Tube to Reservoir Dock Connection

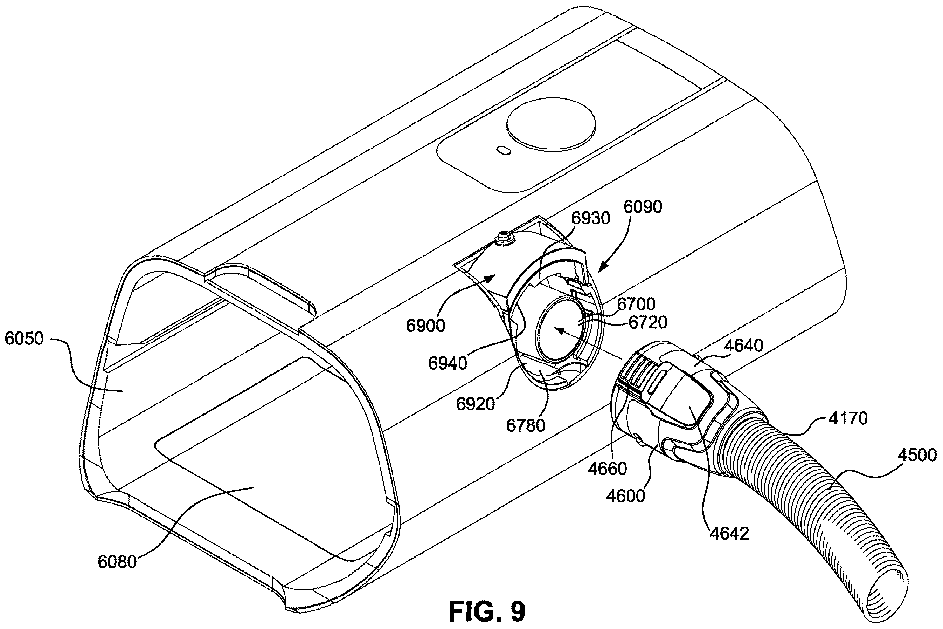

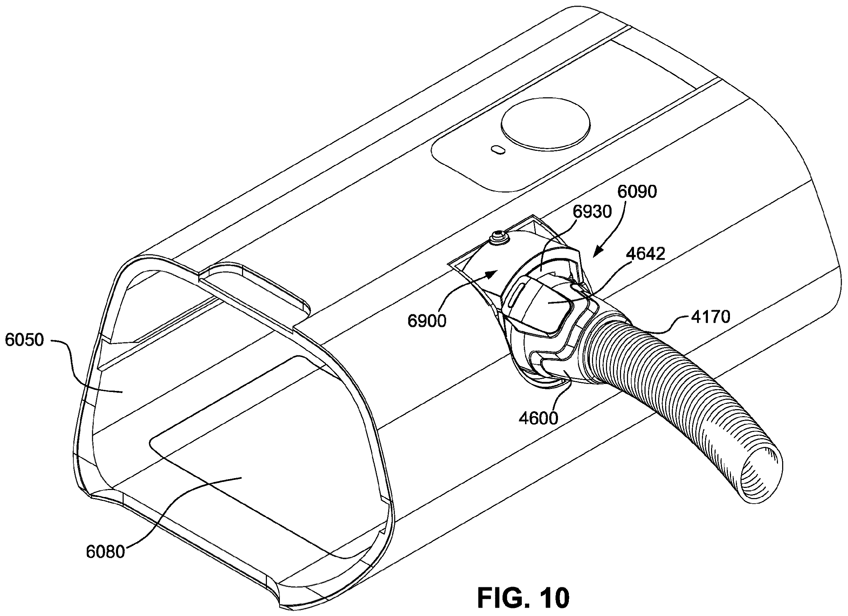

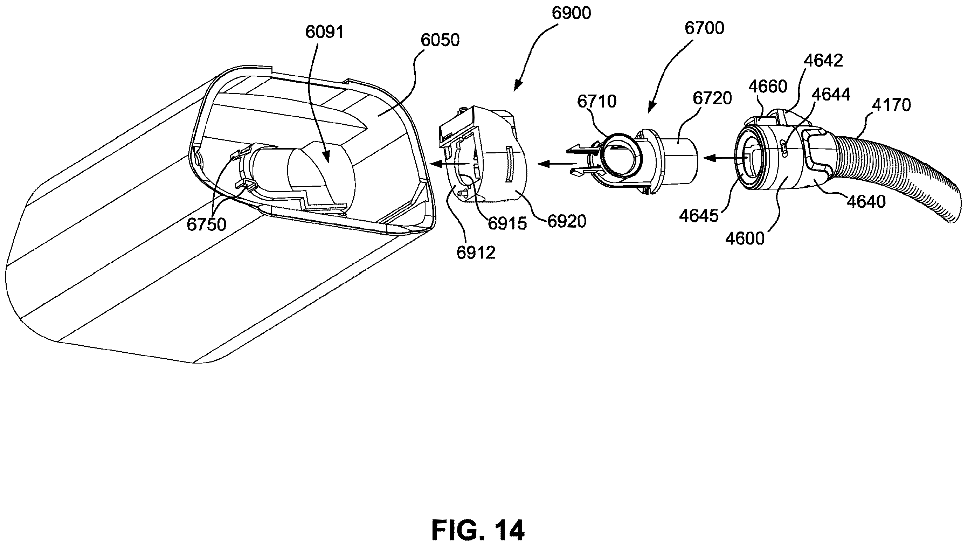

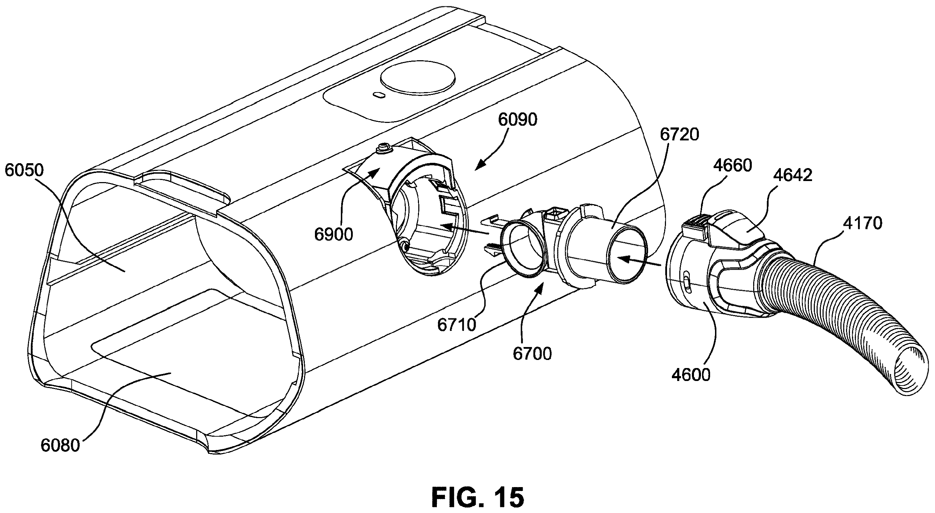

In an example, e.g., as shown in Figs. 9-15, the air delivery tube 4170 includes a tube portion 4500, a dock connector/cuff 4600 (outlet connector) to connect the air delivery tube 4170 to the reservoir dock 6050 and/or the water reservoir 6100, and a patient interface connector/cuff (inlet connector shown in Figs. 1A-1C) to connect the air delivery tube 4170 to the patient interface 3000.

In an example, the dock connector 4600 is structured and arranged to form a mechanical and/or electrical connection with the reservoir dock 6050 and to form a pneumatic connection with the water reservoir 6100 and/or with the reservoir dock 6050. These connections locate and secure the air delivery tube 4170 to the reservoir dock 6050 or the water reservoir 6100, provide electrical power, information and control signals to the heating element and transducers associated with the air delivery tube 4170, and/or allow humidified, pressurized gas to flow from the water reservoir 6100 to the patient interface 3000. During the engagement of the air delivery tube 4170 with the water reservoir 6100 and the reservoir dock 6050, the connections may be formed simultaneously or in series, e.g., one of the mechanical, pneumatic or electrical connections may be completed before others.

The dock connector 4600 of the air delivery tube 4170 includes a retention feature that provides a fixed, non-rotatable connection with the dock outlet 6090 of the reservoir dock 6050.

In an example, the air delivery tube 4170 may include a plurality of wires helically wound around the axis of the air delivery tube 4170 (e.g., along the tube portion 4500 of the air delivery conduit 4170), e.g., configured to heat air in the air delivery tube and/or transmit signal from one or more transducers (e.g., temperature sensor, flow sensor) to a controller of the RPT device.

In an example, the air delivery tube 4170 may comprise four wires, e.g., two wires for powering one or more heating elements and two wires for connecting a temperature sensor/transducer. However, it should be appreciated that other numbers of wires may be used, e.g., two wires, three wires, or five or more wires.

In an example, the dock connector 4600 of the air delivery tube 4170 includes a contact assembly including contacts that, in use, are engaged with respective contacts provided to the reservoir dock 6050 to form electrical connections with the reservoir dock at the dock outlet to provide electrical power and/or control signal transmission. In an example, the contacts of the dock connector 4600 may be joined to respective wires running along the air delivery tube 4170. In an alternative example, the at least some of the contacts are not related to the wires running along the air delivery tube 4170, but are characterised by their own independent and/or unique electrical characteristics (e.g., resistance, conductance, etc.). Such independent and/or unique electrical characteristics may be used for identifying one or more elements of the tube/patient interface system, or of characteristics of these elements.

In an example, the dock outlet 6090 of the reservoir dock 6050 includes a contact assembly in communication with electrical power and electrical signalling within the reservoir dock, e.g., the PCBA 7600. In an example, the contact assembly includes contacts corresponding to the number of contacts provided to the dock connector 4600 of the air delivery tube 4170, e.g. four contacts.

Because each contact, or combination of contacts, in the contact assembly of the air delivery tube 4170 may have unique electrical characteristic, in an example, the contact assembly of the air delivery tube 4170 may be used as an identifier of various parameters of the air delivery tube 4170 and/or the patient interface. For example, the contact assembly may be configured to provide identification of the type of air delivery tube 4170 (e.g., non-heated tube, heated tube, tube with heat and moisture exchanger (HME), tube unknown), size of air delivery tube (e.g., 15mm, 19 mm), presence and type of HME, type of patient interface connected to tube, etc. The data from identification may be communicated and used by a controller, e.g., to optimize operation of the RPT device, humidifier, to facilitate data collection, etc. For example, the controller may be configured to recognize a unique identifying feature provided by the contact assembly so that the controller can recognize the specific characteristics of the air delivery tube 4170 coupled to the reservoir dock 6050, and therefore the controller can automatically configure the RPT device and/or humidifier to optimize operation.

### 5.7.3.6.1 Bayonet-Style Connection and Intermediate Component

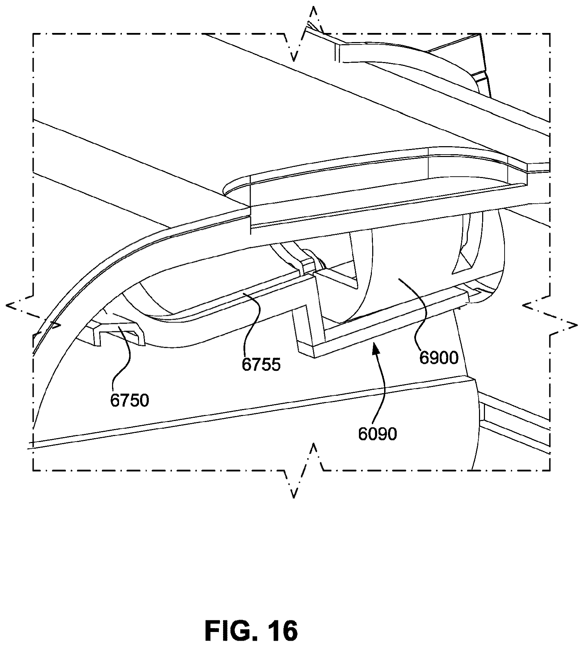

Figs. 9-22 illustrate an alternative example for connecting the air delivery tube 4170 to the reservoir dock 6050 and the water reservoir 6100. In this example, an intermediate component 6700 is removably coupled to the reservoir dock 6050. The intermediate component 6700 is configured to pneumatically connect the water reservoir 6100 to the air delivery tube 4170 so that the pressurized flow of air that has been humidified in the water reservoir 6100 can be delivered from the water reservoir 6100, via the intermediate component 6700, to the air delivery tube 4170. Also, in this example, the dock connector 4600 of the air delivery tube 4170 is structured and arranged to form a bayonet-style connection with the reservoir dock 6050, which mechanically and/or electrically connects the air delivery tube 4170 with the reservoir dock 6050. That is, the bayonet-style connection locates and secures the air delivery tube 4170 to the reservoir dock 6050 and/or provides electrical power, information and control signals to the heating element and transducers associated with the air delivery tube 4170.

### 5.7.4 Humidifier transducer(s)

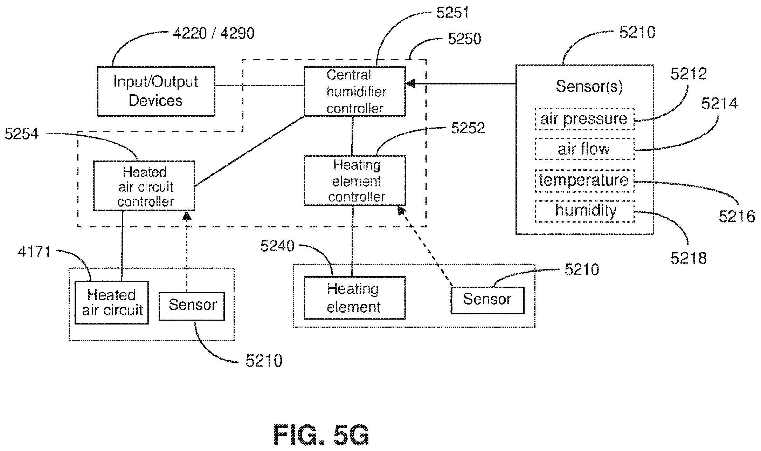

The humidifier 5000 may comprise one or more humidifier transducers (sensors) 5210 instead of, or in addition to, transducers 4270 described above. Humidifier transducers 5210 may include one or more of an air pressure sensor 5212, an air flow rate transducer 5214, a temperature sensor 5216, or a humidity sensor 5218 as shown in Fig. 5G. A humidifier transducer 5210 may produce one or more output signals which may be communicated to a controller such as the central controller 4230 and/or the humidifier controller 5250. In some forms, a humidifier transducer may be located externally to the humidifier 5000 (such as in the air circuit 4170) while communicating the output signal to the controller.

### 5.7.4.1 Pressure transducer

One or more pressure transducers 5212 may be provided to the humidifier 5000 in addition to, or instead of, a pressure sensor 4272 provided in the RPT device 4000.

### 5.7.4.2 Flow rate transducer

One or more air flow rate transducers 5214 may be provided to the humidifier 5000 in addition to, or instead of, a flow rate sensor 4274 provided in the RPT device.

### 5.7.4.3 Temperature transducer

The humidifier 5000 may comprise one or more temperature transducers 5216. The one or more temperature transducers 5216 may be configured to measure one or more temperatures such as of the heating element 5240 and/or of the flow of air downstream of the humidifier outlet. In some forms, the humidifier 5000 may further comprise a temperature sensor 5216 to detect the temperature of the ambient air.

### 5.7.4.4 Humidity transducer

In one form, the humidifier 5000 may comprise one or more humidity sensors 5218 to detect a humidity of a gas, such as the ambient air. The humidity sensor 5218 may be placed towards the humidifier outlet in some forms to measure a humidity of the gas delivered from the humidifier 5000. The humidity sensor may be an absolute humidity sensor or a relative humidity sensor.

### 5.7.5 Heating element

As shown in Figs. 6B and other figures, a heater plate 6080 is used to transfer heat to the water reservoir. In the illustrated example, the heater plate may form a part of the reservoir dock 6050, and may be located on or near the base of the reservoir dock. At least the top layer of the heater plate comprises a hard scratch resistant surface that may be formed, for example, of a nickel chrome alloy, stainless steel or anodised aluminium. The heater plate may transfer heat from a heating element. The heating element may comprise a heat generating component such as an electrically resistive heating track. One suitable example of a heating element is a layered heating element such as one described in the PCT Patent Application Publication No. WO 2012/171072, which is incorporated herewith by reference in its entirety.

### 5.7.6 Humidifier controller

According to one arrangement of the present technology, a humidifier 5000 may comprise a humidifier controller 5250 as shown in Fig. 5G. In one form, the humidifier controller 5250 may be a part of the central controller 4230. In another form, the humidifier controller 5250 may be a separate controller, which may be in communication with the central controller 4230.

In one form, the humidifier controller 5250 may receive as inputs measures of properties (such as temperature, humidity, pressure and/or flow rate), for example of the flow of air, the water in the water reservoir 5110 and/or the humidifier 5000. The humidifier controller 5250 may also be configured to execute or implement humidifier algorithms and/or deliver one or more output signals.

As shown in Fig. 5G, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

### 5.8 INTERMEDIATE COMPONENT

As shown in Figs. 9 to 22, the intermediate component 6700 is provided to the dock outlet 6090 of the reservoir dock 6050 to pneumatically connect the water reservoir 6100 to the air delivery tube 4170. In the illustrated example, the intermediate component 6700 is removably coupled to the reservoir dock 6050 so that the intermediate component 6700 can be disassembled for cleaning, sterilization and/or replacement, e.g., for multi-patient multi-use (MPMU) applications.

As shown in Figs. 9 to 22, the intermediate component 6700 comprises a tubular body 6705 including an inlet end 6710 adapted to interface with the water reservoir 6100 and an outlet end 6720 adapted to interface with the air delivery tube 4170. The intermediate component 6700 also comprises retention and alignment features structured and arranged to align the intermediate component 6700 with the reservoir dock 6050 and provide a removable, non-rotatable connection with the reservoir dock 6050. In addition, the intermediate component 6700 comprises a port 6730, e.g., pressure port for inserting a sensor for measuring air pressure at the dock outlet 6090 or a sound port for propagating sound from within the intermediate component 6700 to a sensor that is external to the intermediate component 6700. The port 6730 includes a port seal 6735 to provide a sealing interface between a sensor, e.g., pressure sensor or a sound sensor, and the intermediate component 6700. As will be discussed later in the text, an acoustically transparent cover, e.g. in the form of a membrane that covers the port 6730, may also be included in the arrangement. The body of the cover may be integral with that of at least one of the port 6730 and the port seal 6735.

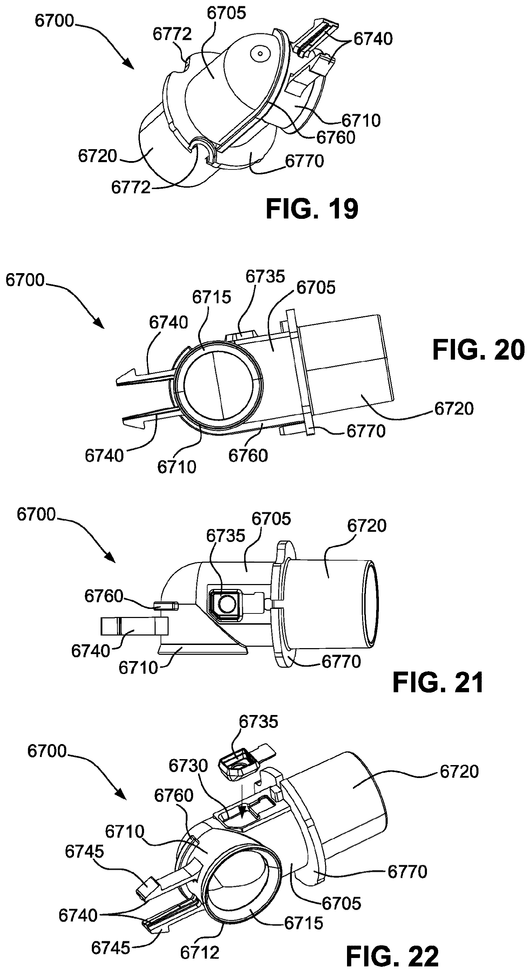

In the illustrated example, e.g., see Fig. 22, the tubular body 6705 (including the inlet end 6710 and the outlet end 6720), along with the retention and alignment features, comprise a first part or base mold constructed of a relatively rigid material (e.g., thermoplastic polymer (e.g., PC, ABS)). The port seal 6735 comprises a second part or overmold constructed of a relatively soft material (e.g., thermoplastic elastomer (TPE) or silicone) that is provided (e.g., by overmolding) to the first part. Thus, the intermediate component 6700 provides a substantially rigid construction, e.g., for durability for MPMU applications.

In the illustrated example, the inlet end 6710 is arranged at an angle to the outlet end 6720, e.g., the axis of the inlet end is arranged at about 90° with respect to the axis of the outlet end. However, it should be appreciated that other suitable angles are possible, e.g., the axis of the inlet end is arranged at about 45° with respect to the axis of the outlet end.

The free end of the inlet end 6710 includes a flange or lip 6712 surrounding the tube opening. The flange or lip 6712 provides a contact surface 6715. When the water reservoir 6100 is coupled to the reservoir dock 6050, the outlet seal 6132 of the outlet tube 6130 (or outlet) of the water reservoir 6100 is structured to engage and provide a face seal with the contact surface 6715 of the inlet end 6710. In an alternative embodiment, the seal between the outlet tube 6130 (or outlet) of the water reservoir 6100 and the contact surface 6715 of the inlet end 6710 may be an integral part of the inlet end 6710, or may be a sealing portion independent from either the outlet tube 6130 or the inlet end 6710. In the illustrated example, the contact surface 6715 comprises a taper into the tube opening, e.g., to enhance sealing and prevent leak.

The outlet end 6720 may comprise an ISO taper, e.g., 22 mm outer diameter ISO taper, for coupling to the air delivery conduit 4170.

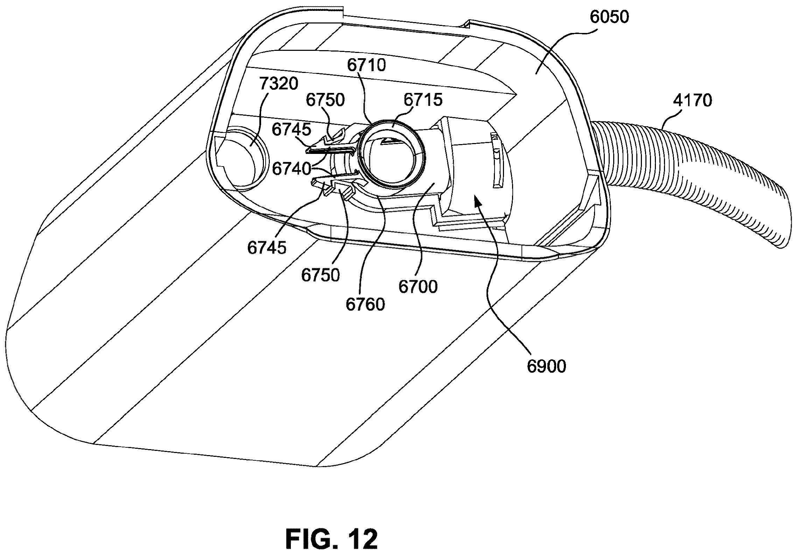

In regards to retention and alignment features, the intermediate component 6700 includes a pair of resilient pinch arms 6740, i.e., cantilevered spring arms. Each of the spring or pinch arms 6740 may include a barbed end or tab 6745 structured to provide a snap-fit connection with respective locking members, e.g., protrusions 6750, provided within the cavity of the reservoir dock 6050 as shown in Fig. 12. The intermediate component 6700 also includes a guide rail 6760 structured and arranged to assist in correct alignment and insertion of the intermediate component 6700 into the reservoir dock 6050 by engagement with a corresponding guide slot 6755 extending into the cavity of the reservoir dock 6050 as shown in Figs. 12, 16 and 18. Further, the intermediate component 6700 includes a flange 6770 arranged between the inlet end 6710 and the outlet end 6720 to assist in locating or positioning the intermediate component 6700 in the reservoir dock 6050 by abutting a flange or wall provided to the reservoir dock 6050, e.g., flange acts as a stop during insertion. The flange 6770 of intermediate component 6700 may include one or more cut-outs or recesses 6772, e.g., to accommodate fasteners or projections along the flange or wall provided to the reservoir dock 6050.

When the intermediate component 6700 is inserted into the dock opening 6091 of the reservoir dock 6050, the intermediate component 6700 is oriented to engage its guide rail 6760 with the guide slot 6755 which correct aligns and guides the intermediate component 6700 into an operative position. Also, the dock opening 6091 and/or an opening provided by the locking and contact assembly 6900 at the dock opening 6091 includes a non-circular profile to facilitate correct orientation of the intermediate component 6700 during insertion. When the intermediate component 6700 reaches an operative position, the barbed ends or tabs 6745 of the spring or pinch arms 6740 are configured and arranged to engage over and/or behind respective protrusions 6750, e.g., see Fig. 12. Each barbed end 6745 and/or each protrusion 6750 may include a taper to facilitate engagement into the operative position. In an example, the engagement of the spring or pinch arms 6740 with the protrusion 6750 may provide sensory feedback, e.g., audible click, to indicate correction connection. This snap-fit connection releasably secures the intermediate component 6700 to the reservoir dock 6050. To disengage the intermediate component 6700, the spring or pinch arms 6740 can be manually depressed towards one another (e.g., with or without a tool) to resiliently flex the spring or pinch arms 6740 and barbed ends 6745 thereof against biasing to an unlocked position, i.e., barbed ends 6745 moved out of engagement with the protrusion 6750 to allow the intermediate component 6700 to be removed from the reservoir dock 6050.

Once the connection is established, the cooperating retention and alignment features provided by the intermediate component 6700/reservoir dock 6050 provides a removable, non-rotatable connection of the intermediate component 6700 to the dock outlet 6090 of the reservoir dock 6050. Also, once connected, the spring or pinch arms 6740 of the intermediate component 6700 are lockingly engaged within the cavity of the reservoir dock 6050, e.g., to prevent removal of the intermediate component 6700 when the water reservoir 6100 is received in the reservoir dock 6050.

When the intermediate component 6700 is connected to the dock outlet 6090 of the reservoir dock 6050, the inlet end 6710 and contact surface 6715 thereof protrudes into the cavity of the reservoir dock 6050 to allow engagement with the outlet seal of the outlet tube 6130 (or outlet) of the water reservoir 6100, e.g., see Fig. 12. Likewise, the outlet end 6720 of the intermediate component 6700 extends within, and/or protrudes out, of the cavity of the reservoir dock 6050 to allow engagement with the air delivery tube 4170, e.g., see Fig. 9. Further, the port 6730 of the intermediate component 6700 is oriented, e.g., upwardly, to interface with the sensor associated with the PCBA disposed superior to the intermediate component 6700 in the operational configuration of the RPT device.

### 5.8.1 Bayonet-Style Locking and Contact Assembly

As shown in Figs. 9-18, a locking and contact assembly 6900 is provided to the dock outlet 6090 of the reservoir dock 6050 to mechanically and electrically connect the reservoir dock 6050 to the air delivery tube 4170. In the illustrated example, the locking and contact assembly 6900 comprises a bayonet-style connection structured and arranged to locate and secure the air delivery tube 4170 to the reservoir dock 6050 and form mechanical, pneumatic and electrical (both power and control signals) connections. The locking and contact assembly 6900 may be separate from, or integral with, the RPT device 6000.

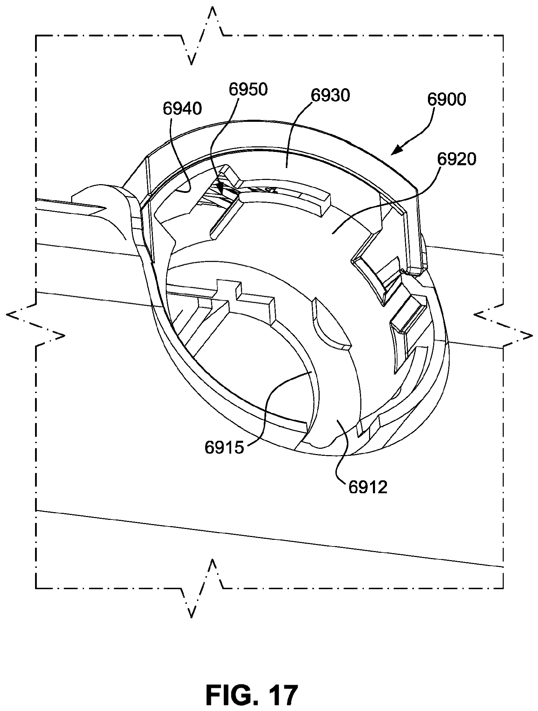

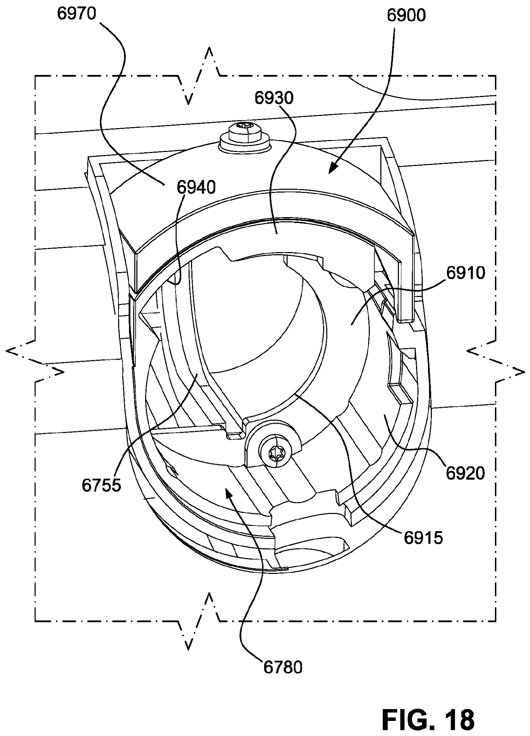

As shown in Figs. 17 and 18, the locking and contact assembly 6900 includes a base 6910, an (electrical) contact assembly 6950 provided to the base, and a cover 6970 provided to the base 6910 to enclose at least a portion of the electrical contact assembly 6950.

The base 6910 of the locking and contact assembly 6900 includes a rear wall 6912 that is secured, e.g., via one or more fasteners, to one or more walls surrounding the dock opening 6091 so as to secure the base 6910 at the dock outlet 6090 of the reservoir dock 6050. As shown in Figs. 17 and 18, the rear wall 6912 includes an opening 6915, e.g., non-circular, that aligns with the dock opening 6091 to allow insertion and connection of the intermediate component 6700 as mentioned above, e.g., a non-circular opening 6915 adapted to receive non-circular profile of the intermediate component 6700. The non-circular profile assists a user with the orientation alignment of the intermediate component 6700 and the locking and contact assembly 6900, during insertion. Further, as mentioned above, the rear wall 6912 provides a stop for the intermediate component 6700 during assembly, e.g., at least a portion of the flange 6770 of the intermediate component 6700 may abut the rear wall 6912.

The base 6910 of the locking and contact assembly 6900 includes an annular side wall 6920 that projects axially and outwardly from the rear wall 6912. When the intermediate component 6700 is connected to the reservoir dock 6050, the outlet end 6720 of the intermediate component 6700 and the annular side wall 6920 cooperate to form a channel 6780 for receiving the air delivery tube 4170. A retaining wall 6930 projects radially outwardly from the annular side wall 6920 along a portion of the perimeter of the annular side wall, e.g., along a portion of the superior side of the annular side wall. A gap is provided in the annular side wall 6920 along a portion of the perimeter of the annular side wall which forms a recess 6940 that leads into the channel 6780 (see Figs. 9 and 18). The recess 6940 is adjacent to, and disposed counter-clockwise from, the retaining wall 6930. As described below, the recess 6940 and retaining wall 6930 are configured and arranged so that a portion of the dock connector 4600 of the air delivery tube 4170 may be inserted into the recess 6940 and then rotated clockwise, to move behind retaining wall 6930, to effect a locking engagement between the air delivery tube and the dock.

Additional retention and alignment features, e.g., recesses and/or grooves, are provided to the annular side wall 6920 along its perimeter that are structured and arranged to interact with corresponding features on the dock connector 4600 of the air delivery tube 4170 during engagement as discussed below.

As shown in Fig. 17, the electrical contact assembly 6950 is supported by the base 6910 adjacent the retaining wall 6930. The contact assembly 6950 is in communication with electrical power and electrical signalling within the reservoir dock 6050, e.g., the PCBA 7600. As illustrated, the contact assembly 6950 includes a support member 6952 and a plurality of contacts, e.g., four contacts. The arrangement is such that, when the air delivery tube 4170 is rotated clockwise, to lockingly engage the dock, a contact assembly of the tube moves into contact with assembly 6950, to allow exchange of power, control and the RPT device (e.g. PCBA 7600).

### 5.8.2 Dock Connector

As shown in Figs. 9-11, the dock connector 4600 of the air delivery tube 4170 is structured to form a pneumatic connection with the intermediate component 6700 and form a mechanical and electrical connection with the locking and contact assembly 6900 provided to the reservoir dock 6050.

In the illustrated example, the dock connector 4600 includes a tubular base portion 4640 and a locking and contact assembly 4660 provided to the base portion 4640.

### 5.8.3 Straight Plug-In Connection and Intermediate Component

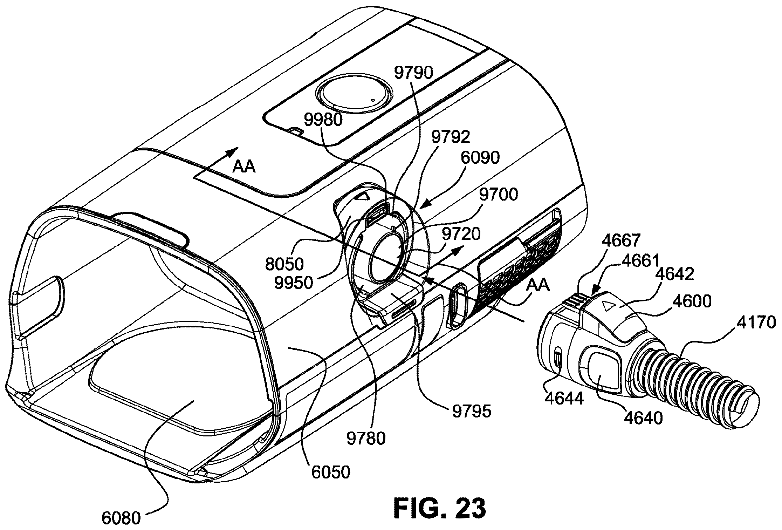

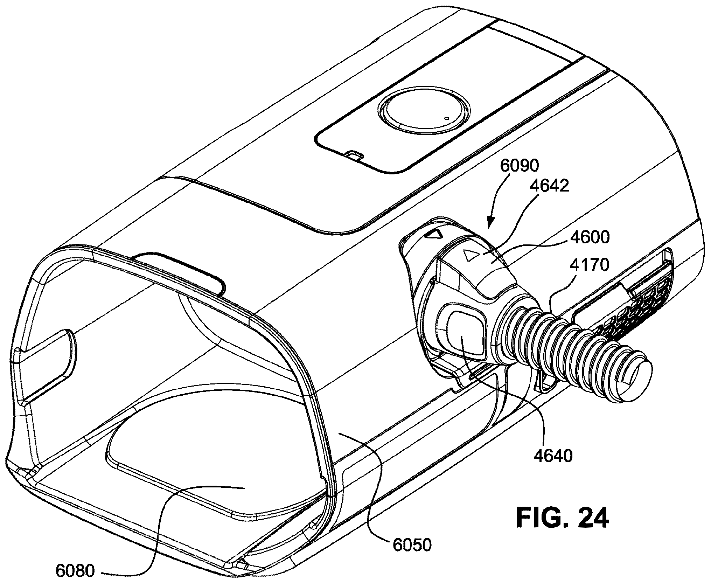

Figs. 23-36C illustrate an alternative example of an intermediate component 9700 for connecting the air delivery tube 4170 to the reservoir dock 6050 and the water reservoir 6100 in accordance with one form of the present technology.

In this example, the intermediate component 9700 is removably coupled to the reservoir dock 6050. The intermediate component 9700 is configured to pneumatically connect the water reservoir 6100 to the air delivery tube 4170 so that the pressurized flow of air that has been humidified in the water reservoir 6100 can be delivered from the water reservoir 6100, via the intermediate component 9700, to the air delivery tube 4170. Also, in this example, the intermediate component 9700 is configured to mechanically connect to the air delivery tube 4170, thus locating and releasably retaining the air delivery tube 4170 to the reservoir dock 6050. Further, in this example, the air delivery tube 4170 is structured and arranged to form an electrical connection with the reservoir dock 6050, which provides electrical power and control signals to the heating element, and sensed data from transducers associated with the air delivery tube 4170 to the reservoir dock 6050.

Accordingly, in contrast to the example described above in relation to Figs. 9-22 (in which the dock connector 4600 pneumatically seals with the intermediate component 6700 and mechanically connects with the reservoir dock 6050), the dock connector 4600 of the air delivery tube 4170 forms both a pneumatic seal and a mechanical (locking) connection with the intermediate component 9700 in the example of Figs. 23-26. By combining the pneumatic and mechanical connections into one component, the dimensional tolerances can improve, which may make the dock connector 4600 more reliable and easier to manufacture, and may also allow reduction in the size of the dock connector 4600.

Intermediate Component

As shown in Figs. 23, 25, 26, and 25A, the intermediate component 9700 is a separate component provided to the dock outlet 6090 of the reservoir dock 6050 to pneumatically connect the water reservoir 6100 to the air delivery tube 4170 and mechanically connect the air delivery tube 4170 to the reservoir dock 6050. In the illustrated example, the intermediate component 9700 is removably coupled to the reservoir dock 6050 so that the intermediate component 9700 can be disassembled for cleaning, sterilization and/or replacement, e.g., for multi-patient multi-use (MPMU) applications.

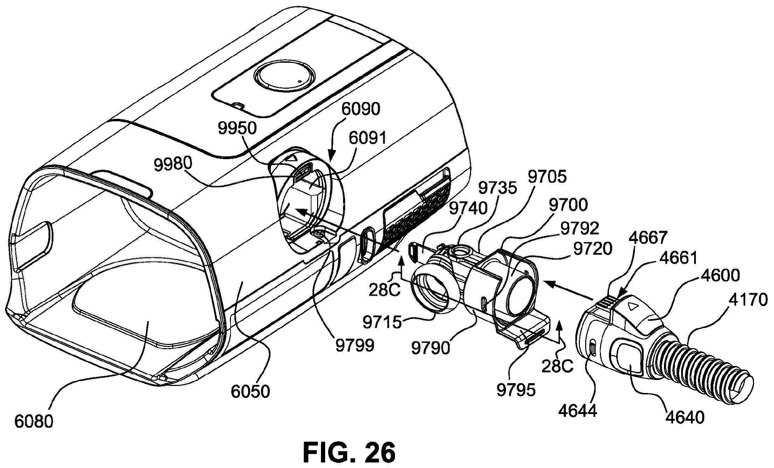

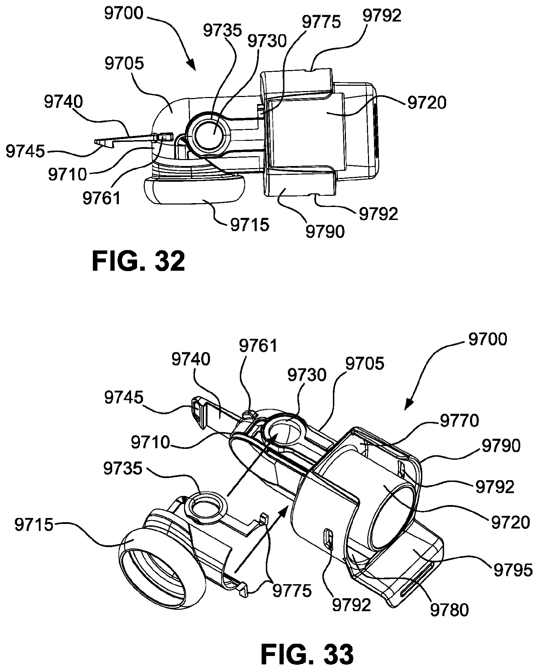

As shown in Figs. 26 and 29-33, the intermediate component 9700 comprises a generally tubular body 9705 including an inlet end 9710 and an outlet end 9720. The inlet end 9710 (Fig. 33) is provided with an inlet seal 9715 adapted to interface with the water reservoir 6100, and the outlet end 9720 is adapted to interface with the air delivery tube 4170. The tubular body 9705 also comprises retention and alignment features structured and arranged to align the intermediate component 9700 with the reservoir dock 6050 and provide a removable, non-rotatable connection with the reservoir dock 6050.

In addition, the tubular body 9705 comprises a port 9730 in the form of an opening, e.g., for communicating with a sensor or a transducer. This sensor or a transducer measures the sound propagated through the port 9730 and could be any type of microphone of pressure sensor (including resistive, capacitive, piezoelectric, optical or other technology -based). The port 9730 is provided with a port seal 9735 around the opening, to provide a sealing interface between the intermediate component 9700 and a chassis opening 7380 (see Figs. 32-36C) associated with the sensor. Further, the intermediate component 9700 comprises retention features structured and arranged to provide a locking, but removable, connection with the dock connector 4600 of the air delivery tube 4170.

In the illustrated example (e.g., see Fig. 33), the tubular body 9705 (including the inlet end 9710, the outlet end 9720, and retention and alignment features) comprises a first part or base mold constructed of a relatively rigid material (e.g., thermoplastic polymer (e.g., PC, ABS)) and the inlet seal 9715 and the port seal 9735 comprise a second part or overmold constructed of a relatively soft material (e.g., thermoplastic elastomer (TPE) or silicone) that is provided (e.g., by overmolding) to the first part.

In the illustrated example, the inlet seal 9715 is arranged at an angle to the outlet end 9720, e.g., the axis of the opening at the inlet seal 9715 is arranged at about 90° with respect to the axis of the opening at the outlet end 9720 (see Fig. 32). However, it should be appreciated that other suitable angles are possible, e.g., the axis of the inlet seal 9715 is arranged at about 45° with respect to the axis of the outlet end 9720.

When the water reservoir 6100 is coupled to the reservoir dock 6050, the inlet seal 9715 of the intermediate component 9700 is structured and arranged to engage and provide a face seal against a contact surface along the outlet end of the outlet tube 6130 (or outlet) of the water reservoir 6100. Such engagement seals the outlet flow path that allows humidified air to flow out of the water reservoir 6100 and into the intermediate component 9700 for delivery to the air delivery tube 4170. As illustrated, the inlet seal 9715 may comprise a bellows-type arrangement that is resiliently compressible to provide a certain degree of decoupling between the intermediate component 9700 and the water reservoir 6100. When an outlet cap muffler 4124 (Fig. 5B) is used instead of a water reservoir, there is a similar sealing engagement between the inlet seal 9715 of the intermediate component 9700 and the opening of the outlet cap muffler.

In an alternative embodiment, the seal between the outlet tube 6130 (or outlet) of the water reservoir 6100 and the intermediate component 9700 may be an integral part of the outlet tube 6130, or may be a sealing portion independent from either the outlet tube 6130 or the intermediate component 9700.

The outlet end 9720 may comprise an ISO taper, e.g., 22 mm outer diameter ISO taper, for coupling to the air delivery conduit 4170.

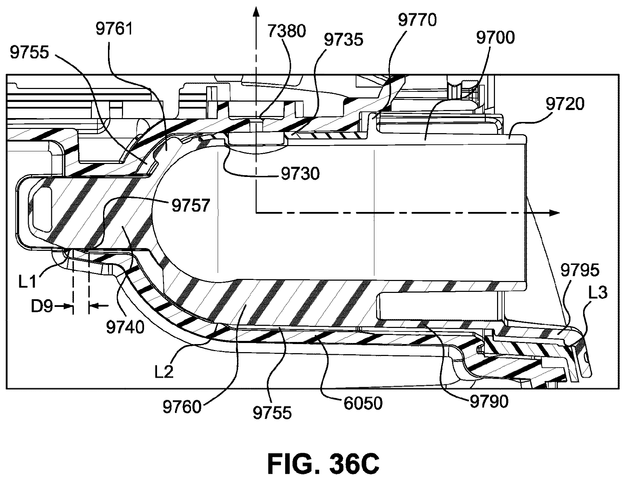

In regards to retention and alignment features to align and retain the intermediate component 9700 to the reservoir dock 6050, the intermediate component 9700 includes a resilient pinch arm 9740, i.e., a cantilevered spring arm. The spring or pinch arm 9740 may include a barbed end or tab 9745 structured to provide a snap-fit connection with a locking member, e.g., cross-bar 9750, provided within the cavity of the reservoir dock 6050 (see Figs. 27 and 36C). The intermediate component 9700 also includes a guide rail 9760 (along a lower side of the intermediate component 9700) and a guide rib 9761 (along a forward, upper side of the intermediate component 9700) structured and arranged to assist in correct alignment and insertion of the intermediate component 9700 into the reservoir dock 6050 by engagement with corresponding guide slots 9755 extending into the cavity of the reservoir dock 6050 (e.g., see Figs. 27, 28B and 30).

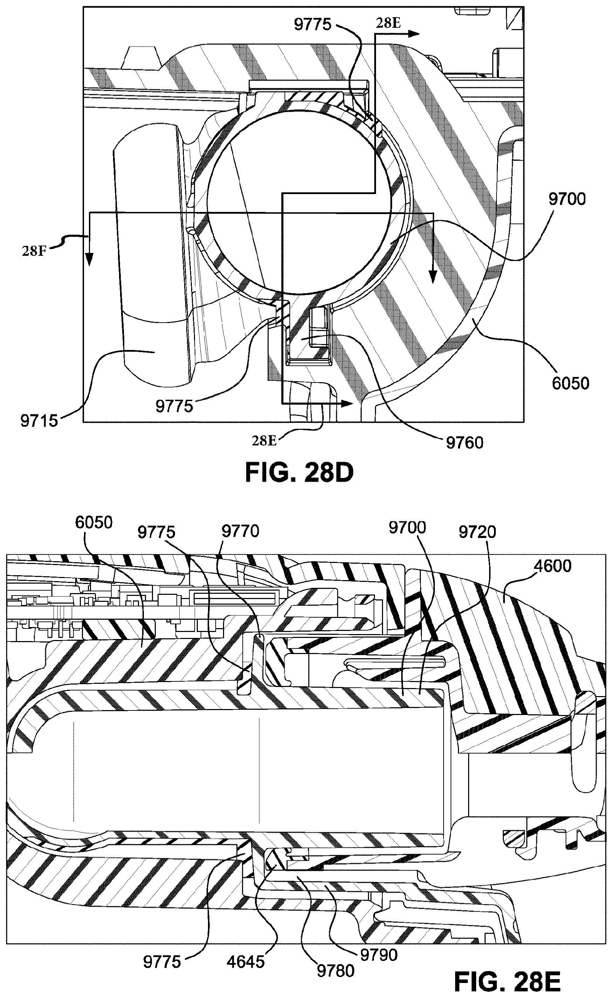

Further, the intermediate component 9700 includes a flange 9770 arranged between the inlet end 9710 and the outlet end 9720 to assist in locating or positioning the intermediate component 9700 in the reservoir dock 6050 by abutting a wall provided to the reservoir dock 6050, e.g., flange acts as a stop during insertion as shown in Fig. 28E. As shown in Figs. 28D, 28E and 33, one or more bumpers 9775 (e.g., constructed of thermoplastic elastomer (TPE) or silicone) are provided to the flange 9770 to soften abutment with the reservoir dock opening during insertion and absorb vibrations in use. Apart from minimising the vibration of the intermediate component 9700, the flexible nature of the bumps ensures that, once they are depressed, there is a resultant spring force that pushes backwards the barbed tab 9745 and ensures that the tab is in a constant locking engagement with the cross-bar 9750. This minimises any vibrations in the locking engagement between barbed tab 9745 and the cross-bar 9750, as well as the likelihood of disengagement. In the illustrated example, a first bumper 9775 is provided to an upper side of the intermediate component 9700, and a second bumper 9775 is provided to a lower side of the intermediate component 9700 (see Fig. 28D). In an example, the bumpers 9775 may be overmolded to the tubular body 9705 along with the inlet seal 9715 and the port seal 9735 (see Fig. 33). The bumpers 9775 can be part of the reason why, upon insertion of the intermediate component 9700 into the tubular opening in reservoir dock 6050, an increased resistance is encountered by the intermediate component during the later stages of insertion and at a location where the bumpers engage with respective portions of the reservoir dock opening.

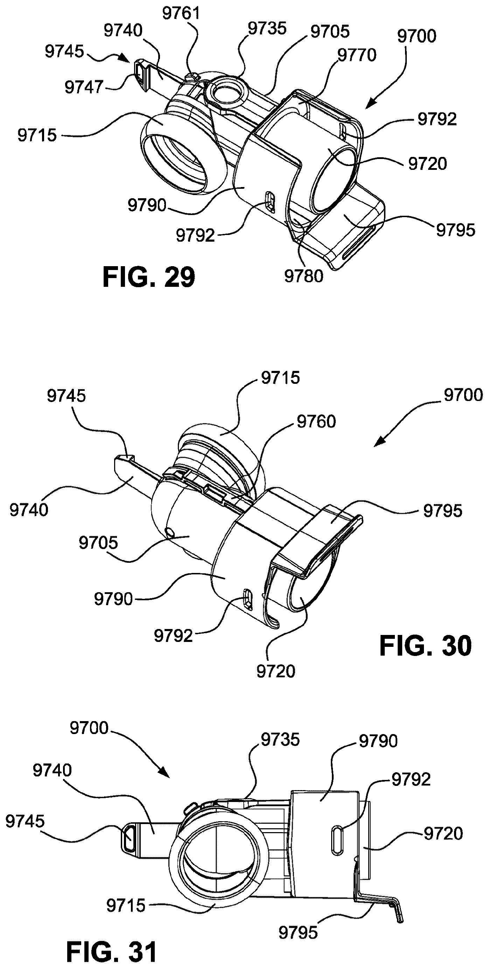

In regards to retention features to retain the dock connector 4600 of the air delivery tube 4170 to the intermediate component 9700, the intermediate component 9700 includes a part-annular side wall 9790 (see Fig. 30) that projects outwardly from the flange 9770 along the outlet end 9720. As illustrated in Fig. 30, the outlet end 9720 and the part-annular side wall 9790 cooperate to form an annular channel 9780 for receiving the air delivery tube 4170. Each of two opposing sides of the part-annular side wall 9790 includes a hole or recess 9792 (Fig. 30) adapted to receive a respective retaining bump 4644 (Fig. 26) provided to the dock connector 4600 of the air delivery tube 4170 during engagement. In the illustrated example, a gap is provided in the part-annular side wall 9790 (along a superior side thereof) to accommodate and facilitate the electrical connection of the dock connector 4600 of the air delivery tube 4170.

Also, the intermediate component 9700 includes a lower tab 9795 (Fig. 30) that projects outwardly and downwardly from the part-annular side wall 9790 along a portion of the perimeter of the part-annular side wall 9790 (along an inferior side thereof). The lower tab 9795 may acts as a finger or push tab to facilitate insertion of the intermediate component 9700 into the reservoir dock 6050 or an extraction of the intermediate component 9700 from the reservoir dock 6050. In addition, the lower tab 9795 may be configured and arranged to cover or hide one or more fasteners (e.g., screws) or edges between outer shroud and chassis components of the integrated RPT device and humidifier 6000 (see Fig. 23 and 26).

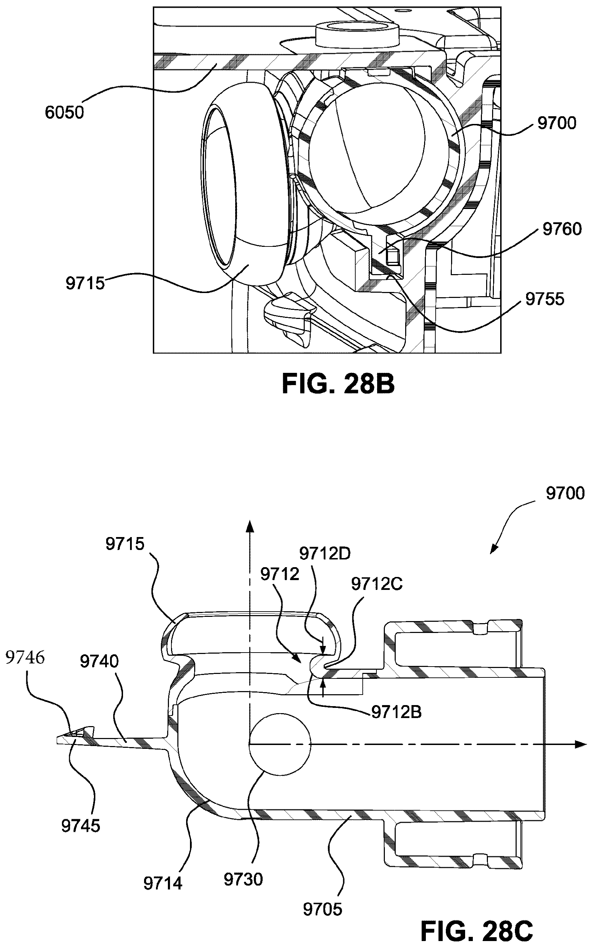

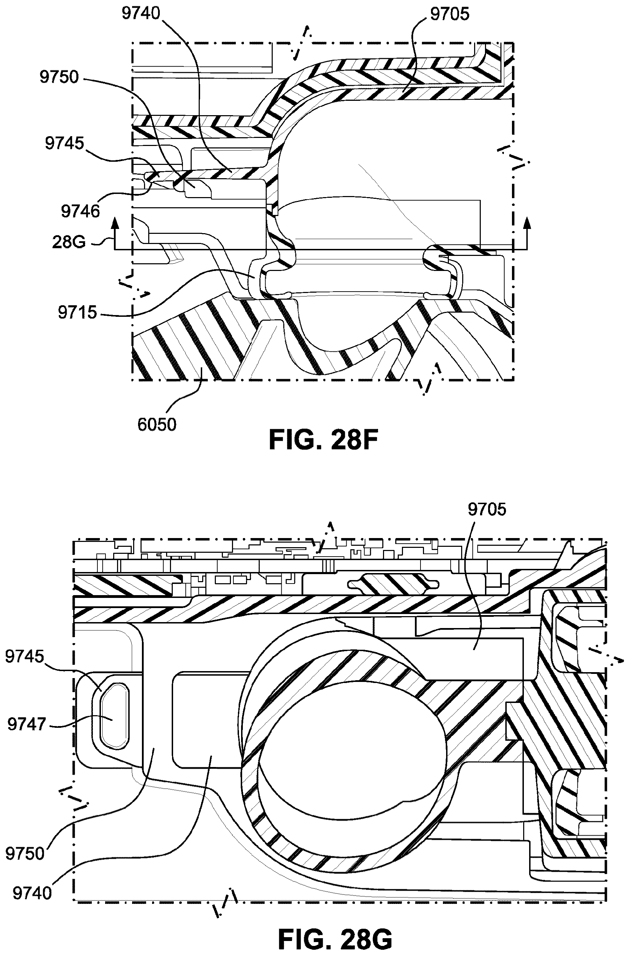

When the intermediate component 9700 is inserted into the dock opening 6091 of the reservoir dock 6050, the intermediate component 9700 is oriented to engage its guide rail 9760 and guide rib 9761 with respective guide slots 9755 which correctly aligns and guides the intermediate component 9700 into an operative position (e.g., see Fig. 26). Also, the dock opening 6091 and the part-annular side wall 9790 of the intermediate component 9700 include non-circular profiles to facilitate correct orientation of the intermediate component 9700 during insertion. When the intermediate component 9700 reaches an operative position, the barbed end or tab 9745 of the spring or pinch arm 9740 is configured and arranged to engage under and behind the cross-bar 9750, e.g., see Figs. 25, 28F and 28G. The barbed end 9745 and/or the cross-bar 9750 may include a taper to facilitate engagement into the operative position, e.g., see Fig. 28F. In an example, the engagement of the spring or pinch arm 9740 with the cross-bar 9750 (e.g., see Figs. 25, 28F and 28G) may provide sensory feedback, e.g., audible click, to indicate correction connection. This snap-fit connection releasably secures the intermediate component 9700 to the reservoir dock 6050. As shown in Fig. 28C and 28F, one side surface 9746 of the barbed end 9745 may be provided at an angle to an opposite side surface of barbed end 9745. As the chassis (e.g., cross-bar 9750) engages against the angled surface 9746 of the barbed end 9745, during insertion of the intermediate component 9700, the barbed end 9745 may be forced to flex until the portion of the chassis engaging the side surface 9746 extends past the side surface 9746. To disengage the intermediate component 9700, the spring or pinch arm 9740 can be manually depressed towards the back of the reservoir dock 6050 (e.g., with or without a tool). Such pressure can resiliently flex the spring or pinch arm 9740 and barbed end 9745 thereof to an unlocked position, i.e., where barbed end 9745 is moved out of engagement with the cross-bar 9750 to allow the intermediate component 9700 to be removed from the reservoir dock 6050. In some examples, the barbed end 9745 may include a groove 9747 (see e.g., Figs. 28G and 29) to allow a tool (e.g., a flat-head screw driver) to grip with the barbed end 9745 for disengaging the barbed end 9745 from the reservoir dock 6050.

As shown in Figs. 28C and 28G, the pinch arm 9740 may be provided at an angle relative to the central axis of the tubular body 9705. The pinch arm 9740 may be angled such that an angle between an axis of the opening at the inlet end 9710 and the pinch arm 9740 is less than 90°. The angled pinch arm 9740 may provide for increased retention as compared to a pinch arm 9740 that is not angled relative to the central axis of the tubular body 9705.

Once the intermediate component 9700 is inserted and locked into the dock opening 6091 of the reservoir dock 6050, the cooperating retention and alignment features provided by the intermediate component 9700/reservoir dock 6050 provides a removable, non-rotatable connection of the intermediate component 9700 to the dock outlet 6090 of the reservoir dock 6050. Also, once connected, the spring or pinch arm 9740 of the intermediate component 9700 is lockingly engaged within the cavity of the reservoir dock 6050, e.g., to prevent removal of the intermediate component 9700 when the water reservoir 6100 is received in the reservoir dock 6050.

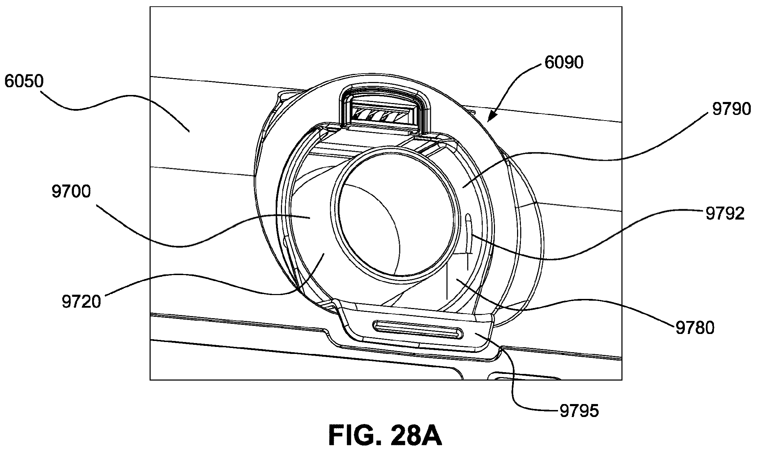

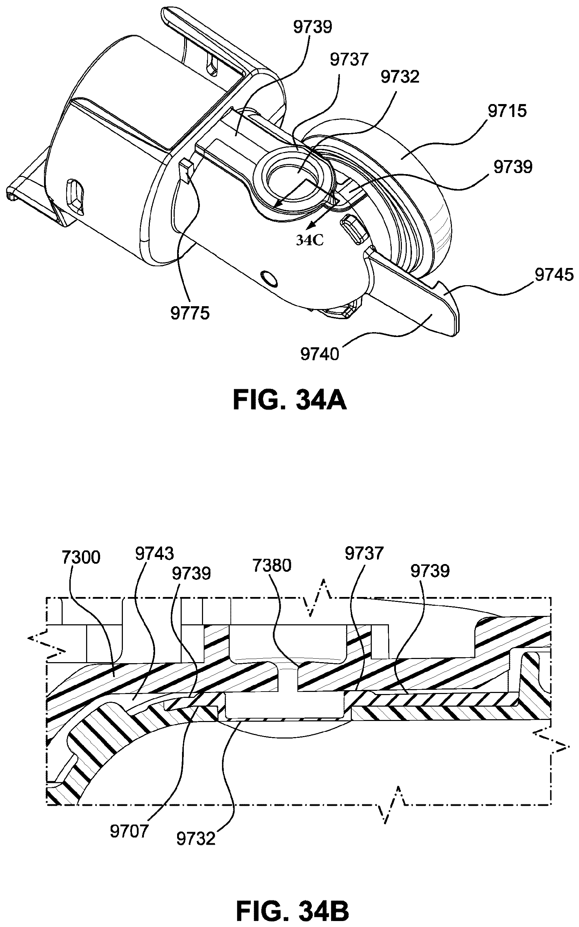

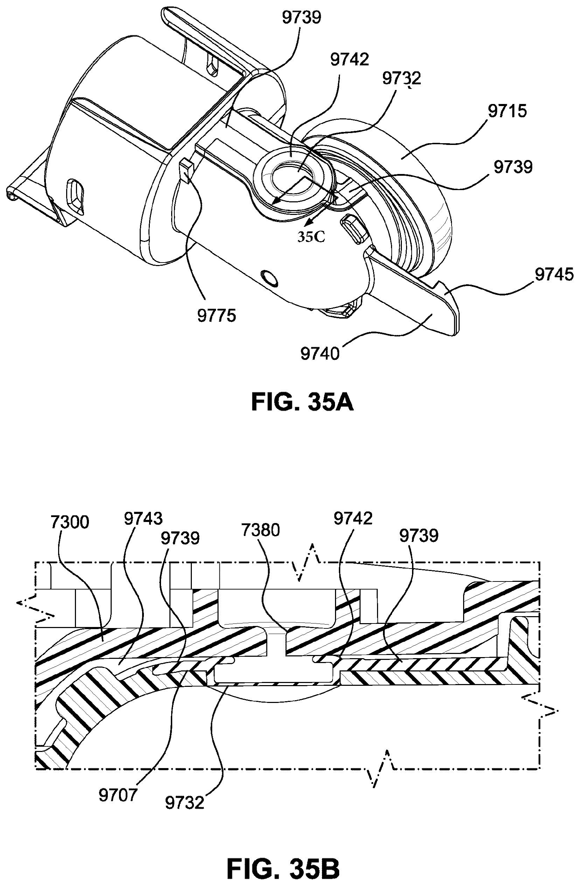

When the intermediate component 9700 is connected to the dock outlet 6090 of the reservoir dock 6050, the inlet seal 9715 thereof protrudes into the cavity of the reservoir dock 6050 to allow engagement with the outlet tube 6130 (or outlet) of the water reservoir 6100 (see Fig. 25). Likewise, the outlet end 9720, along with the part-annular side wall 9790 and holes 9792 thereof, extends within and/or protrudes out of the cavity of the reservoir dock 6050 to allow engagement with the air delivery tube 4170, e.g., see Figs. 23 and 28A. Further, the port 9730 and port seal 9735 thereof, are oriented, to interface with the chassis opening 7380 associated with the sensor, e.g., upwardly as shown in Fig. 34B and 35B.

### 5.8.4 Propagation of Sound in the Intermediate Component

As discussed above, the intermediate component 9700 facilitates a connection between the conduit of the air circuit 4170 and the RPT device (e.g., the reservoir dock 6050 and/or the water reservoir 6100). The intermediate component 9700 in accordance with an aspect of the present technology includes a port 9730 (e.g., a sound port) to facilitate propagation of sound from the intermediate component 9700 to a sensor 4270 (e.g., a microphone) disposed in the RPT device. The sensor 4270 may be attached externally to the intermediate component 9700. While being externally attached, the sensor 4270 can be disposed outside of the intermediate component 9700 and/or the port 9730, or inside (e.g., completely or partially inserted) of the intermediate component 9700 and/or the port 9730. Thus, sounds from any part of the patient interface 300, the RPT device 400, the humidifier 500 and/or the air circuit 4170, which can reach the intermediate component 9700, may be passed on to a sensor 4270 (e.g., a microphone) disposed in the RPT device. As will be discussed in more detail below, the sensed sound can be analysed to determine features of and/or within the respective part (e.g. air circuit 4170) in which the sounds originate from. The structure and dimensions of the intermediate component 9700 are configured to improve the propagation of the sound inside the intermediate component 9700 and to the port 9730. Two variants of the intermediate body have been discussed here (6700 and 9700). For example, Figs. 19-22 depict an intermediate component 6700 that included the electrical connection via a secondary body (e.g., locking and contact assembly 6900), as well a non-circular membrane. However, both 6700 and 9700 variations of the intermediate body behave in the same manner for a sound propagation design intent.

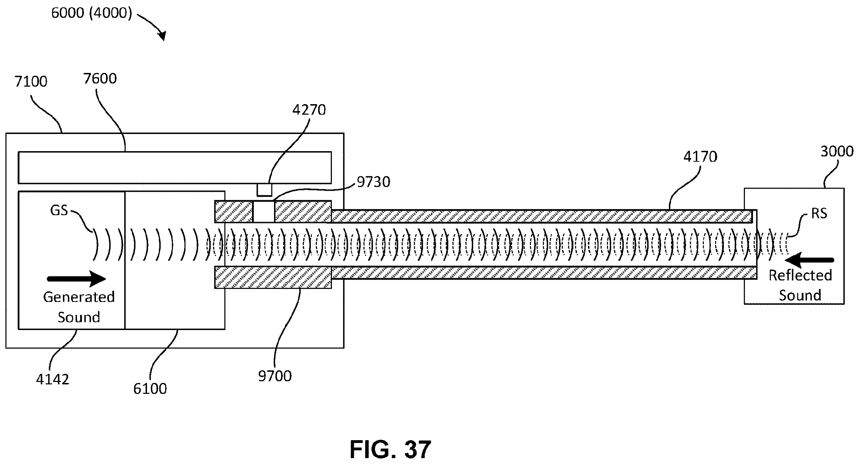

As shown in Figs. 28C, 29, and 31-36C, the intermediate component 9700 includes a port 9730 to facilitate the propagation of a portion of the sound propagating in the airflow path, to the sensor 4270. In particular, the port 9730 allows for a portion of the sound in the airflow path to be passed on by intermediate component 9700, though the port 9730, via a corresponding chassis opening 7380 in the chassis and to the sensor 4270 (e.g., a microphone located inside the chassis - see Fig. 37). The chassis opening 7380 may be provided in a portion of the chassis forming a water reservoir dock (e.g., reservoir dock 6050) that receives a humidification tub. Accordingly, a chassis opening 7380 may correspond to an opening in the water reservoir dock. In other examples, the chassis opening 7380 may be provided in another portion of the chassis. For example, the chassis opening 7380 may be provided in a portion of the chassis 7300 that a different component from the water reservoir dock. As shown in Figs. 32 and 33, the port 9730 comprises an opening in the wall of the intermediate component 9700 and is located near the inlet end 9710. An axis associated with the port 9730 (say the axis being generally transverse to the plane in which the port opening extends) may be arranged at about a 90° with respect to an axis of the opening at the outlet end 9720 and/or at about a 90° with respect to an axis of the opening at the inlet end 9710 (see Figs. 26, 28C, and 36C).

The size of the port may be selected based on the parameters of the sound signals targeted for detection. These could be sound signals generated directly by any of the components in the RPT system (including the RPT device, the humidifier, the air circuit and the patient interface), and/or sound signals generated at one location, but then propagated to and reflected back by, a specific targeted component. The size of the cavity reached by the sound after passing from the intermediate component 9700 and through the port 9730, also matters. In one example, such a cavity may be formed by the wall of the intermediate component 9700 and the PCBA 7600 supporting the sensor 4270, as schematically shown in Fig. 37. A larger size of the port 9730 may introduce spatial averaging and diminish the spatial resolution, but may allow for more sound signal reaching the sensor and improve the signal to noise ratio. In cases where there is more than adequate signal to noise ratio, smaller port dimensions may be preferred in order to retain higher resolution signal information, such as that relating to sound reflected by smaller geometrical features. A larger port 9730 and large adjacent cavity formed by the port, may create a discontinuity in the cross-sectional area or the acoustic impedance of the flow path within the intermediate component and disrupt the signal propagation. Reducing outside noise (e.g., noise from vibrations in a PCBA 7600 supporting the sensor 4270) may allow for the aperture of the port to be reduced and provide better spatial resolution and a reduced signal disruption.

According to one form of the present technology, the port size may be configured to be large enough to allow appropriate target signal level to the sensor 4270 disposed outside of the intermediate component 9700, but not too large to compromise to an inacceptable level the spatial resolution and/or the waveguide nature of the signal path through the intermediate component 9700. According to one form of the present technology, the port 9730 may have a cross section that is in between 0.75 and 180 mm$^2$. For example, in one form of the present technology, the port 9730 may be round and have a diameter in the range of 1mm to 15mm.

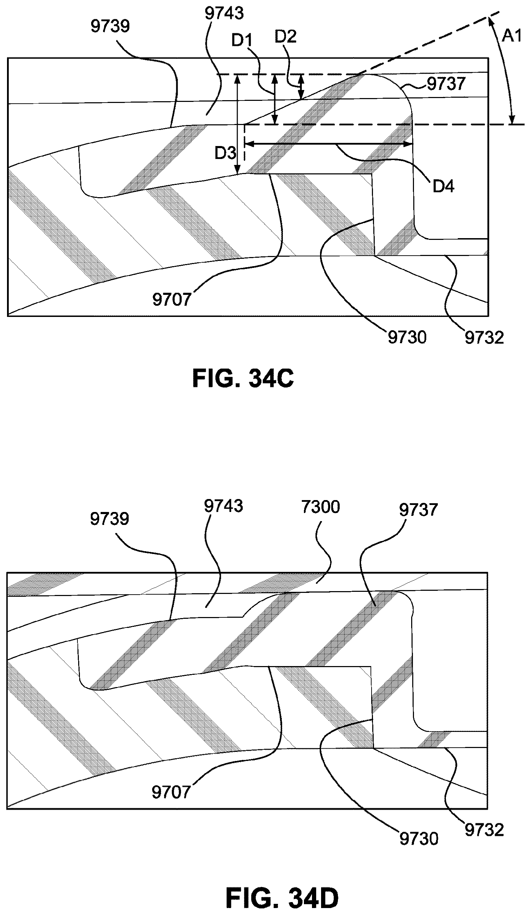

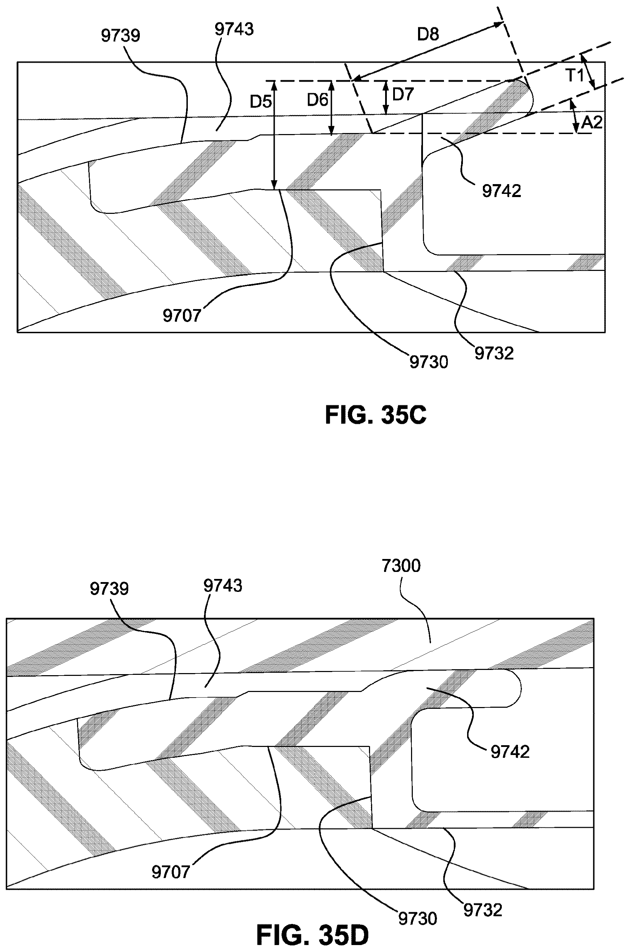

According to one form of the present technology shown in Figs. 34 and 35, the port 9730 is covered by a thin silicone membrane 9732. The membrane 9732 is intended to keep the sensor out of the airflow, whilst still transferring sound propagation along the air-path (airflow) to the sensor. This is why the membrane may be sound permeable, but it is preferably impermeable to liquids and/or gases. Because of this arrangement, when we remove and decontaminate the intermediate component 9700 (or any other component that is in the air-path or is fluidly connected to the air path, but is arranged to enable sound transfer to a shielded sensor located out of the air-path), the sensor itself does not need to be removed and cleaned, as it has not been in the air-path and has not been exposed to contaminants. In some examples, the membrane 9732 is of as large of a diameter (or other cross-sectional dimensions) as possible, and as thin as possible. Such dimensions increase the amount of target signal (directly generated or reflected noise) that is transferred across the membrane. In some examples, the membrane 9732 may have a thickness of between 0.05 to 3mm; or 0.1 to 1mm; or 0.1 to 0.3 mm and a diameter in the range 1 to 15 mm. In some examples, the membrane 9732 may have a thickness of between 0.1 to 0.2 mm and a diameter in the range 1 to 15 mm. In some examples, the membrane 9732 may have a thickness of about 0.2 mm and a diameter in the range 1 to 15 mm, and in one example a diameter of about 8 mm. The example dimensions provide for the membrane to transmit sound sufficiently well across the membrane 9732, while at the same time limit the leakage of the sound signal across the intermediate component 9700 walls to which the membrane is attached. In some examples, the membrane may be provided such that it is planar with the inside surface of the intermediate component 9700. The membrane 9732 may be formed as one integral component with the port seal 9735, or may be provided separate from the port seal 9735. The membrane 9732 may be in, or in no, mechanical contact with the port seal 9735. In the embodiments shown in Figs. 34 and 35, the membrane 9732 is formed as one integral component with the port seal 9735.

According to one form of the present technology, the port 9730 may be without a membrane 9732 and/or the port seal 9737. As was explained above, one benefit of having a membrane 9732 is in that it prevents air from the intermediate component from entering the chassis, thus separating the electronic components on the PCBA from the airflow gasses. This prevents humidified and/or contaminated air from entering the microphone chamber and protects the electronics from moisture or contaminated air (e.g. containing human bodily fluids such as mucus). From a therapy performance point of view, it is desirable to control the leak when providing PAP therapy. The presence of a membrane reduces the unintentional leak from the system. An arrangement where the port 9730 is without a membrane 9732 is also possible. In this case a bleed of high pressure may be introduced inside of the chassis to prevent air from entering the chassis from the intermediate component 9700. Alternatively, an encapsulated sensor may be introduced into the airpath. In this case the sensor may be encapsulated (say in a silicone, rubber or a membrane of another material that will transmit the sound vibrations through and at the same time be able to be decontaminated (e.g. it is washable)). Such a sensor may be made a permanent part of the intermediate component 9700, or any other component that is in the air-path or is fluidly connected to the air path and to which the sensor may be attached to. Because the sensor is encapsulated, it will be able to be removed and cleaned with the respective intermediate component. The electrical terminals of the sensor in this case have to be arranged for disconnection and re-connection during the dissembling /assembling process.

However, apart from restricting the movement of air, the use of membrane 9732 may have other benefits, such as infection control and preventing damage to the circuitry of the PCBA 7600. When a membrane 9732 is used, it may be beneficial to provide a membrane that is as flexible and as light as possible in order to transmit a large range of signal frequencies. According to one form of the present technology, the preferred mass and stiffness may be determined by the density and dimensions of the membrane 9732, and the pressure gradient across the membrane. According to one form of the present technology, for a port 9730 having a 5mm diameter, a thickness in the range between 0.1 and 0.4 mm, or approximately 0.3mm, may provide a reasonable compromise between being sufficiently thick to avoid structural failure, yet being thin enough to allow appropriate sound transmission.

Figs. 34A-35D show examples of a port 9730 provided with a port seal 9735 according to various examples of the present technology. The port seal 9735 provides a peripheral sealing formation (a ridge or a lip) that may be part of the membrane 9732 or may be separate from the membrane 9732. The peripheral sealing formation may include radial seal configured to engage (e.g. as a face seal) and resiliently deform against the surface of the chassis above the intermediate component 9700 and around the chassis opening 7380.

The port seal 9735 can encircle the port 9730 and protrude beyond the port 9730. As shown in Figs. 34A-35D, the port seal 9735 protrudes from the port 9730 and above the exterior surface of the intermediate component 9700. In use, when the intermediate component 9700 is inserted into operational location, the port 9730 may be aligned with the chassis opening 7380 providing a sealed pathway for the sound signal (from the intermediate component 9700, through the port 9730 and the membrane 9732 and through the chassis opening 7380), with the sensor 4270 positioned on the oppose side of the chassis opening 7380.

A central axis of the port 9730 in the intermediate component 9700 may be generally aligned with a central axis of the chassis opening 7380 on a first side of the chassis opening 7380 (see Figs. 35A-36C) and the sensor 4270 may be provided on a second side of the chassis opening 7380. The sensor 4270 may be generally aligned with the central axes of the port 9730 and/or the chassis opening 7380. The axis of the port 9730, the axis of the chassis opening 7380 and the sensor 4270 may be so generally aligned that in the operational configuration, the distance between any two of them is less than; 5mm, preferably 4mm, even more preferably 3mm, further preferably 2mm, most preferably - less than 1mm.

As was already described in the text above, upon assembly of the intermediate component 9700 (by way of inserting the intermediate component 9700 into the dock opening 6091 of the reservoir dock 6050), the port seal 9735 is brought in contact with the periphery of the chassis opening 7380. Because the port seal 9735 contacts the periphery of both the intermediate component 9700 and the chassis opening 7380, when the intermediate component 9700 is assembled, the port seal 9735 seals the periphery of both the port 9730 and the chassis opening 7380. This minimizes the lateral escape of the sound signal transferred via the thin membrane 9732 into the space between the intermediate component wall and the chassis wall. As well as preventing sidewise leak of the sound from the sealed sound pathway into the space 9743 between the intermediate component 9700 wall and the wall of the chassis, the peripheral lip of the port seal 9735 also prevents the entrance of external noise into the sealed signal pathway.

Figs. 34A-34D show an example of a port seal 9735 providing a compression-type seal against the chassis wall by using a ridge according to an example of the present technology. Figs. 34B and 34C show a cross-sectional view of an example of a ridge without compression. Fig. 34D shows the ridge being compressed by the wall of the chassis 7300. The port seal 9735, providing the compression seal, may comprise an overmold constructed of a relatively soft material (e.g., thermoplastic elastomer (TPE) or silicone) that is provided (e.g., by overmolding) to the intermediate component 9700. The port seal 9735 may extend from and/or include the membrane 9732 that is provided at or near the inner surface of the intermediate component 9700, along the inner wall of the port 9730 and protrude from the port 9730 above the exterior surface 9707 of the intermediate component 9700 (e.g., near the edge of the port 9730). The portion of the port seal 9735 protruding above the exterior surface of the intermediate component 9700 may comprise a ridge seal 9737 providing a spherical sealing contact between the port seal 9735 and the chassis during use. In some examples, one or more additional ridge seals (e.g. disposed concentrically) may be provided around the ridge seal 9737.

The cross section of the ridge seal 9737 above the exterior surface of the intermediate component 9700 may be rounded and extend in the outward direction from the perimeter of the port 9730 a predetermined distance D4 (see Fig.34C), before it merges with a surface that is, or is an extension of, the upper surface of the intermediate component wall. D4 may be between 1.2 and 2.8 mm, 1.8 and 2.2 mm, or approximately 2 mm. The sides of the ridge seal 9737 may slope away from the rounded top on either side. The slope toward the centre of the port 9730 may be greater than the slope directed away from the port 9730. The portion of the ridge seal 9737 extending in the outward direction from the perimeter of the port 9730 may be provided at an angle A1 relative to the exterior surface of the intermediate component 9700 that is between 10-35 degrees, in some examples at an angle between 20-25 degrees, and in some examples at an angle of 22 degrees. The portion of the ridge seal 9737 extending in the outward direction may extend outward at an angle and terminate at the upper surface of the of the intermediate component 9700 which may correspond to an surface near the edge of the port 9730 or another exterior surface of the intermediate component 9700.

In some examples, the port seal 9735 may include one or more connecting portions 9739 (see Fig. 34C) adjacent to the ridge seal 9737 and extending in one or more directions and/or connecting to other features (e.g., bumper 9775 and/or inlet seal 9715 - see Fig. 34A). The top surface of the one or more connecting portions 9739 may be level with portions of the exterior surface of the intermediate component 9700. In one example, the connecting portion 9739 may extend around the entire perimeter of the ridge seal 9737. The ridge seal 9737 may extend upward a predetermined distance D1 (see Fig. 34C) above the surface of the connecting portions 9739. The predetermined distance D1 may be between 0.4 to 0.8 mm or approximately 0.6 mm.

In use, a portion of the ridge seal 9737 above the exterior surface of the intermediate component 9700 may be compressed against the chassis at a predetermined distance D2, before it bottoms out. In some examples, the material of the port seal 9735 above the exterior surface 9707 of the intermediate component 9700 (e.g., near the edge of the port 9730) may be configured to compress approximately 20-40% of its height and in some examples approximately 30% of its height. In some examples, the port seal 9735 may extend upward from the exterior surface 9707 of the intermediate component 9700 (e.g., near the edge of the port 9730) a predetermined distance D3 (see Fig. 34C), which may be between 1 and 1.5 mm, between 1.2 and 1.3 mm, or approximately 1.24 mm. In some examples, the nominal seal interference (a measure of the deformation of the seal under compression that may be equivalent to D2) may be between 0.09 mm and 0.59 mm, between 0.15 mm and 0.5 mm, or approximately 0.34 mm.

The compression-type and the spherically -shaped sealing contact of the ridge seal 9737 may provide for the intermediate component 9700 to snap into position when the intermediate component 9700 reaches an operative position during assembly and/or prevent the intermediate component 9700 from disassembling in the absence of appreciable external force. In some example, the compression of the port seal 9735 may maintain the intermediate component 9700 in position even when a barbed end 9745 is moved out of engagement with the cross-bar 9750, to allow for an easy manipulation when the intermediate component 9700 is removed from the reservoir dock 6050.

Figs. 35A-35D show an example of a port seal 9735 providing a lip-type seal according to another example of the present technology. Figs. 35B and 35C show an example of a lip seal without compression (e.g., without being pressed against the chassis 7300) and Fig. 34D shows the lip seal being compressed by the chassis 7300. The port seal 9735 providing the lip seal may comprise an overmold constructed of a relatively soft material (e.g., thermoplastic elastomer (TPE) or silicone) that is provided (e.g., by overmolding) to the intermediate component 9700. As shown in Figs. 35A-35D, the port seal 9735 may extend from and/or include the membrane 9732, that is provided at or near the inner surface of the intermediate component 9700, along the inner wall of the port 9730 to line the interior surface of the port 9730, and protrude from the port 9730 above the exterior surface 9707 of the intermediate component 9700 (e.g., near the edge of the port 9730). The portion of the port seal 9735 protruding above the exterior surface of the intermediate component 9700 may comprise a lip seal 9742 providing a spherical sealing contact between the port seal 9735 and the chassis during use. In some examples, one or more additional lip seals may be provided around the lip seal 9742.

The cross section of the lip seal 9742 above the exterior surface of the intermediate component 9700 may line up with the interior surface of the port 9730 and/or provide a lip that extends concentrically towards a centre of the port 9730 around the perimeter of the port 9730. In some examples, the lip may be provided at a predetermined angle A2 (see Fig. 35C) relative to the upper exterior surface of the intermediate component 9700. The angle A2 may be between 10-35 degrees, between 15-25 degrees and/or about 20 degrees. The lip seal 9742 may extend upward a predetermined distance D5 (see Fig. 35C) from the underlying surface of the intermediate component 9700. The distance D5 can be approximately 1.3 to 1.7 mm, 1.4 to 1.6 mm or 1.56 mm. The lip seal 9742 may have a thickness T1 (see Fig. 35C) which is approximately between 0.2 to 0.6 mm, 0.35-0.45 mm, or 0.4 mm. In some examples, the end of the lip seal 9742 extending above the port 9730 may be rounded. The dimensions of the lip seal 9742 and/or angle at which the lip seal 9742 is provided may be selected to improve acoustic seal and/or reduce lip seal bulking during use. In some examples, the lip may extend directly upwardly, or at an angle away from the centre of the port 9730.

The lip seal 9742 may extend a predetermined distance D8 (see Fig. 35C) from the edge of the port 9730 at the predetermined angle A2. The predetermined distance D8 may be limited by the edge where the upper surface of the lip seal 9742 merges with the surface, or with an extension of the surface of the intermediate component 9700. The distance D8 may be in the range between 0.1 and 3.5mm, in the range between 0.15 and 3mm, or approximately 2 mm.

In some examples, the port seal 9735 may include one or more connecting portions 9739 adjacent to the lip seal 9742 and extending in one or more directions and/or connecting to other features (e.g., bumper 9775 and/or inlet seal 9715 - see Fig. 35A). The top surface of the one or more connecting portions 9739 may be level with portions of the exterior surface of the intermediate component 9700. In one example, the connecting portion may extend around the entire circular perimeter of the lip seal 9742. The lip seal 9742 may extend upward from the upper surface of the intermediate component 9700 a predetermined distance D6 (see Fig. 35C), which is between 0.4 to 0.8 mm or approximately 0.66 mm above the surface of the connecting portions 9739.

In use, a portion of the lip seal 9742 above the exterior surface of the intermediate component 9700 may compress and/or deflected towards the port 9730 and against the chassis. In some examples, the lip seal 9742 may be deflected between a predetermined distance D7 (see Fig. 35C) before it bottoms out, which is between 0.1 and 0.6 mm or between 0.15 and 0.41 mm. In some examples, the nominal seal interference of the lip seal 9742 may be approximately 0.41 mm, the minimum seal interference may be approximately 0.15 mm, and/or maximum seal interference may be approximately 0.66 mm.

The lip seal 9742 may assist to retain the intermediate component 9700 in position when the intermediate component 9700 reaches an operative position during assembly and/or prevent the intermediate component 9700 from dissembling in the absence of appreciable external force.

Two types of sealing formations have been disclosed above. In the port seal 9735 providing the compression seal with the ridge seal 9737 (see Figs. 34A-34D), the cross-section of the sealing formation that protrudes above the intermediate component 9700 surface is more substantial and bulbous than the lip seal 9742. Because of the mass of the ridge seal 9737 with the ridge seal 9737 may compress more easily and it may offer less friction when the intermediate component 9700 is inserted inside the chassis. In the alternative lip seal example (see Figs. 34A-34D), the portion of the lip seal 9742 protruding above the intermediate component 9700 surface is a thin lip that, when compressed, may exhibit limited bending and flexibility. The lip seal 9742 may create appreciable friction between the intermediate component 9700 and the surrounding chassis wall when the intermediate component 9700 is inserted inside the chassis7300.

As discussed above the ridge seal 9737 or lip seal 9742 provide sound isolation of the seal -formed signal path, from the remaining space 9743 between the intermediate component 9700 and the chassis 7300. When the intermediate component 9700 is positioned in the chassis 7300 for use, the distance between the chassis 7300 and the surface of the intermediate component 9700 or the connecting portions 9739, which defines space 9743 (see Fig. 35C), may be between 0.15 and 0.4 mm, 0.2 and 0.3 mm, or 0.25 mm.

In some examples of the present technology, the port seal 9735 may be provided on the surface of the chassis 7300. In this example, the port seal 9735 may be provided on the chassis 7300 instead of the intermediate component 9700. The port seal 9735 may be provided such that it is provided in the chassis opening 7380. The port seal 9735 in the chassis opening 7380 may include a peripheral sealing formation (a ridge or a lip) configured to engage (e.g. as a face seal) and resiliently deform against the surface of the intermediate component below the chassis opening 7380 and around the port 9730.

Features inside the intermediate component 9700 effect the propagation of sound waves (e.g., reflection, refraction, and/or attenuation) inside the intermediate component 9700 and to the port 9730. Examples of the present technology provide features inside the intermediate component 9700 that assist in carrying useful reflected signals from the air circuit 4170 (e.g., tube and/or mask) to the sensor 4270 via the port 9730.

As shown in the figures, the air path between an inlet end and the outlet end of the intermediate component 9700 is nonlinear and includes at least one turn. According to one form of the present technology, to provide proper propagation in the intermediate component 9700, corners inside the intermediate component 9700 are curved. In some examples, at least one of the turns closest to the port may be curved. In one example of the present technology all of the corners inside of the intermediate component 9700 are curved. If the corners are sharp (e.g., 90° angle), the level and quality of the sound signal may deteriorate, even if the overall sound level is higher due to the turbulence created by the sharp corner. Providing corners (e.g., the outer corner 9714, and especially the inner corner 9712) with a curvature, can improve the quality of the sound signal.

Fig. 28C shows a cross section of an intermediate component 9700 according to an example of the present technology. As shown in Fig. 28C, an outer corner 9714 and/or an inner corner 9712, formed between the main body of the intermediate component 9700 and the inlet seal 9715, are curved. In some examples, the inlet seal 9715 at the inlet end 9710 (see Fig. 32) may be arranged at an angle to the outlet end 9720 and/or the main body of the intermediate component 9700. The angle may be approximately 90° or greater than 90°.

In some examples, the curvature formed by the inner corner 9712 may have a radius of 0.2mm-6.5mm, 0.3mm-4mm, 0.4mm - 3mm (such as 0.4 mm or 2 mm), however the range can also be 1.5-6.5 mm 2.5-5 mm etc.

The curved corners (e.g., inner corner 9712) may be provided as part of one or more seal bellows of the inlet seal 9715. As shown in Fig. 28C, the seal bellow of the inlet seal 9715 may include an inner surface 9712B and an outer surface 9712C. The curvature of the inner surface 9712B may determine how sound propagates inside the intermediate component 9700. The outer surface 9712C may include a radius that is approximately 0.2-0.6 mm or 0.4 mm. The inner surface 9712B may include a radius that is approximately 1.5-6.5 mm, 2.5-5 mm, or 2 mm. In some examples, the outer surface radius may be 0.4 mm and the inner surface radius may be 2 mm. In some examples of the present technology, the outer surface radius may be less than half of the inner surface radius. In some examples, a span 9712D between opposite sides of the seal bellow in the intermediate component 9700 may be equal to or less than twice the radius of the inner surface radius.

While the port 9730 and the port seal 9735 is described above with reference to the intermediate component 9700, one or more features of the port 9730 and the port seal 9735 may be provided in the intermediate component 6700 (e.g., in place of port 6730 show in Fig. 22), a portion of the conduit in the air circuit 4170, or dock connector 4600.

### 5.8.5 Features Mitigating the Intermediate Component Seal Insertion Friction

The intermediate component 9700 and the dock outlet 6090 of the RPT device 6000 are arranged so that, during most of the length of the path along which the intermediate component 9700 is inserted into the dock outlet 6090, the path is slightly wider than the intermediate component 9700 diameter and the port seal 9735 (e.g., silicone ridge or lip of the port seal 9735) does not interact with the path wall. This ensures a relatively frictionless insertion of the intermediate component 9700 into the dock outlet 6090. In some examples of the present technology, an elevation of the bottom of the chassis path and/or downward extension et lower portions of the intermediate component may be configured to push the intermediate component 9700 upwardly (with reference to the direction of initial movement of the intermediate component 9700). In the example, this is done shortly before the intermediate component 9700 is locked into its operational position (e.g., via the pinch arm 9740). Because of the upward pressure, the port seal 9735 (e.g., silicone peripheral lip) moves into engagement with the chassis and seal the periphery of the chassis opening 7380.

In some examples, only at the very end of the path, shortly before the intermediate component 9700 is fully locked into its operational position, the elevation of the bottom of the path may push the intermediate component 9700 upwardly and into its operational configuration. In this example, due to the specific structure of the port seal 9735, the intermediate component 9700 and the chassis opening 7380, a user inserting the intermediate component 9700 into the chassis opening 7380 encounters an increased resistance at the very end of the insertion path. The resistance may be encountered at a location where the intermediate component 9700 (one or more features of the intermediate component such as bumpers) encounters an opening of a smaller diameter, which causes additional friction between the ridge or lip of the port seal 9735 and the chassis wall.

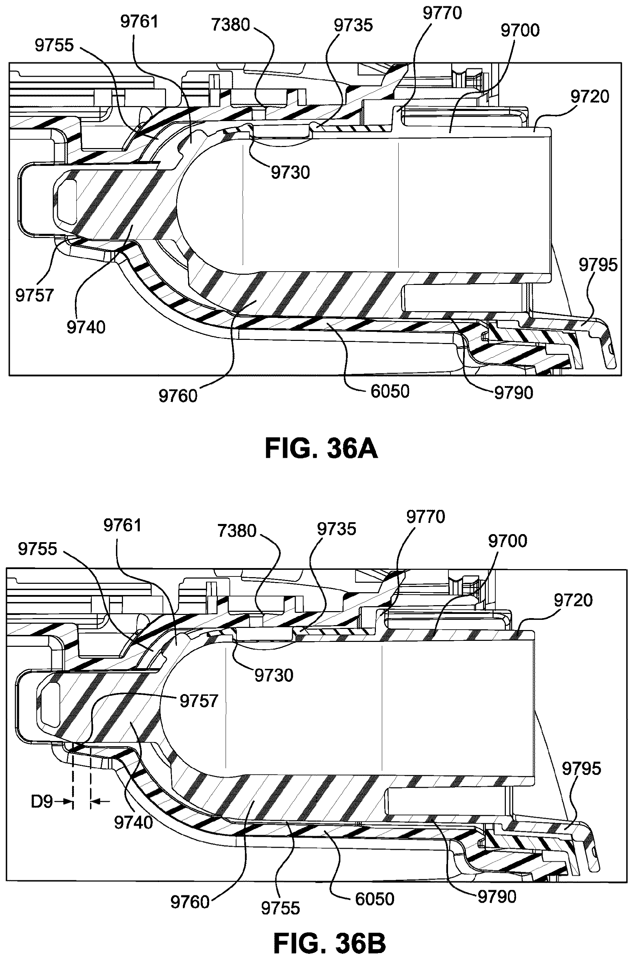

Figs. 36A-36C show the specific sequence of events, as the intermediate component 9700 is being inserted into the receiving reservoir dock 6050 (e.g., dock outlet 6090 of the reservoir dock 6050), according to an example of the present technology. As shown in the figures, in the specific illustrated example both the intermediate component and the receiving opening are of generally tubular shape. The intermediate component 9700 includes a guide rail 9760 (Figs. 36B) (along a lower side of the intermediate component 9700) and a guide rib 9761 (Figs. 36B) (along a forward, upper side of the intermediate component 9700) structured and arranged to assist in correct alignment and insertion of the intermediate component 9700 into the reservoir dock 6050 by engagement with corresponding guide slots 9755 extending into the cavity of the reservoir dock 6050. As the intermediate component 9700 is inserted reservoir dock 6050, the guide rail 9760 and/or the guide rib 9761 follow the chassis guide slots 9755 (shown in Figs. 28B, 28D and 28E). As shown in Fig. 36A and as mentioned in the above text, a clearance is provided between the port seal 9735 (e.g., silicone ridge or lip of the port seal 9735) and the chassis when the intermediate component 9700 is initially inserted into reservoir dock 6050. The clearance between the port seal 9735 and the chassis 7300 reduces the intermediate component 9700 assembly force and/or reduces and/or prevents excessive deformation of the port seal 9735.

Fig. 36B shows a beginning of engagement between the intermediate component 9700 and the respective opening of the receiving reservoir dock 6050, at one or more (in this case - three) engagement formation points L1, L2 and L3 (see Fig. 36C for the engagement formation points L1-L3). Each of these points marks the place where, during the progressive movement of the intermediate component 9700 towards a full engagement with the dock opening, a structural feature of the intermediate component 9700 (such as guide rail, a rib or a bumper) engages a respective engagement feature of the dock opening (e.g., a lower edge of arm 9740 engages sloped ridge 9757 at L1, an edge of guide rail 9760 engages an elevated section of guide slot 9755 at L2 etc.). The engagement in these points may be simultaneous or sequential and it aims to guide the intermediate component 9700 into its fully engaged configuration, where barb end 9745 is securely locked to a respective flange on the chassis/dock opening, and where port seal 9735 has reliably sealed the port 9730. As a result, the port seal 9735 (e.g., silicone ridge or lip of the port seal 9735) also begins to engage the chassis 7300 according to an example of the present technology. As shown in Fig. 36A and 35B, the port seal 9735 may begin to engage the chassis 7300 after an edge of the port 9730 passes a central axis and/or edge of the chassis opening 7380. The snap-fit connection of the intermediate component 9700 (e.g., provided by the barbed end or tab 9745 spring or pinch arm 9740) may be glided up the sloped ridge 9757 provided in the lower portion of the chassis before the snap-fit connection is made. As the snap-fit connection is glided up, guided by an elevation path provided at one or more of the three engagement formation points L1 to L3, the port seal 9735 begins to engage the chassis near the chassis opening 7380. After sliding up the sloped ridge 9757, the snap-fit connection may travel further a predetermine distance (e.g., 1 mm) before completely engaging the snap. The sloped ridge 9757 may be provided at an angle that is between 15 and 25 degrees, 18 and 22 degrees, or 20 degrees. In one example, the sloped ridge 9757 may be provided at an angle of approximately 20 degrees and span a predetermined distance D9 that is between 1.5 and 2.5, 1.8 and 2.2 mm, or 2mm.

Fig. 36C shows the port seal 9735 (e.g., silicone ridge or lip of the port seal 9735) engaging the chassis in a fully assembled position according to an example of the present technology. One or more features in the intermediate component 9700 and/or in the chassis opening may be configured to effect the engagement of these two components by pushing the intermediate component 9700 upwards towards the chassis opening 7380 and/or ensuring a reliable engagement and acoustic seal in the fully assembled configuration. For example, the compression engagement of the port seal 9735 may be effected and/or controlled by one or more of; a step in one or both of a lower edge of arm 9740 and/or in the sloped ridge 9757 in the cantilever snap-fit location (L1), an edge of guide rail 9760 engages an elevated section of guide slot 9755 (L2) (to lift the intermediate component and minimize rocking movement), and/or an outwardly/downwardly extending tab or flange on the lower tab 9795 interacting and or an inwardly (upwardly) extending tab or flange on the corresponding section of the chassis engaging the lower tab 9795 at location (L3). The engagements of the respective elements in locations L1, L2 and L3 can happen substantially simultaneously or sequentially. No particular order is preferred, as long as an effective final engagement is achieved.

In addition to, or instead of, any bumpers 9775 described previously (which may also be considered as engagement formations or engagement features), one or more of the above described engagements in locations L1 to L3 may form part of the increased resistance during the later stages of insertion. However, these engagement formations may be part of the arrangement that guides the intermediate component into a sealing engagement with the opening of the water reservoir and into its final operational configuration.

As shown in Fig. 36C, the location of the broadened section of guide rail 9760 of the chassis location (L2) may correspond to a location of an edge of the port 9730, an edge of port seal 9735 and/or the ridge or lip of the port seal 9735 in the vertical direction. The engagement between the guide rail 9760 and the opposite respective engagement formation at point L2 lifts the intermediate component in the vertical direction towards the chassis opening 7380 to provide a seal between the port seal 9735 and the surface of the chassis around the chassis opening. In other examples, the location of the engagement formation point L2 may be provided closer to the dock opening and correspond to a central axis of the chassis opening 7380 (not shown in Fig. 36C).

The combination of at the supporting engagement in at least one of the above described engagement formation points, when combined with the supporting engagement provided by the port seal, between the intermediate component 9700 and the reservoir dock 6050, is configured so as to hinder a dislodgement of the intermediate component 9700, and therefore breaking of the port sealing arrangement, in the absence of an appreciable external force. Whilst a single engagement point (say L1 or L2 in Fig. 36C) can be sufficient for a stable engagement, because of the large forces that may be applied to the intermediate component (by a user pulling on the tube) during use, the use of more than one engagement formation points (such as all three points L1, L2 and L3 shown in Fig. 36C) may be advantageous for increased mechanical stability of the arrangement. The inclusion of multiple engagement points (at least some of which in this case causing an elevation of the intermediate component or an elevation of at least a portion of it) may help ensuring a robust and consistent seal at 9730 even where the patient may exert pressure on the arrangement by pulling on the tube, and therefore on the intermediate component 9700, during therapy. Additionally, the robust support of the intermediate component enables easier attachment and removal of attached tubes to the intermediate component.

Because of the specific structure of the sealing configuration, the intermediate component 9700 and the receiving opening, a user inserting the intermediate component 9700 into the chassis opening encounters some resistance only at the very end of the insertion path. This is the location at the end of the insertion path where the intermediate component 9700 encounters an opening of a smaller diameter (effected by elevation engagement points L1 to L3). The minimised friction makes for a more convenient insertion of the intermediate component into the dock outlet 6090 of the reservoir dock 6050. In addition, the described process of guided insertion and the flexible port seal provide for an insertion and sealing with relatively high mechanical tolerance and a reduced likelihood of damage to the membrane 9732 and/or the port seal 9735.

In Figs. 36A - 36C, the engagement (in the illustrated example - elevation) formations at points L1 to L3 are generally part of the reservoir dock opening that receives the intermediate component. However, one or more of these can be formed as part of the intermediate component or of a third element.

In the example illustrated in Figs. 36A - 36C, the intermediate component 9700 at the end of its insertion path is elevated and moved into a sealing engagement with the chassis opening by way of port seal 9735. Examples of the present technology include implementing similar acoustically transmissible engagement without the help of port seal 9735 and/or a membrane 9732. In these examples, the change in the elevation of the intermediate component 9700 may bring port 9730 of the intermediate component 9700 and the chassis opening 7380 in close proximity, or even in an abutment engagement. In some examples of the present technology, the surface of the intermediate component 9700 near the port 9730 and/or the surface of the chassis 7300 near the chassis opening 7380 may be elevated to provide the close proximity or the abutment engagement.

As discussed above, the function of the three engagement points L1 to L3 includes: (a) elevating and moving intermediate component 9700 into a sealing engagement with the chassis opening 7380; and/or (b) reliably locking the intermediate component 9700 into the engaged configuration. Examples of the present technology include a configuration where the chassis opening 7380 is not provided above the intermediate component 9700 (as shown in Figs. 36A to 36C), but in other directions/locations around the intermediate component 9700, such as on the side or below the intermediate component 9700. In these examples, the position of the port 9730 will be provided on the intermediate component 9700 such that it corresponds to the position of the chassis opening 7380.

The engagement formation points L1 to L3 may also be moved such that one or more of them is/are provided on a side of the intermediate component 9700 that is opposite to the position of the port 9730. In these examples, the engagement formation points L1 to L3 will not be "elevation" points, as they will be urging a movement of the intermediate component 9700 in other directions (e.g. to the side). When the chassis opening 7380 is provided on a side, and especially below the intermediate component 9700, the function of the engagement formation points L1 to L3 will still be to push the intermediate component 9700 in a particular direction, as well as to lock the intermediate component 9700 in the engaged configuration. Depending on the location of the chassis opening 7380, the function of the engagement formation points L1 to L3 to push the intermediate component 9700 towards the chassis opening 7380 may be at least partially provided by the force of gravity.

In the example with the port 9730 of the intermediate component 9700 including an associated port seal (i.e. port seal 6735 or port seal 9735), the one or more engagement formation points L1 to L3 may still be tasked with providing a stable sealing engagement between the intermediate component port 9730, the chassis opening 7380, and the port seal. Examples of the present technology include the port seal (e.g., port seal 6735 or port seal 9735) being part of the intermediate component 9700, part of the membrane 9732, part of the chassis opening 7380, and/or an independent element.

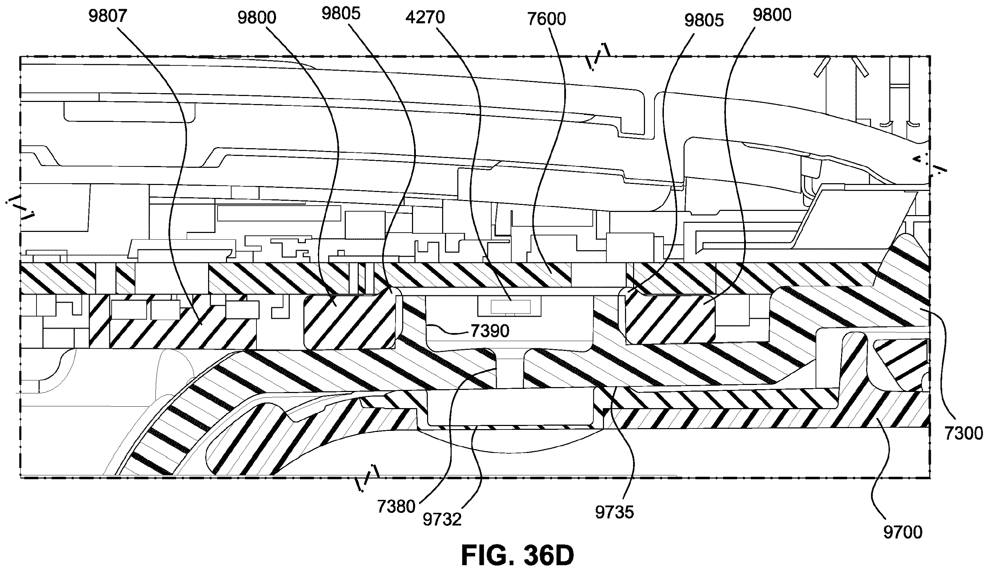

The chassis opening 7380 may have an opening that is smaller than the port 9730 and/or the opening formed by the port seal 9735. In some examples, the chassis opening 7380 may include an inside surface providing a varying cross-sectional profile. Fig. 36D illustrates a profile of the inside surface in which a first end of the chassis opening 7380 adjacent to the port 9730 is provided with an opening that is smaller than a second end of the chassis opening 7380 opposite of the first end. The chassis opening 7380 may have a circular shape having a first diameter formed at the first end and a circular opening at the second end having a second diameter that is greater than the first diameter. In some examples, the second diameter may be equal to a diameter of the port 9730 and/or the opening formed by the port seal 9735. The larger opening may be needed to accommodate the sensor which may be larger than the opening on the first end. The opening on the first end may be smaller to reduce the possibility of allowing contaminants onto the sensor and/or other components on the circuit board.

The specific cross-sectional profile features (in this case the two openings discussed in the previous paragraph) may be formed in the chassis wall. However, the second (or a further) opening may also be formed by providing a flange to the upper surface of the chassis, thus forming a side wall 7390 surrounding the chassis opening 7380 formed on the chassis wall. Fig. 36D illustrates such an optional side wall 7390 extending from a surface of the chassis 7300 towards the PCBA 7600 to provide the second end having the second diameter. The side wall 7390 can improve the acoustic seal between the PCBA 7600 and the first end of the chassis opening 7380 and provides audio integrity for reception by the microphone. The inside wall of the chassis opening 7380 may include different profiles between the first and second end having the different diameters. Fig. 36D shows a profile in which the chassis opening 7380 is flared at its external surface, defining two different diameters at the two opposite surfaces of the chassis wall.

As shown in Fig. 36D, a sensor may be disposed adjacent to and/or at least partially inside of the second end of the chassis opening 7380. In some examples, the sensor 4270 may be spaced, or extend away, from the PCBA 7600 such that the sensor 4270 is almost entirely disposed inside of the chassis opening 7380. A seal made of silicone, a thermoplastic or another flexible material, may be included at the interface between the chassis wall opening and the PCB and/or between the chassis wall opening and the detector.

## 5.9 ACOUSTIC ANALYSIS AND DETECTION

An RPT device (4000 or 6000) in accordance with an aspect of the present technology may be configured to identify physical characteristics of and/or within an air circuit 4170 of a RPT system (including a conduit and/or a mask) based on detection of an acoustic signal propagating along at least a portion of the air circuit 4170 (e.g., the conduit and/or the mask). Examples of acoustic detection for a respiratory treatment apparatus are described in PCT Patent Application Publication No. WO 2010/091462, which is incorporated herewith by reference in its entirety.

RPT device may be configured to identify physical characteristics of and/or within an air circuit 4170 using the RPT device generated sound (e.g., noise from impeller and/or motor of a blower, or other mechanical sounds activated by device assembly such as the insertion of a humidifier reservoir) that travels downstream, for example, along the conduit and to the mask. Similarly, the system could take advantage of environmental noise that makes its way into the airpath of the RPT device. Some of the device generated sound may be reflected by various physical features along the air circuit 4170, including the conduit and the mask, and travel back along the conduit to return to the RPT device to form an "echo" signal. The "echo" signal may be thought of as a filtered reflection of the signal that was incident to a particular feature, and the filter may be thought of as the reflective Frequency Response Function of the particular feature. Someone skilled in the art will understand that the Frequency Response Function is the complex spectrum of the Impulse Response Function, and that either may be used to define the response of a physical feature, such as the reflective response to an incident excitation signal.

A sensor 4270 (e.g., a microphone) (see Fig. 37) may be configured to detect the sound signal. In some examples, the sensor 4270 may be configured to detect the combination of the original source signals and the returned sound ("echo" signal). The sensor 4270 may be located in the inner space of the RPT device 4000. Circuitry (e.g., integrated circuit and/or a processor) coupled to the sensor 4270 may be configured to determine physical characteristics of and/or within an air circuit 4170 based on the detected sound. In some examples, the circuitry may be configured to send the signal to a processor, where signal processing is used to estimate the reflective Impulse Response Function of one or more physical features of interest, and further one or more Impulse Response Functions maybe classified using a classification system. In some examples, the circuitry may be configured to compare the detected returned sound to the originally generated sound, and based on differences in the two, deduct one or more parameters of the downstream system. For example, the circuitry may be configured to determine the type of conduit and/or mask used in the system, presence of obstructions in the air circuit 4170, leaks in the air circuit 4170 etc. In some examples the processing of signals may be distributed, for example an integrated digital sensor can convert the acoustic signal into a digital signal before passing it to a separate processor in which the Impulse Response Functions are estimated. The Impulse Response Function estimates may then be passed to another processor where the classification of physical features takes place. In another example, the RPT device could be connected to a network of other devices, located either locally or remotely, and any combination of processing could be performed on other devices.

In accordance with an aspect of the present technology, the sensor 4270 may be located in the inner space of the RPT device 4000 near a conduit of the air circuit 4170 or features connecting the conduit to the humidifier and/or the pressure generator. A port 6730 (9730) is provided to facilitate the propagation of the incident and/or reflected sound to the sensor 4270. In some examples of the present technology, an intermediate component 9700 for connecting the air circuit 4170 to the RPT device 4000 (e.g. via connection to the reservoir dock 6050 and/or the water reservoir 6100) may include the sound port 9730 facilitating the propagation of the sound (originally generated and/or reflected) to the sensor 4270.

Fig. 37 illustrates example components of a system for detecting sound signals according to the present technology. A generated sound (GS) may be generated by one or more components in the integrated RPT device and humidifier 6000 (or RPT device 4000). The sound may be generated by operation of the blower 4142. The generated sound may propagate directly or via one or more other components to the air circuit 4170. As shown in Fig. 37, the sound is propagated to the air circuit 4170 via the water reservoir 6100 and the intermediate component 9700. Portions of the generated sound GS in the air circuit 4170 may be reflected by various physical features along the air circuit 4170 and or patient interface 3000. The reflected sound may travel back along the conduit to return to the RPT device 6000. Portions of the generated sound may be reflected at different locations within the signal propagation path including the conduit, mask and/or patient.

The sensor 4270 (e.g., a microphone) is provided in the RPT device to sense the generated sound GS and the reflected sound RS. As shown in Fig. 37, the sensor 4270 is disposed in close proximity to the port 9730 of the intermediate component 9700. The sensor 4270 may be coupled to the PCBA 7600 such that the sensor 4270 is positioned a predetermined distance above the port 9730. The sensor 4270 can also be coupled to other circuitry on the PCBA 7600 or another PCBA including processing circuitry configured to deduct one or more parameters of the air circuit 4170 based on the sensed sound. Vibrations in the PCBA 7600 may be picked up by the sensor 4270, which may lead to deterioration of the detected "echo" signal. To reduce the generation of such vibrations, one or more mass/spring/dampening elements, such as rubber and/or silicone washers, can be used in the fastening of the PCBA 7600 to the chassis, which can dampen some of the vibrations of the PCBA 7600 and improve the reception of the "echo" signal.

According to one form of the present technology, the sensor 4270 (e.g., a microphone) is provided a predetermined distance from the port 9730. In one example, the sensor 4270 (e.g., a microphone) is provided as close as possible to the port 9730. The predetermined distance may be measured from the surface defined by the outlet of the chassis opening 7380 to the sensor 4270. In some examples, the predetermined distance may be measured from the sensor 4270 to the inside surface of the intermediate component 9700 where the membrane 9732 may extend. However, these distances are not particularly critical to the design as the resonance effects the incident and reflected waves equally.

The predetermined distance may be determined based on the wavelength of the highest frequency that the system is configured to resolve. In some examples, the sensor 4270 may be provided at a distance that is approximately one fourth of the wavelength of the signals the system will resolve. For example, if the system is configured to sense and process highest frequency of about 10 kHz (wavelength 3.5 cm), the coupling of the sensor 4270 may be provided at a distance of one fourth of the wavelength (e.g., less than around or slightly less than 1 cm) to avoid resonances in the coupling guide between the intermediate component and the microphone. Whilst the above defined distance would usually be of less than 1cm, in some examples, the sensor 4270 may be provided at a distance of up to 2 cm from the opening in the intermediate component 9700.

If the sensor 4270 is located close to a signal propagation path discontinuity, a strong reflection may be created which may mask the reflections created from various features that can otherwise be detected in the conduit and/or the patient interface based on the reflected signal. In some examples of the present technology, the sensor 4270 may be located further away from the closest signal propagation path discontinuity, than the largest distance between any two targeted physical features (say in a detected patient interface or conduit). In this configuration, a clearer time separation is provided between components of the Impulse Response Function (IRF) that are associated with the conduit and/or patient interface and those that are associated with the pressure generator in the RPT device. IRF is the system response to a unit impulse input. In this configuration, the system may provide for detecting features that are less than this distance independently of the properties of the pressure generator IRF.

One approximation of the largest distance between physical features in a patient interface (or a conduit) is the largest dimension of the patient interface or component of the patient interface (or a conduit), when the components are connected via a waveguide. Usually, the ends of the intermediate component 9700 are continuously connected to other components. The outlet of the intermediate component is typically connected to the air circuit, whilst the inlet end of the intermediate component is connected to the inlet end of the outlet tube of the water reservoir. Because of these connections, the signal propagation path discontinuity may shift - in the case of the inlet end of the intermediate component, the discontinuity now shifts to the inlet end of the outlet tube of the water reservoir 6100. For detection of small masks, the distance between the sensor 4270 from inlet end of the outlet tube of the water reservoir 6100 should generally be at least 2-6 cm. For the detection of larger masks, the distance between the sensor 4270 from the inlet end of the outlet tube of the water reservoir 6100 should generally be at least 4-15 cm. Similar considerations apply to the dimensions of the outlet tube of an outlet cap muffler 4124 (e.g. the length of the outlet tube, which determines the distance between the sensor 4270 and the inlet end of the outlet tube), when such a muffler is used instead of the water reservoir. In alternative embodiments of the current technology, the IRF of the RPT device may be characterised and filtered out of the recorded microphone signals to reduce the effects of the above mention dimensions.

According to one form of the present technology, to fully separate the IRF of the mask from the IRF of the pressure generator, the distance between the sensor 4270 from the inlet end of the outlet tube of the water reservoir 6100 should be larger than the largest dimension of the mask. For instance, for a mask with a 40 cm connecting tube and a cuff, which are used to attach the mask and the connecting tube to a standard length tube, the dimension should be larger than 40 cm plus the length of the other mask components. However, alternative arrangements are also possible. When the distance between the sensor 4270 and the closest discontinuity is not larger than the largest dimension of the mask, the mask signature may appear superimposed on a reflected and attenuated version of itself (i.e. the secondary reflection of the mask signature off the device discontinuity). This composite signature may be just as useful for mask identification. For instance, in the case where the distance between the sensor and the closest discontinuity is significantly smaller, the superposition of the IRFs can be accounted for algorithmically, for the case that the IRF of the pressure generator is consistent or deterministic. Examples of acoustic feature detection based IRF in the system are described in PCT Patent Application Publication No. WO 2010/091462, which is incorporated herewith by reference in its entirety.

In some examples of the present technology, a seal 9800 (e.g., constructed of thermoplastic elastomer (TPE) or silicone) may be disposed between the PCBA 7600 and the chassis 7300 (e.g., see Fig. 36D). The main portion of seal 9800 may form a sealing wall adjacent to, and at least partially surrounding, the side wall 7390. The wall portion of seal 9800 may partially or completely surround the side wall 7390 that extends from the chassis and provides an opening facing the PCBA 7600. The sensor 4270 may be aligned with, and/or be at least partially provided inside, the opening of the chassis facing the PCBA 7600.

In one example, the seal may further include a "ceiling" portion facing the PCBA 7600 and extending above and into the area surrounded by the sealing wall and adjacent side wall 7390, thus effectively forming a ceiling above both the sealing wall and the side wall 7390. An opening may be provided in this ceiling portion to allow sound to pass through on its way from the chassis opening 7380 to the sensor. Alternatively, as shown in Fig. 36D, a surface of the ceiling portion of seal 9800 facing the PCBA 7600, may include an opening corresponding to the opening formed by the side wall 7390. The opening in the seal 9800 allows for the sensor 4270 mounted on the PCBA 7600 to be disposed at least partially into the chassis opening formed by the side wall 7390. The seal 9800 may include a peripheral sealing formation 9805 around the perimeter of the opening in the seal 9800. As shown in Fig. 36D, the peripheral sealing formation 9805 may be provided at the outside edge of the side wall 7390. The peripheral sealing formation 9805 may provide a compression-type seal against the PCBA 7600 when the PCBA 7600 and the chassis 7300 are assembled. A portion of the chassis 7300 adjacent to the side wall 7390 may press the seal 9800 against the PCBA 7600 when assembled.

The seal 9800 and/or the peripheral sealing formation 9805 may prevent the entrance of external noise into the chassis opening and/or reduce transfer of vibrations between the PCBA 7600 and the chassis 7300. The seal 9800 and/or port seal 9735 provides for an acoustic seal to guide sound to the sensor. The peripheral sealing formation 9805 may include a ridge sealing formation described with reference to Figs. 34A-34D or a lip sealing formation described with reference to Figs. 35A-35D.

The seal 9800 may be attached to the PCBA 7600 and/or to the chassis 7300 using an adhesive or by mechanical means (e.g., screws or bolts). In some examples, the seal 9800 may be secured by being pushed against the PCBA 7600 by the chassis 7300 without the use of an adhesive or by mechanical means.

### 5.9.1 Coupling Between a Sensor and an Intermediate Component

As discussed above with reference to Fig. 37, a sensor 4270 (e.g., a microphone) is mounted on the PCBA 7600 to sense sound in the air path. The sound reaches the sensor 4270 via a port 9730 in the intermediate component 9700 or another portion of the air circuit 4170 (e.g., a conduit). The port 9730 may include a membrane and/or a port seal. The sound may be conveyed via the port seal from the membrane to an opening 7380 in the chassis 7300 behind which, and/or in which, sits the sensor 4270, mounted on the PCBA 7600. The sound from the sensor 4270 is analysed to determine one or more characteristics of the RPT device and/or one or more characteristics of the air circuit 4170.

Various problems can affect sound quality conveyed to and sensed by the sensor 4270 and/or analysis consistency between RPT devices and over time. For example, variations in relative position of the sensor 4270 and features of the air circuit 4170 (e.g., the intermediate component 9700, the port 9730, the port seal 9735 and/or the membrane 9732 discussed above with reference to Figs. 34A-36C) as a result of manufacturing tolerances and/or wear of components; escape of sound to ambient once it passes through the port 9730 and/or chassis opening 7380; and/or vibration of components of the RPT device (e.g., PCBA 7600) and/or the air circuit 4170 during use that is transmitted to the sensor 4270.

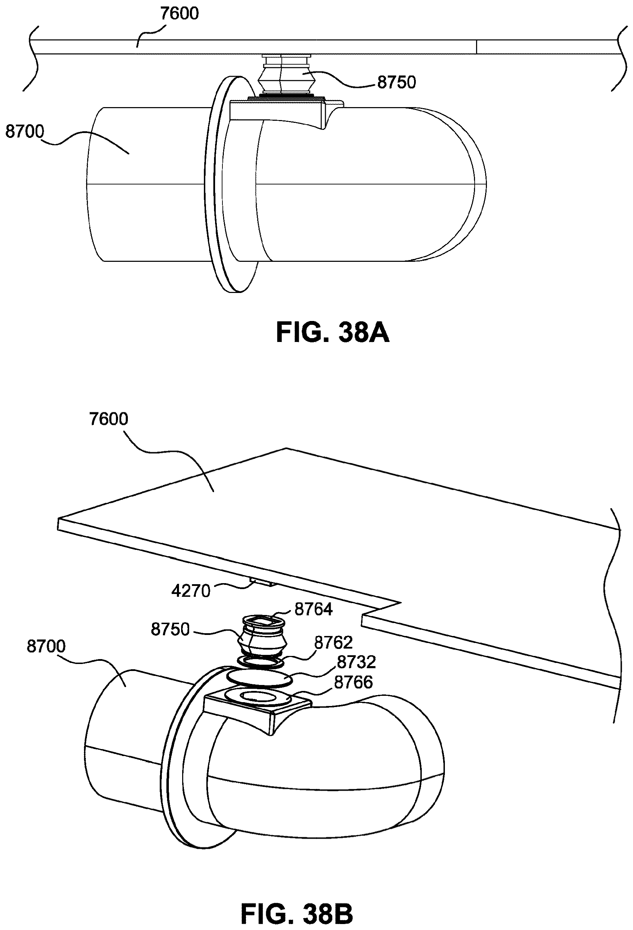

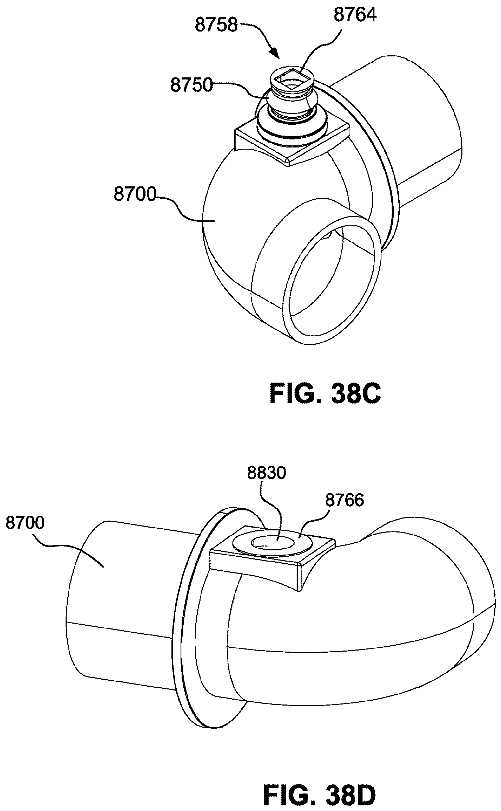

Examples of the present technology (see Figs. 38A-39D) provide a coupler 8750 configured to flexibly couple a sensor 4270 to features of the air circuit 4170 (e.g., a port and/or a membrane provided in an intermediate component or another portion of the air circuit) to improve sound quality and consistency. The coupler 8750 is configured to self-align to ensure consistency of relative positioning (e.g., horizontal and/or vertical) of the sensor 4270 to a port 8830 and/or a membrane 8732. The coupler 8750 may be a magnetic coupler including one or more magnets to align and hold the coupler 8750 in place. The coupler 8750 also acts as a complete sealed path to minimise escape of sound to ambient. In this sense the coupler, at least to some extent, guides the sound along its path to the sensor. The flexible nature of the coupler 8750 damps at least some of the vibration of the air circuit 4170 components (e.g., an intermediate component 8700 and/or PCBA 7600). To reduce transmission of vibration from the PCBA 7600 to the sensor 4270, the sensor 4270 may be mounted using one or more damping features (e.g., rubber feet) on a tab on the PCBA 7600, which may be partially surrounded by a cut-out channel. The coupler 8750 can be "bellows" type to give added vertical flexibility.

While the features related to coupling the sensor 4270 to the air circuit 4170 are described with reference to coupling the sensor 4270 to an intermediate component, the features related to coupling the sensor 4270 to the air circuit 4170 may be included in an implementation where the sensor 4270 is coupled, directly or indirectly, to another portion of the air circuit 4170 (e.g., a conduit or a mask). For example, the features related to coupling the sensor 4270 to the air circuit 4170 may be applied to examples in which the sensor 4270 is coupled to a port (e.g., a membrane-covered opening) in a conduit of the air circuit 4170. In this example, the sensor 4270 may be mounted independently, or be mounted on a PCBA 7600 inside of the RPT device housing, or on another PCBA 7600 outside of the RPT device housing.

In the contect of the above paragraph, it may be considered that the term "intermediate component" (e.g., intermediate component 6700, intermediate component 8700, or intermediate component 9700), actually covers any component that links a sound source linked to the air circuit and a detector. In this sense, it may be considered that "intermediate" is used in the context of being between the sound source and the sensor, in order to provide an acoustic link between them and is not necessarily limited to a component located between a conduit and a blower. Thus, in some examples of the present technology, the intermediate component may not be in a path between a blower and a conduit of the air circuit 4170 (e.g., see Fig. 37), but may be pneumatically connected to the path. In one such example, the intermediate component may connect to an opening in the tube of the air circuit and connect to a sensor via a port and a seal provided in the intermediate component.

In other examples, the intermediate component may be disposed inside of a housing of the RPT device with other components (e.g., pressure generator, humidifier, circuit board, and/or sensor).

### 5.9.1.1 Sensor Positioning and Sound Path Alignment

As discussed above, in some examples of the present technology, the sensor 4270 (e.g., a microphone) is provided a predetermined distance from a port 8830 in an intermediate component 8700. In some examples, the sensor 4270 is provided at a specific location and as close as possible to the port (e.g., port 8830). However, due to variations in manufacturing tolerances, wear of components, and variations in positioning of the air circuit 4170 features (e.g., position of the intermediate component 8700 when inserted into the receiving chassis opening), the relative positioning (e.g., horizontal and/or vertical) of the sensor 4270 to a port 8830 and/or a membrane 8732 may be different for the same components used in different RPT devices or may change over time or when components are replaced or reassembled for use (e.g., after cleaning).

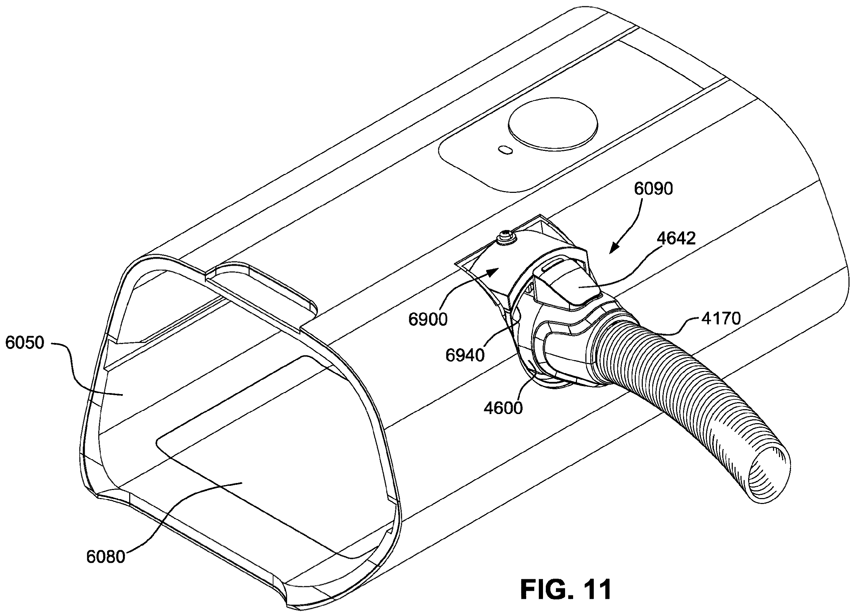

Figs. 38A-39D illustrate a coupler 8750 coupling a sensor 4270 to an intermediate component 8700 according to an example of the present technology. The coupler 8750 is configured to align the sensor 4270 to a port 8830 in the intermediate component 8700, when the intermediate component 8700 is inserted into the RPT device (e.g., a dock outlet 6090 of the reservoir dock 6050 shown in Figs. 11-13).

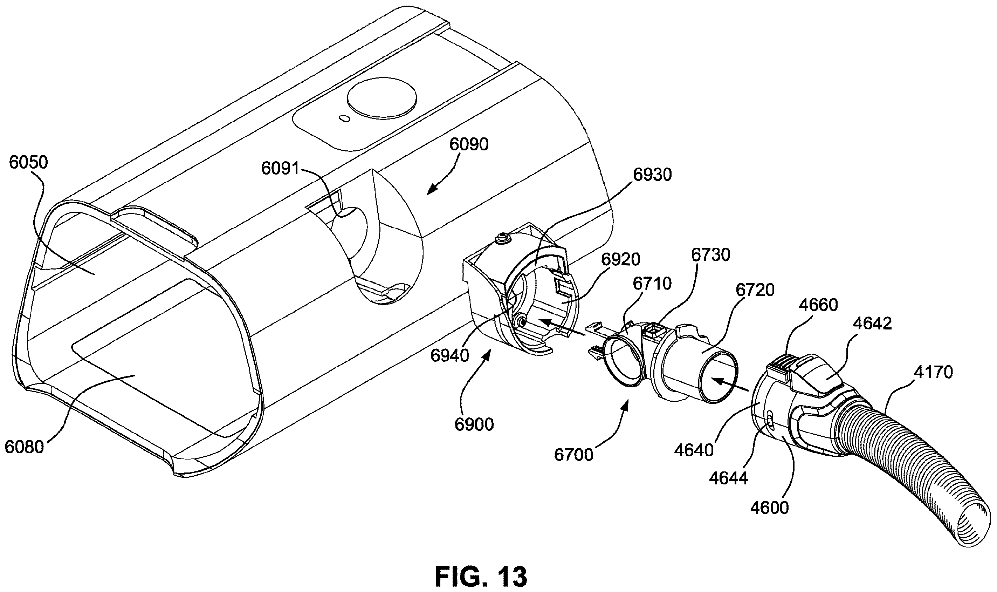

In some examples, the intermediate component 8700 may include one or more features described with reference to the intermediate component 6700 (see Figs. 13-15 and 19-22) and/or intermediate component 9700 (see Figs. 23-36C), or correspond to the intermediate component 6700 or the intermediate component 9700. Similarly, the intermediate component 6700 or the intermediate component 9700 may include one or more features described with reference to the intermediate component 8700.

The coupler 8750 is positioned between and coupled to the PCBA 7600 and the intermediate component 8700. The coupler 8750 is removably coupled to the intermediate component 8700. While Figs. 38A-39D illustrate the coupler 8750 coupled to the intermediate component 8700, in some examples of the present technology, the coupler 8750 may be removably coupled to another component of the air circuit 4170 (e.g., a conduit) including an opening to allow sound to reach the sensor 4270 positioned outside of the air circuit 4170.

The coupler 8750 comprises a body 8752, which may be a tubular body, including an inlet end 8756 adapted to interface with the intermediate component 8700 and an outlet end 8758 adapted to interface with the sensor 4270 and/or the PCBA 7600. The coupler 8750 may be constructed of relatively soft material (e.g., thermoplastic elastomer (TPE) or silicone). The body 8752 may include one or more bellows 8754 disposed between the inlet end 8756 and the outlet end 8758. The one or more bellows 8754 are adapted to allow the inlet end 8756 to be displaced in the horizontal and/or vertical directions relative to the outlet end 8758, which may be fixed to the sensor 4270 and/or the PCBA 7600, without disturbing the coupling.

In one form of the present technology, flexibility of the material of the coupler 8750 may allow the inlet end 8756 to be displaced in the horizontal and/or vertical directions relative to the outlet end 8758 without disturbing the coupling. In this example, the horizontal and/or vertical direction displacement due to the flexibility of the material may be provided in addition to the one or more bellows 8754 or instead of the one or more bellows 8754.

The outlet end 8758 includes an opening 8764 configured to engage the sensor 4270 provided on the PCBA 7600. The opening 8764 in the outlet end 8758 may correspond to the shape and size of the sensor 4270 (e.g., a sensor housing 4271). The sensor 4270 may be press fit into the opening 8764 in the outlet end 8758 and/or glued or cold welded to secure the sensor 4270 to the coupler 8750. The sensor 4270 may be removably coupled in the opening 8764.

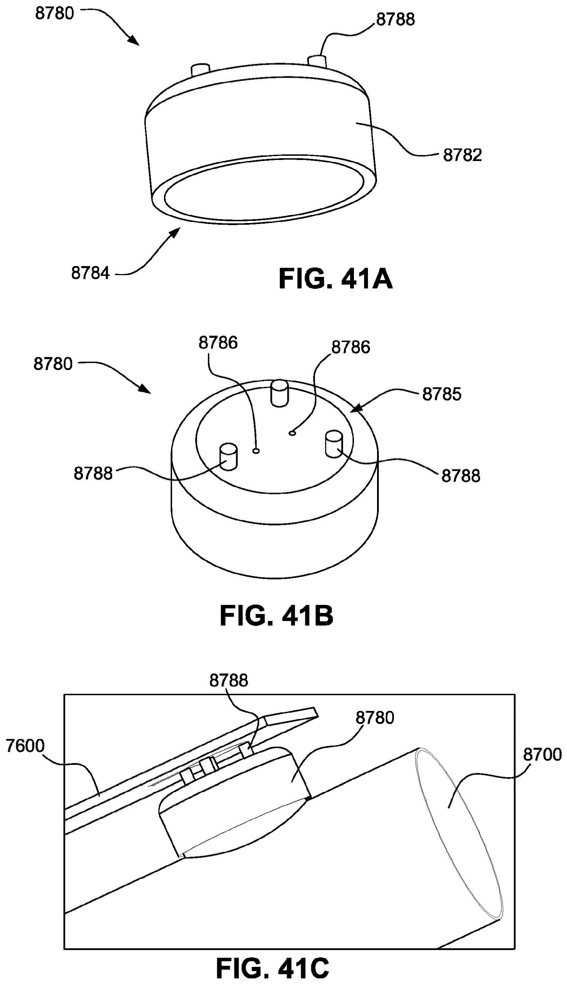

The outlet end 8758 may include a flange 8759 adapted to abut against a portion of the sensor 4270 and/or a portion of the PCBA 7600 surrounding the sensor. The flange 8759 may extend from the body 8752 and in an outward direction from a central axis of the body 8752 and/or from the body 8752 and towards the central axis of the body 8752. In one example, the outlet end 8758 may be coupled to the sensor 4270 without directly touching the PCBA 7600. In another example, at least a portion of the outlet end of the coupler 8750 may be in contact with the PCBA 7600. In another example, the outlet end 8758 may contact (and/or attach to) the PCBA 7600, but the body of the sensor 4270 is offset at a predetermined distance from the PCBA 7600 such that the sensor is not directly located on the PCBA 7600, but is still attached to it. In this example, the outlet end may be closed, one or more rubber feet (see rubber feet 8788 in Figs. 41A-C) may be provided on the outlet end 8758 for connecting the coupler 8750 to the PCBA 7600 and one or more ports 8786 may be made available for passing the electrical wires connecting the sensor to the PCBA 7600.

The inlet end 8756 includes a coupling portion 8760 adapted to receive a first connecting element 8762. The coupling portion 8760 may include a flange comprising a complementary shape corresponding to a shape of the first connecting element 8762. In some examples, the first connecting element 8762 may be a ring adapted to be press fit against the flange and on a portion of the body 8752 extending past the flange. The first connecting element 8762 may be glued or cold welded to the inlet end 8756. In some examples, the inlet end 8756 may be overmoulded to the first connecting element 8762 such that the inlet end 8756 encloses the first connecting element 8762.

The first connecting element 8762 is adapted to removably connect to a second connecting element 8766 disposed on or in the intermediate component 8700. The first connecting element 8762 and/or the second connecting element 8766 may include magnets adapted to connect to each other. In some examples, one of the first connecting element 8762 and the second connecting element 8766 comprises a magnet and the other one of the first connecting element 8762 and the second connecting element 8766 comprises a metal material adapted to connect to the magnet. The shape of the first connecting element 8762 may correspond to the shape of the second connecting element 8766. In one example, the shape and size of the first connecting element 8762 may be the same as the shape and size of the second connecting element 8766. In one example, the shape of the first connecting element 8762 may be the same as the shape of the second connecting element 8766 and the size of the second connecting element 8766 may be larger than the first connecting element 8762.

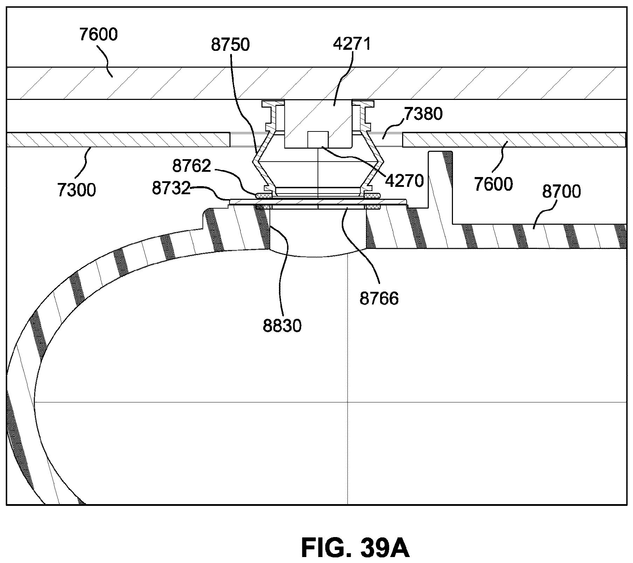

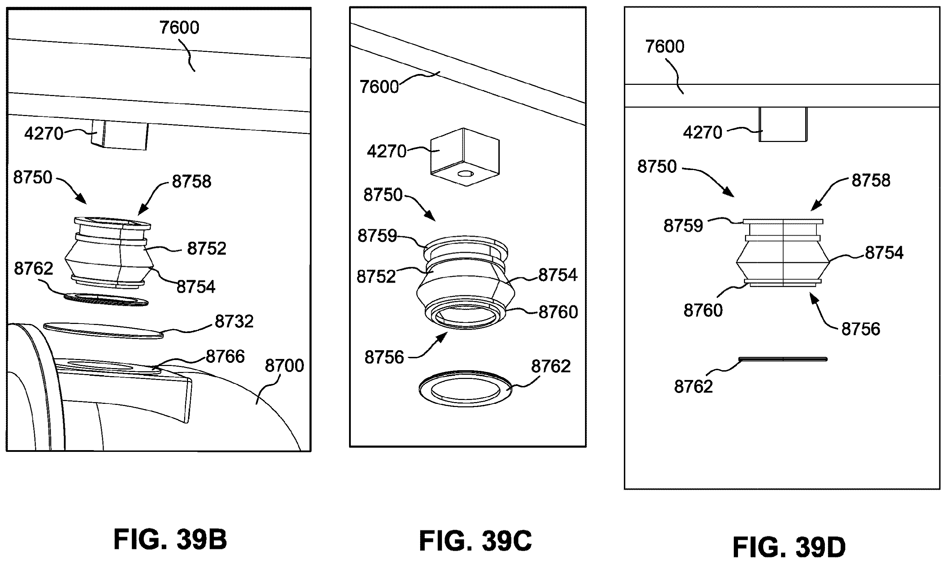

The second connecting element 8766 is provided on a surface of the intermediate component 8700 (see e.g. Fig. 39B) or disposed below at least a portion of the surface of the intermediate component 8700 (see e.g. Fig. 39A). The second connecting element 8766 is attached (e.g., glued or cold welded) to the intermediate component 8700. The second connecting element 8766 may be a ring positioned around a port 8830 in the surface of the intermediate component 8700. The second connecting element 8766 may be positioned such that a central axis of the second connecting element 8766 is common with the central axis of the port 8830. In some examples, the opening of the port 8830 may correspond in shape and/or size to an opening in the second connecting element 8766 and/or the first connecting element 8762.

In some examples, the second connecting element 8766 may be provided under a membrane 8732 or another element (e.g., a silicone ring shaped element) adapted to cover at least a portion of the second connecting element 8766 (e.g., along the perimeter of the ring). The membrane 8732 or the other element may couple to the surface of the intermediate component 8700 adjacent to an outside perimeter of the second connecting element 8766. The membrane may be a thin silicone membrane that is sound permeable but impermeable to liquids and/or gases. The membrane 8732 may have a shape and/or size that corresponds to the outside shape and/or size of the first connecting element 8762 and/or the second connecting element 8766. As shown in Figs. 38B, 39A and 39B, the membrane 8732 may be circular and extend beyond the second connecting element 8766 in some examples of the present technology.

In some examples of the present technology, the membrane 8732 may include one or more features of the membrane 9732 discussed above (see e.g., Figs. 34A-36C). In some examples of the present technology, the membrane 8732 may be provided on an inside surface of the intermediate component 8700 (see e.g., the membrane 9732 illustrated in Figs. 34A-36C).

In some examples of the present technology, the membrane 8732 may be configured to cover an end of the coupler 8750 adjacent to the intermediate component 8700. For example, the membrane may be provided between an end of the coupler 8750 and the first connecting element 8762.

In some examples of the present technology, the second connecting element 8766 may be provided as part of or inside of a port seal (see port seal 9735 in Figs. 34A-36C). In this example, the second connecting element 8766 may be included in or under a portion of the port seal 9735. For example the second connecting element 8766 may be included in the ridge seal 9737, in the lip seal 9742, in connecting portions 9739, or under the connecting portions 9739.

In some examples of the present technology, other mechanical couplings such as mechanical locking method, temporary sticky method or suction methods may be used to removably couple the coupler 8750 to the intermediate component 8700.

As illustrated in Fig. 39A, the coupler 8750 may extend from the PCBA 7600 and towards the port 8830 through the chassis opening 7380 in the chassis 7300. In some examples, at least a portion of the sensor 4270 may extend to or through the chassis opening 7380. Fig. 36D shows the PCBA 7600 disposed above the chassis opening 7380 and the sensor 4270 supported by the PCBA 7600 extending at least partially into the chassis opening 7380. In some examples, a centre of the sensor may be aligned with a central axis of the chassis opening 7380. In an alternative arrangement, the intermediate component may not be directly connecting the pressure generator with the air delivery tube, but may still be pneumatically connected to both of them. Also, in an alternative arrangement, there may not be a chassis wall located between the opening of the intermediate component and the sensor. In this case, all the above description is still applicable, but in the context of a direct connection between the opening of the intermediate component and the PCBA 7600 (e.g. see Fig. 37) or another component on which the acoustic sensor might be located.

When the intermediate component 8700 is inserted into the receiving reservoir dock 6050 and positioned in place (e.g., see Figs. 36A-36C), the inlet end 8756 of the coupler 8750 may not align with the port 8830 in the horizontal and/or vertical direction. The attraction and connecting properties between the first connecting element 8762 and the second connecting element 8766 and the one or more bellows 8754 allow the inlet end 8756 to be displaced in the horizontal and/or vertical directions, allowing for the first connecting element 8762 to align and connect to the second connecting element 8766 when they are brought in close proximity to each other. The alignment (e.g., by having a common axis) of the first connecting element 8762 and the second connecting element 8766, as well as any additional elements that may be included in the connecting arrangement may provide a path for the sound to travel directly from the intermediate component 8700 to the sensor 4270, without necessarily any of the elements being in a mechanical contact with the chassis 7300 or the PCBA 7600. When aligned, the port 8830, the first connecting element 8762 and the second connecting element 8766 may have a common vertical axis.

Alignment of the inlet end 8756 with the port 8830 provides an aligned path for the sound to travel from the intermediate component 8700 to the sensor 4270 via the port 8830. This path is consistently provided even with variations in the final position of the intermediate component 8700 relative to the fixed position of the sensor 4270.

### 5.9.1.2 Features Mitigating Sound Escape

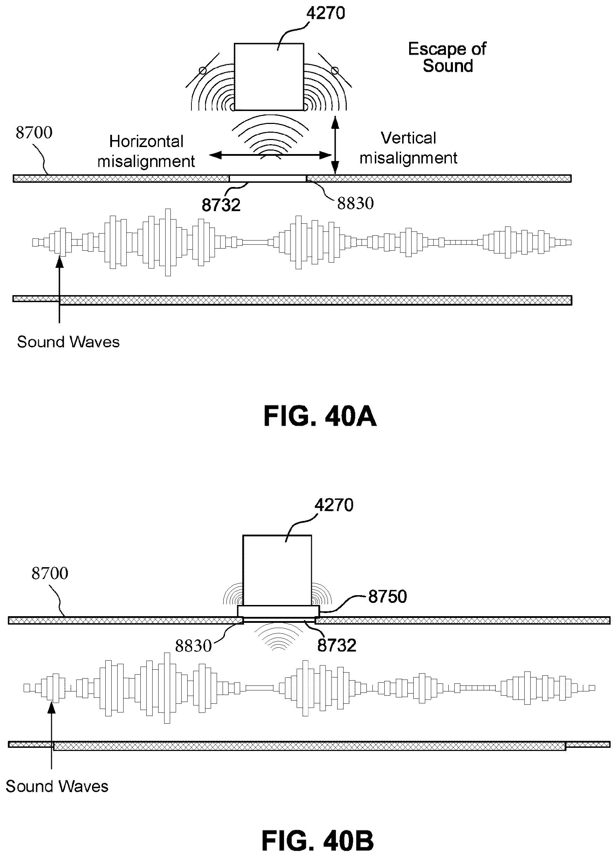

The coupler 8750 also acts as a complete sealed path to minimise escape of sound to ambient. Fig. 40A illustrates the escape of sound that may occur without including a coupler 8750 between the sensor 4270 and the intermediate component 8700. As shown in Fig. 40A, after the sound passes the membrane 8732, the sound is directed towards the sensor 4270. However, horizontal misalignment between the sensor 4270 and the membrane 8732 and/or the port 8830, may cause a portion of the sound to be directed to the sides of the sensor 4270 causing reduction in sound captured by the sensor 4270, affecting the quality of captured sound. Changes in the vertical alignment may increase or decrease the amount of sound that is directed to the sides of the sensor 4270.

Fig. 40B illustrates the escape of sound that may occur in a system including a coupler 8750 between the sensor 4270 and the intermediate component 8700, according to an example of the present technology. The self-alignment of the coupler 8750 to the port 8830 in the intermediate component 8700 provides a sealed path for sound to travel from the intermediate component 8700 to the sensor 4270. As shown in Fig. 40B, without the vertical and /or horizontal misalignment, very little or no sound may escape to the sides of the sensor 4270. Accordingly, the coupler 8750 acts as a complete sealed path to minimise escape of sound to ambient.

### 5.9.1.3 Features Mitigating Vibrations

Vibrations in the components of the RPT device and/or the air circuit 4170 may degrade the quality of the sound captured by the sensor 4270. The flexible nature of the coupler 8750, provided by the material of the coupler 8750 and/or the bellows 8754, damps at least some of the vibration generated in and/or passed via the air circuit 4170 to the sensor 4270.

Vibrations that may cause deterioration of the detected "echo" signal may also be passed to the sensor 4270 from the PCBA 7600. The PCBA 7600 is connected to the chassis which picks up vibrations caused by components of the RPT device (e.g., motor of a blower). As discussed above, to reduce the generation of such vibrations in the PCBA 7600, one or more dampening elements, such as rubber and/or silicone washers, can be used in the fastening of the PCBA 7600 to the chassis, which can dampen any vibrations of the PCBA 7600 and improve the reception of the "echo" signal.

### 5.9.1.3.1 Rubber Interface

Another feature that may be included with the other examples disclosed in this specification, is providing one or more damping elements in the fastening of the sensor 4270 to the PCBA 7600 to dampen vibrations from the PCBA 7600 passed to the sensor 4270. Figs. 41A-41C show a rubber interface 8780 for coupling the sensor 4270 to the PCBA 7600. The rubber interface 8780 is provided between the sensor 4270 and the PCBA 7600. The rubber interface 8780 may act as a vibration damper. The rubber interface 8780 provides a flexible housing for supporting the sensor 4270 and for isolating vibrations from the PCBA 7600. The rubber interface 8780 may also reduce vibrations passed to the sensor 4270 from the intermediate component 8700.

The rubber interface 8780 may include a tubular body 8782 with one end including an interface opening 8784 and an opposite end 8785 being closed. While Figs. 41A-41C show the rubber interface 8780 including a tubular interior and exterior shape, examples of the present technology are not so limited and may include other shapes (e.g., a square or rectangular interior or exterior shape).

The interface opening 8784 may be configured to receive and hold the sensor 4270 (not shown in Figs. 41A-41C) at least partially inside of the rubber interface 8780. In some examples, a portion of the sensor 4270 may extend out of the tubular body 8782. In some examples, the sensor 4270 may be provided inside of the rubber interface 8780 without extending outside of the tubular body 8782.

One or more connecting ports 8786 may be provided on a closed end 8785 of the rubber interface 8780 adjacent to the PCBA 7600. One or more connections (not shown in Figs. 41A-41C) between circuit elements on the PCBA 7600 and electrical connections on the sensor 4270 may be routed via the one or more connecting ports 8786.

The rubber interface 8780 may include a plurality of rubber feet 8788 on the closed end 8785 configured to offset the rubber interface 8780 closed end from the PCBA 7600 and couple the rubber interface 8780 to the PCBA 7600. The rubber feet 8788 may reduce the contact area between the PCBA 7600 and the rubber interface 8780, reducing the amount of vibration transmitted from the PCBA 7600 to the sensor 4270.

In one example of the present technology, the rubber interface 8780 may be configured to engage the outlet end 8758 of the coupler 8750 shown in Figs. 38A-39D. In this example, the rubber interface 8780 may correspond to the sensor housing 4271 shown in Fig. 39A.

In another example of the present technology, the rubber interface 8780 may correspond to the coupler 8750 configured to couple the sensor 4270 to the intermediate component 8700 (see Fig. 41C). In this example, the rubber interface 8780 may include a first connecting element 8762 (not shown in Fig. 41C) inside of the rubber interface 8780 or attached to the interface opening 8784. The first connecting element 8762 configured to couple to a second connecting element 8766 (not shown in Fig. 41C) provided in or on a surface of the intermediate component 8700. In this example, the rubber interface 8780 provides coupling to the intermediate component 8700 while at the same time providing vibration damping for vibrations from the intermediate component 8700 and from the PCBA 7600.

### 5.9.1.3.2 Channel cut-out in the Circuit Board

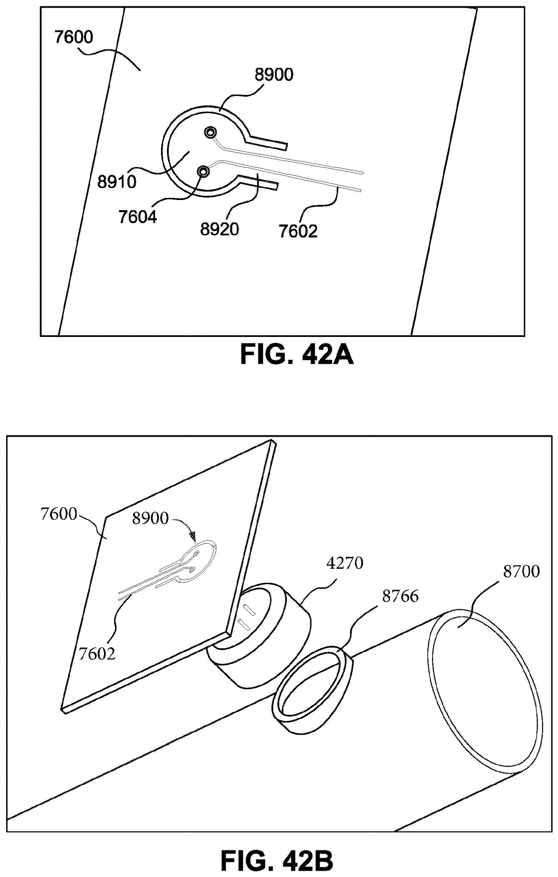

Another feature for mitigating vibrations passed to the sensor 4270, that may be included with the other examples disclosed in this specification, includes providing one or more channels 8900 cut out in the PCBA 7600 to reduce / dampen vibrations from the PCBA 7600 in the sensor 4270. The channel 8900 is cut though the thickness of the PCBA 7600. Figs. 42A-42C show an example of a channel 8900 cut out in the PCBA 7600. The channel 8900 cut-out width may correspond to the thickness of the PCBA 7600.

The PCBA 7600 includes conductive tracks 7602 configured to connect to the sensor 4270 and transmit signals to other components provided on and/or off the PCBA 7600. The PCBA 7600 includes through holes 7604 configured to couple leads of the sensor 4270 to the conductive tracks 7602. The channel 8900 is provided at least partially around the through holes 7604 provided on a suspended tab 8910 of the PCBA 7600 formed by the channel 8900. The conductive tracks 7602 extend from the through holes 7604 to other components via a connected portion 8920 of the tab 8910.

The channel 8900 reduces the amount of vibration transmitted to the sensor 4270. While vibrations to the sensor 4270 may still be transmitted via the connected portion 8920, the amount of vibration transmitted to the suspended tab 8910 is greatly reduced.

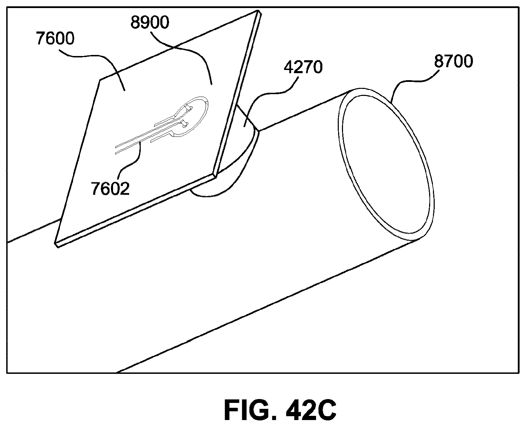

In some examples, in addition to reducing the vibrations, the tab 8910 may deflect in a vertical direction to align the sensor 4270 coupled to the tab 8910 due to attraction caused by the coupling of the first connecting element 8762 and the second connecting element 8766. For example, the attraction forces between the first connecting element 8762 and the second connecting element 8766 may cause the tab 8910 to deflect downwards to position the sensor 4270 closer to the port 8830 than would be the case without the channel 8900. The sensor 4270 shown in Fig. 42B and 42C may include the first connecting element 8762 or may be provided with the coupler 8750 for connecting to the second connecting element 8766.

In some examples of the present technology, a cut-out channel providing a tab may be provided at each of the locations that the PCBA 7600 is connected to the chassis. In this example, the tabs provided by the cut-out channels may reduce the vibrations passed to the PCBA 7600 from the chassis.

### 5.9.1.4 Flexible Sensor Housing

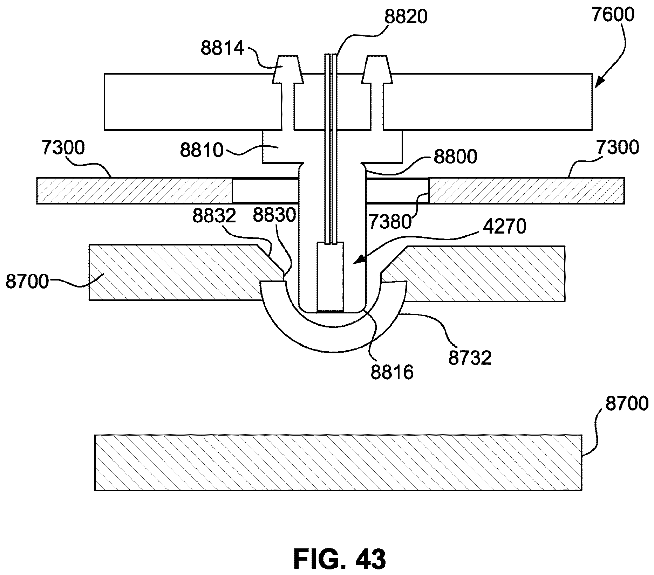

Another feature that may improve sensor positioning and mitigate sound escape and/or vibration is providing the sensor 4270 in a flexible housing 8800. Fig. 43 shows a sensor 4270 disposed in a flexible housing 8800 according to an example of the present technology. One or more of the features illustrated in Fig. 43 may be provided in the other examples of the present technology disclosed in this specification.

As illustrated in Fig. 43, the sensor 4270 is disposed in a flexible housing 8800 including the sensor 4270 at one end of the flexible housing 8800. One end of the housing 8800 includes a connecting portion 8810 configured to couple the flexible housing 8800 to the PCBA 7600. The connecting portion 8810 may include a plurality of posts 8814 adapted to engage through holes in the PCBA 7600 and secure the flexible housing 8800 against the surface of the PCBA 7600. Flexible wires 8820 are coupled to the sensor 4270 in the flexible housing 8800 and run inside of and along the length of the flexible housing 8800 to an end of the flexible housing 8800 including the connecting portion 8810. The flexible wires 8820 provide a connection between the sensor 4270 and components on and/or off the PCBA 7600.

The flexible housing 8800, which may comprise moulded silicone or rubber, may be overmoulded on the sensor 4270 and/or the flexible wires 8820. The flexible housing 8800 is adapted to flex when forces are applied, for example to the end of the flexible housing 8800 including the sensor 4270.

In use, when the intermediate component 8700 (or another air circuit component including a port 8830) is inserted, the end of the flexible housing 8800 including the sensor 4270 may be pushed by the inserted intermediate component 8700, causing the flexible housing 8800 to deflect from an equilibrium position. The deflection of the flexible housing 8800 allows the intermediate component 8700 to engage the chassis in a fully assembled position (see e.g., Figs. 36A-36C) without damaging the sensor 4270. In the fully assembled position, the flexible housing 8800 and the sensor 4270 are aligned with the port 8830 allowing for the flexible housing 8800 to deflect back into the equilibrium position. In this position, the sensor 4270 can be positioned inside of the port 8830 and/or the intermediate component 8700. When the intermediate component 8700 is removed, the flexible housing 8800 may be pushed by the side of the port 8830 and deflect from the equilibrium position allowing for the intermediate component 8700 to be removed without damaging the sensor 4270.

As shown in Fig. 43, in the fully assembled position the end of the flexible housing 8800 including the sensor 4270 is positioned at least partially inside of the port 8830. In some examples, a portion of the sensor 4270 may be provided below the outside surface of the intermediate component 8700 and below the inside surface of the intermediate component 8700 (see Fig. 43).

The flexible housing 8800 allows for the sensor 4270 to be positioned at least partially inside of the port 8830, inside of the intermediate component 8700 and/or in close proximity of the membrane 8732 to improve the sound captured by the sensor 4270.

The port 8830 and/or the flexible housing 8800 may include one or more features configured to reduce wear on the port 8830 and/or the flexible housing 8800 and/or to provide gradual deflection of the flexible housing 8800. For example, as shown in Fig. 43, the flexible housing 8800 may include rounded edges 8816 and/or an edge 8832 of the port 8830 may be chamfered to reduce wear and/or provide gradual deflection of the flexible housing 8800. In some examples, the edge 8816 may be chamfered and/or the edge 8832 may be rounded.

While not illustrated in Fig. 43, a port seal (i.e. port seal 9735) including a ridge seal 9737 or a lip seal 9742 may be provided between the intermediate component 8700 and the surface of the chassis 7300.

### 5.9.2 Example methodology for acoustic detection and analysis

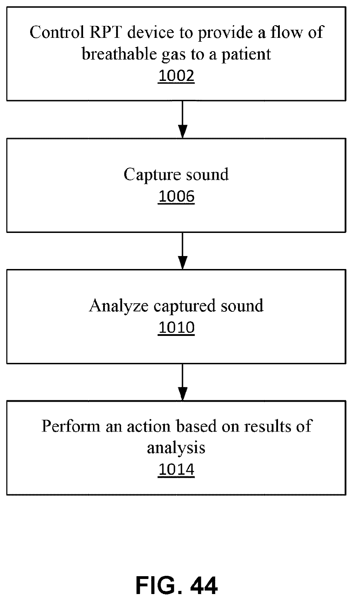

Fig. 44 illustrates an example methodology for acoustic detection and analysis according to the present technology. One or more of the operations may be performed by hardware circuitry (e.g., a processor) and/or software. In step 1002, the RPT device is controlled to provide a flow of breathable gas to the patient via the air circuit 4170. Controlling the RPT device may include controlling operation of at least one of the blower, the humidifier and the tube. Operation of the RPT device may generate a sound (e.g., from the blower) that is propagated in the air circuit 4170. The sound in the air circuit 4170 in this case is generated by elements that move/vibrate during the operation of the RPT device. A dedicated speaker provided as a part of the RPT device for generating the sound, can also be used. Similarly, a feature such as a sharp edge in the flow path within the RPT or other component could be used to generate turbulent flow sounds.

In step 1006, sound detected by the sensor 4270 is captured. The captured sound may represent sound in the air circuit 4170 generated by the operation of the RPT device and sound reflected by one or more features in the air circuit 4170 towards the RPT device. The sound may be captured continuously or at predetermined intervals during operation of the RPT device. In some examples, the sound may be captured during a diagnostics stage initiated by a patient, during start-up of the RPT device, after the RPT device has been used for a predetermined amount of time, and/or connection of a new conduit and/or patient interface. The signal provided by the sensor 4270 may be an analog signal or a digital signal. The circuitry may include analog-to-digital (A/D) converters /samplers configured to convert the analog signal into a digital signal. The circuitry may include one or more low pass filters, one or more high pass filter and/or one or more band pass filters configured to process the sound signal before it is analysed.

In step 1010, the sensed sound is analysed to deduct one or more characteristics of the RPT device and/or one or more characteristics of the air circuit 4170 based on the processed sound signal. The one or more characteristics of the RPT device may include proper or improper operation of a component in the RPT device. For example, based on the sensed sound, proper or improper operation of the blower may be determined. The one or more characteristics of and/or within the air circuit 4170 may include a type of conduit connected to the RPT device, a type of patient interface connected to the RPT device, detection of obstruction such as within the patient interface and/or conduit, and/or determine a patient using the patient interface. The circuitry may be configured to generate an output indicating one or more determined characteristics of and/or within the air circuit 4170.

In step 1014, an action is performed based on the result of the analysis in step 1010. The action can include any one or more of: controlling the RPT device based on the results of the analysis, recording the result of the analysis, displaying the result of the analysis and/or forwarding the result of the analysis via the data communication interface 4280. The RPT device may also be controlled on signals received from one or more other sensors.

Controlling the RPT device based on the results of the analysis may include modifying one or more operation parameters (e.g., of the blower and/or humidifier), providing access to one or more additional features, and/or removing access to one or more features. For example, based on the results of the analysis, the speed of the blower may be increased or decreased. If an obstruction is detected in the conduit and/or the patient interface, the blower may be controlled to stop operating and an error message may be displayed. If a specific type of conduit and/or patient interface is detected, the blower and/or humidifier parameters may be modified to operating parameters associated with the identified type of conduit and/or patient interface. The RPT device may be controlled based on the results of the analysis and on signals received from one or more other sensors (e.g., a pressure sensor or a temperature sensor).

Recording the result of the analysis may include storing in local and/or removable memory the analysis results. The stored analysis results may include processed sound signals.

Displaying the result of the analysis may include displaying a notification indicating one or more identified issues, type of mask and/or conduit identified, instructions for actions that need to be taken to address an identified issue, and/or need to service the RPT device and or a component associated with the RPT device. The results may be displayed on a display (e.g., output device 4290) physically coupled to the RPT device or a display in communication with the RPT device.

Forwarding the result of the analysis via the data communication interface 4280 may include transmitting the results to another device associated with the patient, manufacturer and/or clinician.

Determining the type of patient interface may include determining whether a patient interface includes a nasal or full face mask. The type of conduit and/or patient interface may be identified based on differences between mask's acoustic reflections and/or conduit's acoustic reflections and may permit the identification of different conduits and/or masks without user or patient intervention. The mask's acoustic reflections and/or conduit's acoustic reflections may be determined by mask shapes, mask configurations, mask materials, conduit materials, and/or conduit dimensions (e.g. thickness and/or length of conduit tubing). The air circuit 4170 can have many different variations of conduits and masks, each combination having different acoustic properties. The acoustic properties may be determined based on reflected frequencies and/or timing of the reflected signals. Based on determining that a specific patient is using a specific patient interface, the patient may be authenticated and provided access to one or more specialised therapies and/or features in the RPT device, which may be pre-stored. The access to one or more specialised therapies and/or features may be provided automatically upon authentication of the user.

The analysis of the sensed sound may include determining the originally generated sound and the reflected sound (e.g., echo signal) in the sensed sound signal and comparing the reflected sound to the corresponding generated sound. Alternative analysis may involve using the inverse Fourier Transform of the logarithmic spectrum to separate the Impulse Response Function (IRF) of the targeted element (the tube or the mask), or components of the targeted element, from the original sound and the RPT device impulse response function. The mask IRF is used to generate features that can be used to classify the targeted element via a machine learning classifier. In some examples, the sensed sound signal may be compared to stored reference signals. In some examples, characteristics of the sensed sound signal may be determined and compared to known characteristics associated with specific conduits, masks and/or users.

The analysis of the sensed sound may include identifying physical characteristics of and/or within an air circuit 4170 based on the comparison. The analysis may include comparing at least one of; the sensed sound, the originally generated sound extracted from the sensed sound and/or the sound reflective properties of a system component extracted from the sensed sound, to at least one of; a reference signal associated with proper operation of the RPT device, a reference signal associated with a specific conduit, a reference signal associated with a specific patient interface, a reference signal associated with a specific patient, and/or a reference signal associated with any combination of a specific conduit, a specific patient interface and/or a specific patient. Further examples of analysing sensed audio signal are described in PCT Patent Application Publication No. WO 2010/091462, which is incorporated herewith by reference in its entirety.

The features of the intermediate component and the sensor 4270 (e.g., microphone) may be configured to sense a large range of frequencies that may carry a useful reflected signal from the conduit and/or the patient interface. In some examples, at least some of the high signal frequencies may be lost in the conduit and/or the mask. In such cases, it may be preferred to tune the transfer function of the membrane 9732 or 8732 and/or the sensor 4270 to the remaining signal that is of lower frequencies. In some examples, the membrane 9732 or 8732 and/or the sensor 4270 can be tuned to obtain signals that are below about 10 kHz. In some examples, the characteristics of the air circuit 4170 may be modified to increase the frequency range of the returned signal by increasing the wall stiffness of the mask and/or conduit. However, this is not always practical, as modifying characteristics of the air circuit 4170 (e.g., by increasing the wall thickness and/or constructing the tube wall from higher density materials) may be in conflict with the general respiratory system preferences of having lighter and more flexible air circuit 4170.

Most of the above description, including that of the intermediate component 6700, 8700, 9700 and the associated sound detection arrangements, was directed to a system consistent with the presence of an integrated humidifier, as shown in Fig. 5C. However, the description is equally applicable to alternative arrangements, such as the non-humidified systems. An illustrative example of such systems is illustrated in Fig. 5B, where the water reservoir 6100 is replaced by a muffler 4124. In this case, the intermediate component's inlet end 6710 (9710) is configured for engagement not with the water reservoir 6100, but directly with the reservoir dock 6050. In the absence of a water reservoir dock, such is the case with non-humidified RPT devices, the intermediate component's inlet end 6710 (9710) may be connected to the pneumatic block of the RPT device, which includes the motor and the propeller of the device. Furthermore, the intermediate component may not be a removable component, but a part of the RPT device arranged to propagate, preferably to guide by way of defining a flow path, a sound signal from the water reservoir, pneumatic block and/or the air delivery tube 4170, to a detecting sensor (e.g. a microphone) in the device.

Similarly, the sensor 4270, instead of being located adjacent (in the vicinity), but outside of the port 8830 or 9730, may actually be arrange to protrude within the port, or even within the inner space of the intermediate component 6700, 8700 or 9700.

5.10 BREATHING WAVEFORMS

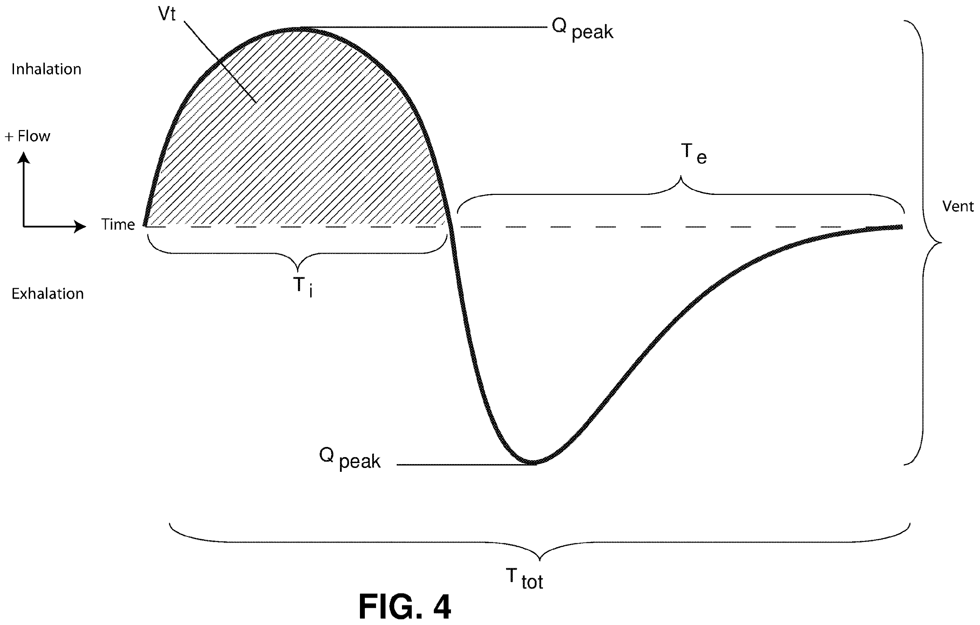

Fig. 4 shows a model typical breath waveform of a person while sleeping. The horizontal axis is time, and the vertical axis is respiratory flow rate. While the parameter values may vary, a typical breath may have the following approximate values: tidal volume $Vt$ 0.5L, inhalation time $Ti$ 1.6s, peak inspiratory flow rate $Qpeak$ 0.4 L/s, exhalation time $Te$ 2.4s, peak expiratory flow rate $Qpeak$ -0.5 L/s. The total duration of the breath, $Ttot,$ is about 4s. The person typically breathes at a rate of about 15 breaths per minute (BPM), with Ventilation $Vent$ about 7.5 L/min. A typical duty cycle, the ratio of $Ti$ to $Ttot,$ is about 40%.

5.11 GLOSSARY

For the purposes of the present technology disclosure, in certain forms of the present technology, one or more of the following definitions may apply. In other forms of the present technology, alternative definitions may apply.

**5.11.1 General**

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Total flow rate, *Qt,* is the flow rate of air leaving the RPT device. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Humidifier:* The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including $cmH_2O$, $g-f/cm^2$ and hectopascal. 1 $cmH_2O$ is equal to 1 $g-f/cm^2$ and is approximately 0.98 hectopascal. In this specification, unless otherwise stated, pressure is given in units of $cmH_2O$.

The pressure in the patient interface is given the symbol *Pm,* while the treatment pressure, which represents a target value to be achieved by the mask pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator*: A mechanical device that provides pressure support to a patient to perform some or all of the work of breathing.

### 5.11.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.11.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions.

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH$_2$O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.11.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:

(i) *Flattened:* Having a rise followed by a relatively flat portion, followed by a fall.

(ii) *M-shaped*: Having two local peaks, one at the leading edge, and one at the trailing edge, and a relatively flat portion between the two peaks.

(iii) *Chair-shaped:* Having a single local peak, the peak being at the leading edge, followed by a relatively flat portion.

(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr)*: These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume *Ve* (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.11.3 Ventilation

*Adaptive Servo-Ventilator (ASV):* A servo-ventilator that has a changeable, rather than fixed target ventilation. The changeable target ventilation may be learned from some characteristic of the patient, for example, a respiratory characteristic of the patient.

*Backup rate:* A parameter of a ventilator that establishes the minimum breathing rate (typically in number of breaths per minute) that the ventilator will deliver to the patient, if not triggered by spontaneous respiratory effort.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP):* a base pressure, to which a pressure varying within the breath is added to produce the desired mask pressure which the ventilator will attempt to achieve at a given time.

*End expiratory pressure (EEP):* Desired mask pressure which the ventilator will attempt to achieve at the end of the expiratory portion of the breath. If the pressure waveform template $\Pi(\Phi)$ is zero-valued at the end of expiration, i.e. $\Pi(\Phi) = 0$ when $\Phi = 1$, the EEP is equal to the EPAP.

*Inspiratory positive airway pressure (IPAP)*: Maximum desired mask pressure which the ventilator will attempt to achieve during the inspiratory portion of the breath.

*Pressure support:* A number that is indicative of the increase in pressure during ventilator inspiration over that during ventilator expiration, and generally means the difference in pressure between the maximum value during inspiration and the base pressure (e.g., *PS = IPAP - EPAP*). In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous/Timed (S/T):* A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator delivers a breath of *air* to a spontaneously breathing patient, it is said to be triggered to do so at the initiation of the respiratory portion of the breathing cycle by the patient's efforts.

### 5.11.4 Patient interface

*Anti-asphyxia valve (AAV):* The component or sub-assembly of a mask system that, by opening to atmosphere in a failsafe manner, reduces the risk of excessive $CO_2$ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.11.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

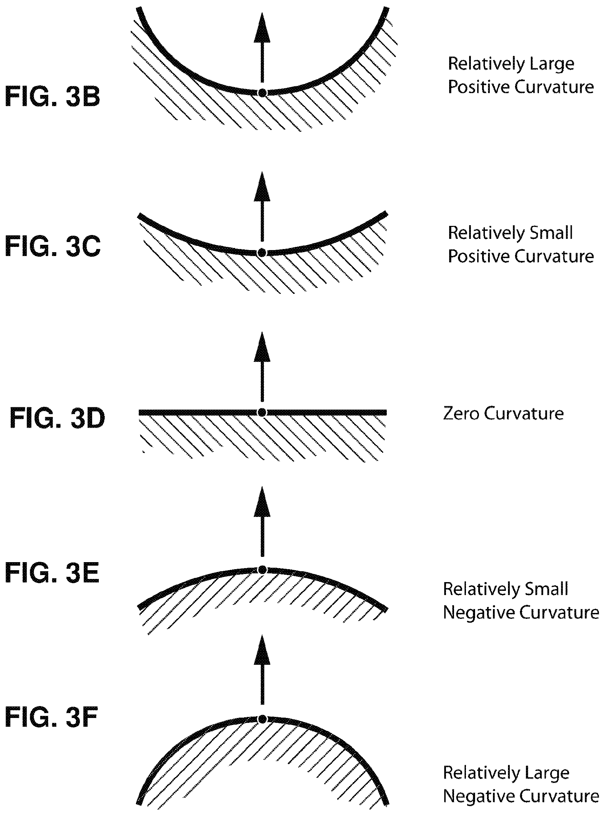

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 5.11.5.1 Curvature in one dimension

The curvature of a plane curve at *p* may be described as having a sign (e.g. positive, negative) and a magnitude (e.g. 1/radius of a circle that just touches the curve at *p*).

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 5.11.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from *f*(0) to *f*(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.11.5.3 Space curves

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix. A typical human right ear comprises a helix, which is a right-hand helix.

The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule, or alternatively by a left-hand rule.

*Osculating plane:* The plane containing the unit tangent vector and the unit principal normal vector.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path).

With reference to the right-hand rule, a space curve turning towards the direction of the right-hand binormal may be considered as having a right-hand positive torsion. A space curve turning away from the direction of the right-hand binormal may be considered as having a right-hand negative torsion (e.g. a left-hand helix).

Equivalently, and with reference to a left-hand rule, a space curve turning towards the direction of the left-hand binormal may be considered as having a left-hand positive torsion (e.g. a left-hand helix). Hence left-hand positive is equivalent to right-hand negative.

### 5.11.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3G, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. In a yet another example, a conduit may comprise a one-dimension hole (e.g. at its entrance or at its exit), and a two-dimension hole bounded by the inside surface of the conduit. See also the two dimensional hole through the structure shown in Fig. 3I, bounded by a surface as shown.

### 5.12 OTHER REMARKS

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are incorporated herein by reference in their entirety to disclose and describe the methods and/or materials which are the subject of those publications. The publications discussed herein are provided solely for their disclosure prior to the filing date of the present specification. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the spirit and scope of the technology.

5.13 REFERENCE SIGNS LIST

| Feature Item | Number |
|---|---|
| patient | 1000 |
| step | 1002 |
| step | 1006 |
| step | 1010 |
| step | 1014 |
| bed partner | 1100 |
| patient interface | 3000 |
| seal - forming structure | 3100 |
| plenum chamber | 3200 |
| structure | 3300 |
| vent | 3400 |
| connection port | 3600 |
| forehead support | 3700 |
| RPT device | 4000 |
| main panel | 4010 |
| front panel | 4012 |
| side panel | 4014 |
| chassis | 4016 |
| pneumatic block | 4020 |
| air filter | 4110 |
| inlet air filter | 4112 |
| outlet air filter | 4114 |

(continued)

| Feature Item | Number |
|---|---|
| muffler | 4120 |
| inlet muffler | 4122 |
| outlet muffler | 4124 |
| pressure generator | 4140 |
| blower | 4142 |
| motor | 4144 |
| anti - spill back valve | 4160 |
| air circuit | 4170 |
| air circuit | 4171 |
| supplemental oxygen | 4180 |
| electrical components | 4200 |
| PCBA | 4202 |
| power supply | 4210 |
| input device | 4220 |
| central controller | 4230 |
| clock | 4232 |
| therapy device controller | 4240 |
| protection circuits | 4250 |
| memory | 4260 |
| transducer | 4270 |
| sensor housing | 4271 |
| pressure sensor | 4272 |
| flow rate sensor | 4274 |
| motor speed transducer | 4276 |
| ambient light sensor | 4278 |
| data communication interface | 4280 |
| remote external communication network | 4282 |
| local external communication network | 4284 |
| remote external device | 4286 |
| local external device | 4288 |
| output device | 4290 |
| display driver | 4292 |
| display | 4294 |
| algorithms | 4300 |
| pre - processing module | 4310 |
| pressure compensation algorithm | 4312 |
| vent flow rate estimation algorithm | 4314 |
| leak flow rate estimation algorithm | 4316 |
| respiratory flow rate estimation algorithm | 4318 |

(continued)

| Feature Item | Number |
| --- | --- |
| therapy engine module | 4320 |
| phase determination algorithm | 4321 |
| waveform determination algorithm | 4322 |
| ventilation determination algorithm | 4323 |
| inspiratory flow limitation determination algorithm | 4324 |
| apnea / hypopnea determination algorithm | 4325 |
| snore determination algorithm | 4326 |
| airway patency determination algorithm | 4327 |
| target ventilation determination algorithm | 4328 |
| therapy parameter determination algorithm | 4329 |
| therapy control module | 4330 |
| fault condition detection algorithm | 4340 |
| tube portion | 4500 |
| dock connector | 4600 |
| base portion | 4640 |
| tapered protrusion | 4642 |
| retaining bumps | 4644 |
| contact assembly | 4660 |
| humidifier | 5000 |
| water reservoir | 5110 |
| humidifier transducers | 5210 |
| air pressure sensor | 5212 |
| flow rate transducers | 5214 |
| temperature sensor | 5216 |
| humidity sensor | 5218 |
| heating element | 5240 |
| humidifier controller | 5250 |
| central humidifier controller | 5251 |
| heating element controller | 5252 |
| air circuit controller | 5254 |
| integrated RPT device and humidifier | 6000 |
| reservoir dock | 6050 |
| slot | 6055 |
| guide slot | 6060 |
| heater plate | 6080 |
| dock outlet | 6090 |
| dock opening | 6091 |
| water reservoir | 6100 |
| base | 6112 |

(continued)

| Feature Item | Number |
|---|---|
| reservoir lid | 6114 |
| inlet tube | 6120 |
| outlet tube | 6130 |
| outlet seal | 6132 |
| main body | 6140 |
| conductive portion | 6150 |
| guide rails | 6200 |
| latch | 6400 |
| intermediate component | 6700 |
| tubular body | 6705 |
| inlet end | 6710 |
| lip | 6712 |
| contact surface | 6715 |
| outlet end | 6720 |
| port | 6730 |
| port seal | 6735 |
| pinch arms | 6740 |
| barbed end | 6745 |
| protrusion | 6750 |
| guide slot | 6755 |
| guide rail | 6760 |
| flange | 6770 |
| recesses | 6772 |
| channel | 6780 |
| locking and contact assembly | 6900 |
| base | 6910 |
| rear wall | 6912 |
| opening | 6915 |
| annular side wall | 6920 |
| wall | 6930 |
| recess | 6940 |
| contact assembly | 6950 |
| support member | 6952 |
| cover | 6970 |
| pneumatic block | 7100 |
| chassis | 7300 |
| chassis outlet | 7320 |
| chassis opening | 7380 |
| chassis side wall | 7390 |

(continued)

| Feature Item | Number |
|---|---|
| PCBA | 7600 |
| conducting tracks | 7602 |
| through holes | 7604 |
| intermediate component | 8700 |
| membrane | 8732 |
| coupler | 8750 |
| body | 8752 |
| bellows | 8754 |
| inlet end | 8756 |
| outlet end | 8758 |
| flange | 8759 |
| portion | 8760 |
| first connecting element | 8762 |
| opening | 8764 |
| second connecting element | 8766 |
| rubber interface | 8780 |
| tubular body | 8782 |
| interface opening | 8784 |
| end | 8785 |
| connecting ports | 8786 |
| rubber feet | 8788 |
| housing | 8800 |
| portion | 8810 |
| posts | 8814 |
| edge | 8816 |
| flexible wires | 8820 |
| port | 8830 |
| edge | 8832 |
| channel | 8900 |
| tab | 8910 |
| connected portion | 8920 |
| intermediate component | 9700 |
| tubular body | 9705 |
| exterior surface | 9707 |
| inlet end | 9710 |
| inner corner | 9712 |
| inner surface | 9712B |
| outer surface | 9712C |
| span | 9712D |

(continued)

| Feature Item | Number |
|---|---|
| outer corner | 9714 |
| inlet seal | 9715 |
| outlet end | 9720 |
| port | 9730 |
| membrane | 9732 |
| port seal | 9735 |
| ridge seal | 9737 |
| connecting portions | 9739 |
| pinch arm | 9740 |
| lip seal | 9742 |
| space | 9743 |
| barbed end | 9745 |
| side surface | 9746 |
| groove | 9747 |
| cross - bar | 9750 |
| chassis guide slots | 9755 |
| slope | 9757 |
| guide rail | 9760 |
| guide rib | 9761 |
| flange | 9770 |
| bumper | 9775 |
| channel | 9780 |
| part - annular side wall | 9790 |
| holes | 9792 |
| tab | 9795 |
| sleeve | 9800 |
| peripheral sealing formation | 9805 |
| additional sealing portion | 9807 |

The following aspects are preferred embodiments of the invention.

1. An apparatus for treatment of a respiratory condition, the apparatus comprising:

a pressure generator configured to generate a flow of breathable gas;

an intermediate component pneumatically connected to an air delivery tube, the intermediate component comprising a port configured to facilitate propagation of sound outside of the intermediate component;

a sensor attached externally to the intermediate component and located adjacent to the port of the intermediate component, the sensor configured to sense sound propagated through the port; and

a controller configured to:

receive a sound signal generated by the sensor as a result of sensing sound during operation of the apparatus,

analyse the received sound signal, and

effect a response based at least in part on the analysing.

2. The apparatus according to aspect 1, wherein the intermediate component is configured to pneumatically connect an air delivery tube to the pressure generator.

3. The apparatus according to any one of aspects 1 to 2, where the response comprises at least one of:

recording a result of the analysing,

displaying a result of the analysing,

forwarding a result of the analysing, and

controlling operation of the pressure generator based at least in part on the analysing.

4. The apparatus according to any one of aspects 1 to 3, further comprising a chassis including a chassis opening, wherein the port in the intermediate component is located on a first side of the chassis opening, and the sensor is positioned on a second side of the chassis opening.

5. The apparatus according to aspect 4, further comprising a circuit board located on the second side of the chassis opening, wherein the sensor is coupled to the circuit board.

6. The apparatus according to any one of aspects 1 to 5, further comprising a flexible coupler configured to pass sound from the port to the sensor.

7. The apparatus according to aspect 6, further comprising a circuit board to which the sensor is coupled, wherein the coupler includes an outlet end configured to directly engage the sensor and an inlet end configured to removably engage the intermediate component.

8. The apparatus according to any one of aspects 1 to 7, further comprising the air delivery tube configured to pass the flow of breathable gas from the pressure generator to a patient interface.

9. The apparatus according to any one of aspects 1 to 8, further comprising a membrane configured to cover the port and to transmit sound from inside of the intermediate component to the outside of the intermediate component.

10. The apparatus according to aspect 9, wherein the membrane is provided below an outside surface of the intermediate component.

11. The apparatus according to any one of aspects 1 to 10, further comprising at least one of a membrane and a port seal, the membrane configured to cover the port and to transmit sound from inside of the intermediate component to the outside of the intermediate component, and the port seal being disposed so as to provide a sealing engagement between the port and the chassis opening when the intermediate component is in its operational configuration.

12. The apparatus according to any one of aspects 1 to 11, further comprising:

a water reservoir including a cavity structured to hold a volume of water and receive the flow of breathable gas so that the flow of breathable gas is humidified before being passed to the patient interface; and
a water reservoir dock structured and arranged to receive the water reservoir in an operative position, wherein the intermediate component is removably coupled to the water reservoir dock so as to receive a humidified flow of breathable gas and pass it to the air delivery tube.

13. The apparatus of aspect 12, wherein the intermediate component is of a generally tubular shape and the water reservoir dock includes a generally tubular opening for receiving the intermediate component, the intermediate component and the generally tubular opening being configured for a generally frictionless insertion of the intermediate component into the opening.

14. The apparatus of aspect 13, wherein at least one of the intermediate component and the generally tubular opening comprises at least one engagement formation arranged so that, during an insertion of the intermediate component into the opening, an engagement of at least one of the engagement formations brings the intermediate component into its operational configuration in which at least one of the following is effected: a sealing engagement of the port seal with the chassis opening; and supporting engagement effected by the at least one engagement formation is configured to impede dislodgement of the intermediate component from its operational configuration in the absence of an appreciable external force.

15. The apparatus of aspect 14, wherein the engagement of at least one of the engagement formations occurs at a later point of an insertion path.

16. The apparatus of aspect 15, wherein one or more of the engagement formations includes an elevation feature causing an upward movement of at least a portion of the intermediate component.

17. The apparatus of aspect 16, wherein the elevation feature is provided on a bottom of the tubular opening and pushes the intermediate component upwards to reduce a clearance between a top of the tubular opening and the intermediate component after the intermediate component is inserted a predetermined distance into the tubular opening.

18. The apparatus according to any one of aspects 14 to 16, each of at least one of the intermediate component and the generally tubular opening comprising a plurality of engagement formations, each of the engagement formations arranged to engage approximately simultaneously during the insertion of the intermediate component into the opening.

19. The apparatus according to any one of aspects 1 to 18, further comprising a port seal configured to surround the port, wherein the port seal includes a peripheral sealing formation including a ridge configured to abut a surface of the chassis around the chassis opening, when the intermediate component is coupled to a water reservoir dock.

20. The apparatus according to any one of aspects 1 to 18, further comprising a port seal configured to surround the port, wherein the port seal includes a peripheral sealing formation including a lip configured to abut a surface of a chassis around the chassis opening, when the intermediate component is coupled to a humidifier.

21. The apparatus according to aspect 20, wherein the lip extends from perimeter of the port at an angle above the port and toward a central axis of the port.

22. The apparatus according to any one of aspects 11 to 21, wherein the port seal covers an inside surface of the port and includes the membrane.

23. The apparatus according to any one of aspects 9 to 22, wherein the membrane is coplanar with at least an inside or an outside surface of the intermediate component.

24. The apparatus according to any one of aspects 9 to 18 or 23, wherein the membrane is impermeable to liquids and/or gases.

25. The apparatus according to any one of aspects 1 to 24, wherein the controller is configured to determine a characteristic of the air delivery tube or a patient interface, based on the analysing.

26. The apparatus of aspect 25, wherein the controller is further configured to determine a type or a size of the air delivery tube or a type or a size of a patient interface coupled to the air delivery tube, based on the analysing.

27. The apparatus according to any one of aspects 12 to 19, wherein the intermediate component comprises an outlet end configured to connect the air delivery tube to the intermediate component and an inlet end configured to connect the water reservoir to the intermediate component, wherein an air path between the inlet end and the outlet end is nonlinear and includes at least one turn, wherein at least the turn closest to the port is curved.

28. The apparatus according to aspect 27, wherein a central axis of the inlet end is substantially transverse to a central axis of the outlet end, defining corresponding transverse air paths, an outer and an inner corner, each corner comprising a rounded inner surface.

29. The apparatus according to aspect 28, wherein the transverse air path between the inlet end cross adjacent the inlet end, the inlet end includes an inlet seal adapted to interface with the water reservoir.

30. The apparatus according to any one of aspects 28 to 29, wherein an inside corner comprises a bellow including a span between opposite sides of the bellow that is equal to or less than twice a radius of the inside corner.

31. The apparatus according to any one of aspects 28 to 30, wherein an inner surface of the inside corner comprises a curvature of a radius of 0.2-5 mm.

32. The apparatus according to any one of aspects 1 or 31, wherein the intermediate component is configured to pneumatically connect the air delivery tube to the water reservoir, and to mechanically connect the air delivery tube to the water reservoir dock.

33. The apparatus according to any one of aspects 1 to 32, wherein the air delivery tube is configured to form a mechanical and electrical connections simultaneously when the air delivery tube is connected to the intermediate component.

34. The apparatus according to any one of aspects 1 to 33, further comprising a transducer configured to generate a flow signal representing a property of flow of air, wherein the controller is configured to:

control operation of the pressure generator; and

during operation of the pressure generator:

receive the flow signal from the transducer and sound signal sensed by the sensor;
analyse the received sounds signal; and
modify operation of the pressure generator based at least in part on the analysing and the flow signal.

35. The apparatus according to any one of aspects 1 to 3 and 6 to 34, further comprising:

a chassis including a chassis opening extending through the chassis, wherein the port in the intermediate component is located adjacent to a first end of the chassis opening; and
a circuit board located adjacent to a second end of the chassis opening, wherein the sensor is positioned on the circuit board and aligned with the chassis opening.

36. The apparatus according to aspect 35, wherein the second end of the chassis opening includes a larger opening than an opening of the first end.

37. The apparatus according to any one of aspects 35 to 36, wherein the second end of the chassis opening is provided by a side wall extending from a surface of the chassis facing the circuit board.

38. The apparatus according to any one of aspects 35 to 37, wherein the sensor is positioned at least partially inside of the chassis opening and/or the side wall.

39. The apparatus according to any one of aspects 35 to 38, further comprising a seal disposed between the chassis and the circuit board and adjacent the second end of the chassis opening.

40. The apparatus according to aspect 39, wherein the seal includes a peripheral sealing formation including a lip or a ridge configured to abut a surface of the circuit board adjacent the sensor.

41. The apparatus according to any one of aspects 1 to 40, further comprising a generally tubular opening in a chassis of the apparatus for receiving the intermediate component of a generally tubular shape, and the intermediate component comprises an inlet end adapted to be inserted into the opening, an outlet end adapted to interface with the air delivery tube, a flange arranged between the inlet end and the outlet end, and one or more flexible bumpers provided adjacent to a side of the flange facing the inlet end.

42. The apparatus according to aspect 41, wherein the intermediate component further comprises a barbed tab at one end of the intermediate component opposite to the outlet end, wherein the barbed tab is structured to provide a snap-fit connection with a locking member of the chassis and the one or more bumpers are depressed against a portion of the chassis by the flange during the snap-fit connection and force the barbed tab against the locking member to impede disengagement of the barbed tab from the locking member in the absence of an appreciable external force.

43. The apparatus according to aspect 42, wherein during initial insertion of the intermediate component into the generally tubular opening, minimal resistance is provided between the intermediate component and the generally tubular opening, and resistance is increased at a later stage of the insertion at a location where one or more engagement features of the intermediate component engage respective engagement features in the generally tubular opening of the chassis, the engagement between respective engagement features of the intermediate component and the chassis opening directing the intermediate component into its operational configuration.

44. The apparatus according to any one of aspects 42 to 43, further comprising a port seal disposed so as to provide a sealing engagement between the port and the chassis opening in an operative position, wherein during a later stage of insertion of the intermediate component into the generally tubular opening, an interaction between respective engagement features of the intermediate component and the chassis opening positions the intermediate component to ensure a sealing engagement between the port seal and a surface of the chassis around the chassis opening.

45. The apparatus according to aspect 44, further comprising a water reservoir including a cavity structured to hold a volume of water and receive the flow of breathable gas so that the flow of breathable gas is humidified before being passed to a patient interface; and a water reservoir dock structured and arranged to receive the water reservoir in the operative position, wherein

the inlet end of the intermediate component is removably coupled to the water reservoir dock so as to receive a humidified flow of breathable gas and pass it to the air delivery tube,
the inlet end includes an inlet seal, and
in the operative position, the port seal provides sealing engagement between the port and the chassis opening and the inlet seal provides a sealing engagement between the inlet end and the water reservoir dock.

46. The apparatus according to any one of aspects 41 to 45, wherein the intermediate component further comprises a guide rib on an outside surface of the intermediate component and/or a guide rail on the outside surface of the intermediate component, the guide rib and the guide rail are structured and arranged to assist in alignment and insertion of the intermediate component into the generally tubular opening in a chassis by engagement with corresponding guide slots extending into the generally tubular opening in the chassis.

47. The apparatus according to aspect 46, wherein the guide rib is provided on forward and upper side of the intermediate component and the guide rail is provided on a lower side of the intermediate component.

48. The apparatus according to any one of aspects 1 to 38, wherein the chassis includes a generally tubular opening for receiving the intermediate component of a generally tubular shape, and the intermediate component comprises an inlet end adapted to be inserted into the opening and an outlet end adapted to interface with the air delivery tube, wherein a clearance between the port seal and the chassis near the chassis opening is provided during insertion of the intermediate component into the opening and the port seal begins to engage the chassis after an edge of the port passes a central axis or an edge of the chassis opening during insertion of the intermediate component into the opening.

49. The apparatus according to aspect 48, wherein, after the port seal begins to engage the chassis, the intermediate component is further inserted a predetermined distance to bring the intermediate component into its operational configuration.

50. The apparatus according to aspect 5, further comprising a flexible housing overmoulded on the sensor that is displaced from the circuit board at least partially past the chassis opening.

**Claims**

**1.** An intermediate component for pneumatically connecting an air delivery tube to a respiratory treatment apparatus, the air delivery tube configured to deliver breathable gas provided by the respiratory treatment apparatus to a patient interface, said intermediate component comprising:

an outlet end configured to connect to an air delivery tube;
an inlet end configured to connect to the respiratory treatment apparatus;
a sound port configured to facilitate propagation of sound outside of the intermediate component; and
a sound permeable membrane configured to cover the port.

2. The intermediate component of claim 1, further comprising a port seal disposed around the sound port and configured to provide a peripheral sealing formation on an outside surface of the intermediate component.

3. The intermediate component of claim 2, wherein the membrane and the port seal are formed as one integral component.

4. The intermediate component of any one of claims 2 and 3, wherein the port seal covers an inside surface of the sound port and includes the membrane.

5. The intermediate component of any one of claims 2 to 4, wherein the port seal comprises a relatively soft material provided to a relatively rigid base mold of the intermediate component;

6. The intermediate component of claim 5, wherein the base mold comprises a thermoplastic polymer and the relatively soft material of the port seal comprises a thermoplastic elastomer or silicone.

7. The intermediate component of any one of claims 2 to 6, wherein the peripheral sealing formation comprises: 1) a ridge configured to abut a chassis surface around a chassis opening to provide a sealing engagement; or 2) a lip configured to abut a chassis surface around a chassis opening to provide a sealing engagement.

8. The intermediate component of any one of claims 1 to 7, wherein the membrane is impermeable to liquids and/or gases.

9. The intermediate component of any one of claims 1 to 7, wherein the membrane is configured to, in use, transmit sound from inside of the intermediate component to outside of the intermediate component while keeping a sensor of the respiratory treatment apparatus out of an airflow path.

10. The intermediate component of any one of claims 1 to 9, wherein the membrane has a thickness between 0.05 mm and 3 mm.

11. The intermediate component of any one of claims 1 to 10, wherein a central axis of the inlet end is provided at an angle greater than zero to a central axis of the outlet end.

12. The intermediate component of any one of claims 1 to 11, further comprising an inlet seal at the inlet end configured to interface with a water reservoir of the respiratory treatment apparatus.

13. The intermediate component of claim 12, wherein the inlet seal comprises a bellows-type arrangement that is resiliently compressible to provide a degree of decoupling between the intermediate component and the water reservoir.

14. The intermediate component of any one of claims 12 and 13, when dependent on claim 5, wherein the inlet seal is formed as part of the relatively soft material provided to the base mold of the intermediate component.

15. The intermediate component of any one of claims 1 to 14, further comprising:

a flange arranged between the inlet end and the outlet end; and
one or more bumpers provided adjacent to a side of the flange facing the inlet end and configured to absorb vibrations during use.

16. The intermediate component of any one of claims 1 to 15, further comprising a barbed tab structured to provide a snap-fit connection with a locking member of a chassis of the respiratory treatment apparatus when the intermediate component is inserted into a tubular opening of the respiratory treatment apparatus.

FIG. 1A

FIG. 1B

FIG. 1C

**FIG. 2A**

**FIG. 2B**

FIG. 3A

**FIG. 3B**

Relatively Large
Positive Curvature

**FIG. 3C**

Relatively Small
Positive Curvature

**FIG. 3D**

Zero Curvature

**FIG. 3E**

Relatively Small
Negative Curvature

**FIG. 3F**

Relatively Large
Negative Curvature

FIG. 3H ↑     Curve     ↑ FIG. 3H

**FIG. 3G**

Surface

**FIG. 3H**

Surface

**FIG. 3I**

FIG. 4

EP 4 732 876 A2

FIG. 5A

FIG. 5B

EP 4 732 876 A2

FIG. 5C

EP 4 732 876 A2

**FIG. 5D**

FIG. 5E

**FIG. 5F**

FIG. 5G

FIG. 6A

EP 4 732 876 A2

FIG. 6B

**FIG. 7**

FIG. 8

**FIG. 9**

EP 4 732 876 A2

FIG. 10

FIG. 11

**FIG. 12**

EP 4 732 876 A2

**FIG. 13**

EP 4 732 876 A2

FIG. 14

**FIG. 15**

EP 4 732 876 A2

**FIG. 16**

**FIG. 17**

**FIG. 18**

FIG. 19

FIG. 20

FIG. 21

FIG. 22

FIG. 23

FIG. 24

FIG. 25

FIG. 26

**FIG. 27**

EP 4 732 876 A2

**FIG. 28A**

FIG. 28B

FIG. 28C

**FIG. 28D**

**FIG. 28E**

**FIG. 28F**

**FIG. 28G**

**FIG. 29**

**FIG. 30**

**FIG. 31**

**FIG. 32**

**FIG. 33**

FIG. 34A

FIG. 34B

9739   9743   D1   D2   A1

9737

D3

D4

9707

9730   9732

**FIG. 34C**

9739   9743   7300   9737

9707

9730   9732

**FIG. 34D**

9739

9742

9732

9715

9739

35C

9745

9740

9775

**FIG. 35A**

7300  9743  9739  7380  9742  9739

9707  9732

**FIG. 35B**

**FIG. 35C**

**FIG. 35D**

**FIG. 36A**

**FIG. 36B**

**FIG. 36C**

FIG. 36D

FIG. 37

**FIG. 38A**

**FIG. 38B**

8758    8764

8750

8700

**FIG. 38C**

8830
8766

8700

**FIG. 38D**

**FIG. 39A**

**FIG. 39D**

**FIG. 39C**

**FIG. 39B**

4270

Escape of
Sound

8700

Horizontal
misalignment

Vertical
misalignment

8732

8830

Sound Waves

**FIG. 40A**

4270

8700

8750

8830

8732

Sound Waves

**FIG. 40B**

8780

8788

8782

8784

**FIG. 41A**

8780

8786    8786

8785

8788

8788

**FIG. 41B**

8788

8780

7600

8700

**FIG. 41C**

7600

8900

8910

7604

8920

7602

**FIG. 42A**

7600

8900

7602

4270

8766

8700

**FIG. 42B**

**FIG. 42C**

**FIG. 43**

Control RPT device to provide a flow of
breathable gas to a patient
1002

Capture sound
1006

Analyze captured sound
1010

Perform an action based on results of
analysis
1014

FIG. 44

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63011052
- US 63048535
- US 4944310 A, Sullivan
- US 6532959 B, Berthon-Jones
- WO 2015089582 A
- US 7866944 B
- US 8638014 B
- US 8636479 B
- WO 2013020167 A
- US 8733349 B
- WO 2012171072 A
- WO 2010091462 A

**Non-patent literature cited in the description**

- **JOHN B. WEST**. Respiratory Physiology. Lippincott Williams & Wilkins, 2012